# EUROPEAN PATENT APPLICATION

(11) **EP 4 831 295 A2**
(43) Date of publication of application: **28.10.2026**
(21) Application number: 26204488.6
(22) Date of filing: 17.07.2018
(51) Int. Cl.: C12P 19/04

(54) **IMMUNOGENIC COMPOSITION**

(30) Priority: 19.07.2017 GB 201711637
(62) Document number(s) of the earlier application(s) in accordance with Art. 76 EPC: 18758809.0 / 3 655 030
(71) Applicant: GlaxoSmithKline Biologicals SA, 1330 Rixensart (BE)
(72) Inventor: FARIDMOAYER, Amirreza, 8952 Schlieren (CH); FOLLADOR, Rainer, 8952 Schlieren (CH); KEMMLER, Stefan Jochen, 8952 Schlieren (CH); KOWARIK, Michael Thomas, 8952 Schlieren (CH); POSCH, Gerald Johann, 8952 Schlieren (CH); SERVENTI, Fabio, 8952 Schlieren (CH)
(74) Representative: Baker, Suzanne J.

(57) **Abstract**

The disclosure provides synthetic (e.g. recombinant) pneumococcal saccharides comprising one or more repeat unit(s) →4)-β-D-Glc*p*-(1→4)-[Gro-(2→P→3)]-β-D-Gal*p*-(1→4)-β-L-Rha*p-*(1→. Also provided are conjugates comprising a →4)-β-D-Glc*p*-(1→4)-[Gro-(2→P→3)]-β-D-Gal*p*-(1→4)-β-L-Rha*p*-(1→, immunogenic compositions, vaccines and their use in preventing or treating infection by *Streptococcus pneumoniae.*

## Description

### Technical Field

The present invention relates to the field of immunogenic compositions and vaccines, their manufacture and the use of such compositions in medicine. More particularly, it relates to a saccharide from *Streptococcus pneumoniae* comprising one or more repeat units having the structure →4)-β-D-Glc*p*-(1→4)-[Gro-(2→P→3)]-β-D-Gal*p*-(1→4)-β-L-Rhap-(1→. The pneumococcal saccharide can be synthesized (e.g. recombinantly) and used either on its own or as conjugate, coupled to a carrier protein, for use in an immunogenic composition or vaccine.

### Background

*Streptococcus pneumoniae* is a globally important encapsulated human pathogen [van Tonder, A.J.; Bray, J.E.; Quirk, S.J.; Haraldsson, G.; Jolley, K.A.; Maiden, M.C.; Hoffmann, S.; Bentley, S.D.; Haraldsson, Á.; Erlendsdóttir, H.; Kristinsson, K.G. Microb. Genom. 2016, 2(10).]. *Streptococcus pneumoniae* (*S. pneumoniae,* pneumococcus) is a Gram-positive bacterium responsible for considerable morbidity and mortality (particularly in infants and the elderly), causing invasive diseases such as bacteraemia and meningitis, pneumonia and other non-invasive diseases, such as acute otitis media. About 800,000 children die annually due to pneumococcal disease, especially in emerging countries (O-Brien et al. 2009 Lancet 374:893-902). The increasing number of antibiotic-resistant strains (Linares et al. 2010 Cin. Microbiol. Infect. 16:402-410) and the severity of pneumococcal diseases make vaccination the most effective intervention. The major clinical syndromes caused by *S. pneumoniae* are widely recognized and discussed in standard medical textbooks (Fedson DS, Muscher DM. In: Plotkin SA, Orenstein WA, editors. Vaccines. 4th edition. Philadelphia WB Saunders Co, 2004a: 529-588). For instance, Invasive Pneumococcal Disease (IPD) is defined as any infection in which *S. pneumoniae* is isolated from the blood or another normally sterile site (Musher DM. *Streptococcus pneumoniae*. In Mandell GL, Bennett JE, Dolin R (eds). Principles and Practice of Infectious diseases (5th ed.). New York, Churchill Livingstone, 2001, p2128-2147).

*S. pneumoniae* is encapsulated with a covalently linked polysaccharide which confers serotype specificity. There are more than 90 known serotypes of pneumococci, and the capsule is the principle virulence determinant for pneumococci, as the capsule not only protects the inner surface of the bacteria from complement, but is itself poorly immunogenic. Certain serotypes are more abundant than others, to be associated with clinically apparent infections, to cause severe invasive infections and to acquire resistance to one or more classes of antibacterial agents (Rueda, A. M. M. MSc; Serpa, José A. MD; Matloobi, Mahsa MD; Mushtaq, Mahwish MD; Musher, Daniel M. MD. 2010. The spectrum of invasive pneumococcal disease at an adult tertiary care hospital in the early 21st century. Medicine (Baltimore) 89:331-336). According to previous analyses approximately 10 or 11 serotypes account for over 70% of invasive pediatric infections in all regions of the world (Hausdorff WP, Bryant J, Paradiso PR, Siber GR: Which pneumococcal serogroups cause the most invasive disease: implications for conjugate vaccine formulation and use, part I. Clinical infectious diseases: an official publication of the Infectious Diseases Society of America 2000, 30(1):100-121). The distribution of serotypes causing disease varies by age, disease syndrome, disease severity, geographic region, and over time. Pneumococci that are resistant to penicillin, erythromycin, co-trimoxazole or multiple drugs are common in many regions (Evolving trends in *Streptococcus pneumoniae* resistance: implications for therapy of community-acquired bacterial pneumonia. Jones RN, Jacobs MR, Sader HS. Int J Antimicrob Agents. 2010 Sep;36(3):197-204).

Bacterial polysaccharides may elicit a long-lasting immune response in humans if they are coupled to a protein carrier that contains T-cell epitopes. This concept was elaborated almost 100 years ago (Avery, O. T. and W. F. Goebel, 1929, J. Exp. Med. 50:521-533), and proven later for the polysaccharide of *Haemophilus influenzae* type B (HIB) coupled to the protein carrier diphtheria toxin (Anderson, P. 1983, Infect Immun 39:233-8; Schneerson, R. O. Barrera, A. Sutton, and J. B. Robbins. 1980, J Exp Med 152:361-76). This glycoconjugate was also the first conjugated vaccine to be licensed in the USA in 1987 and introduced into the US infant immunization schedule shortly thereafter. Besides HIB, conjugated vaccines have been successfully developed against the encapsulated human pathogens *Neisseria meningitidis* and *S. pneumoniae*. After initial licensure of a 7-valent conjugate vaccine containing serotypes 4, 6B, 9V, 14, 18C, 19F, 23F (PCV7), two pneumococcal conjugate vaccines (PCVs) designed to broaden coverage have been licensed. The 10-valent pneumococcal *Haemophilus influenzae* protein D conjugate vaccine (PCV10) contains serotypes 1, 4, 5, 6B, 7F, 9V, 14 and 23F conjugated to nontypeable *H. influenzae* protein D, plus serotype 18C conjugated to tetanus toxoid and serotype 19F conjugated to diphtheria toxoid. The 13-valent pneumococcal conjugate vaccine (PCV13) contains the PCV7 (4, 6B, 9V, 14, 18C, 19F, 23F) serotypes plus serotypes 1, 3, 5, 6A, 7F and 19A, conjugated to cross-reactive material CRM197 (a genetically detoxified form of diphtheria toxin).

T-independent antigens, for example saccharides, are antigens that elicit antibody production via B lymphocytes without involvement of T-cells. Conjugation of T-independent antigens to carrier proteins has long been established as a way of enabling T-cell help to become part of the immune response for a normally T-independent antigen. In this way, an immune response can be enhanced by allowing the development of immune memory and boostability of the response. Successful conjugate vaccines which have been developed by conjugating bacterial capsular saccharides to carrier proteins are known in the art; the carrier protein having the known effect of turning the T-independent saccharide antigen into a T-dependent antigen capable of triggering an immune memory response. Several carrier proteins are known in the art with tetanus toxoid, diphtheria toxoid, CRM197 and protein D from *Haemophilus influenzae* being used as carrier protein in commercialised vaccines. CRM197 is currently used in the *Streptococcus pneumoniae* capsular polysaccharide conjugate vaccine PREVENAR (Pfizer) and protein D, tetanus toxoid and diphtheria toxoid are currently used as carriers for capsular polysaccharides in the *Streptococcus pneumoniae* capsular polysaccharide conjugate vaccine SYNFLORIX (GlaxoSmithKline). Other carrier proteins known in the art include EPA (exotoxin A of *P. aeruginosa*) (Wacker et al. J Infect. Dis, 2014 May 15: 209(10):1551-1561) and Outer Membrane Protein (OMP) (Wu et al. Infect. Imun. 1999 Oct 67(1): 5508-5513). In an embodiment, the carrier protein is detoxified Exotoxin A from *Pseudomonas aeruginosa*.

While development of vaccines against such infection is ongoing, there remains a major need for effective vaccines against *Streptococcus pneumoniae* infection that can safely be produced in high quantities.

Despite extensive genetic and serological studies, the polysaccharide structures of several serotypes have yet to be determined. Serogroup 23 consists of serotypes 23F, 23A and 23 B for which only the structure of polysaccharide 23F has been published [Richards, J.C.; Perry, M.B. Biochem. Cell Biol. 1988, 66(7), 758-771.]. Furthermore, only polysaccharide 23F has been included in commercially available vaccines to date. The capsule biosynthetic genes and repeat units for all 90 serotypes are described in Bentley *et al.* (2006) [Bentley, S.D.; Aanensen, D.M.; Mavroidi, A.; Saunders, D.; Rabbinowitsch, E.; Collins, M.; Donohoe, K.; Harris, D.; Murphy, L.; Quail, M.A.; Samuel, G. PLoS Genet. 2006, 2(3), e31] together with the repeat unit structure for 23F, but not 23A or 23B. Based on the epidemiology at the time, serotype 23F was chosen for inclusion in the 23-valent polysaccharide vaccine (PPV23) [Robbins, J.B.; Austrian. R.; Lee, C.J.; Rastogi, S.C.; Schiffman, G.; Henrichsen, J.; Mäkelä, P.H.; Broome, C.V.; Facklam, R.R; Tiesjema, R.H.; Parke, J.C. J. Infect. Dis. 1983, 148(6), 1136-1159] and 23F is currently present in all licensed conjugate vaccines. Epitope specificity studies on synthetic conjugates and killed *S. pneumoniae* 23F in animals showed that the terminal α-Rha is immunodominant [De Velasco, E.A.; Verheul, A.F.; Van Steijn, A.M.; Dekker, H.A.; Feldman, R.G.; Fernandez, I.M.; Kamerling, J.P.; Vliegenthart, J.F.; Verhoef, J.; Snippe, H. Infect. Immun. 1994, 62(3), 799-808]; this has also been observed in human sera from subjects immunized with PPV23 [Park, S.; Nahm, M .H. PLoS One. 2013, 8(12), e83810]. Early studies showed that typing antiserum prepared in rabbits with type 23F bacteria reacts only slightly with serotype 23A and hardly at all with serotype 23B [Robbins, J.B.; Austrian. R.; Lee, C.J.; Rastogi, S.C.; Schiffman, G.; Henrichsen, J.; Mäkelä, P.H.; Broome, C.V.; Facklam, R.R; Tiesjema, R.H.; Parke, J.C. J. Infect. Dis. 1983, 148(6), 1136-1159.] and therefore crossprotection from the 23F polysaccharide and conjugate vaccines is not expected. It is therefore unsurprising that serotype 23A and 23B have been identified as emerging pathogens due to a combination of serotype replacement and antimicrobial resistance.

The genes required for pneumococcal capsular polysaccharide (CPS) synthesis are generally encoded on the cps locus [Bentley, S.D.; Aanensen, D.M.; Mavroidi, A.; Saunders, D.; Rabbinowitsch, E.; Collins, M.; Donohoe, K.; Harris, D.; Murphy, L.; Quail, M.A.; Samuel, G. PLoS Genet. 2006, 2(3), e31]. The locus contains three types of enzymes: those responsible for (i) biosynthesis of nucleotide-activated sugars, (ii) polysaccharide repeat-unit synthesis and (iii) assembly of the repeat units and transport across the membrane. In 23F, WchA initiates the repeat-unit synthesis by catalysing the production of an undecaprenyl pyrophosphate-linked D-Glucose (UndPP)-D-Glucose. In successive steps, WchF adds a L-rhamnose to the UndPP-Glucose, followed by WchV linking a D-galactose. This D-galactose is extended by WchW and WchX adding a L-rhamnose and a glycerol-2-phosphate, respectively. Wzx flips a single repeat unit into the periplasm, where the Wzy polymerase links the D-glucose at reducing end of the growing chain to the position 4 of the single repeat units' D-galactose, resulting in the mature polysaccharide [Morona, J.K.; Miller, D.C.; Coffey, T.J.; Vindurampulle, C.J.; Spratt, B.G.; Morona, R.; Paton, J.C. Microbiology. 1999, 145(4), 781-789]. Wzg, Wzh, Wzd, and Wze are involved in the modulation of capsule synthesis [Yother, J. Annu. Rev. Microbiol. 2011, 65, 563-581]. Biosynthesis of dTDP-L-rhamnose is achieved via the RmlACBD genes, while CDP-2-glycerol biosynthesis requires Gtp123 [Wang, Q.; Xu, Y.; Perepelov, A.V.; Xiong, W.; Wei, D.; Shashkov, A.S.; Knirel, Y.A.; Feng, L.; Wang, L. J. Bacteriol. 2010, 192(20), 5506-5514]. The serogroup 23 cps loci sequences share the same 18 genes with a varying degree of similarity.

The published structure for the 23F polysaccharide was elucidated by use of chemical and spectroscopic analysis performed on the native and de-phosphorylated polysaccharide and fragments generated by partial hydrolysis and periodate treatment [Richards, J.C.; Perry, M.B. Biochem. Cell Biol. 1988, 66(7), 758-771.]. ¹H NMR assignments were presented for the native and de-phosphorylated polysaccharide, however, the ¹³C NMR spectrum was not assigned. As described in the present patent application, detailed ¹D and ²D ¹H, ¹³C and ³¹P NMR experiments on the 23F polysaccharide were performed in order to make full NMR assignments that were used to facilitate the structural elucidation of the structurally related serotype 23B polysaccharide.

### Summary of the Invention

Serogroup 23 consists of serotypes 23F, 23A and 23 The structure of the pneumococcal capsular polysaccharide serotype 23B was determined using genetic analysis, composition analysis and NMR spectroscopy of polysaccharide 23B. The structure was compared to the serologically and genetically related serotype 23F for which full NMR assignments have been made.

The published structure of the serotype 23F capsular polysaccharide is:

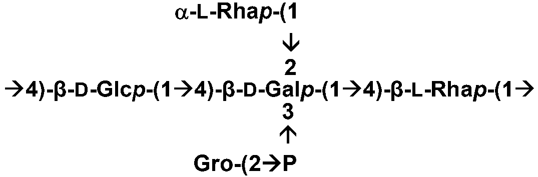

The structure of the serotype 23B capsular polysaccharide of the present invention is:

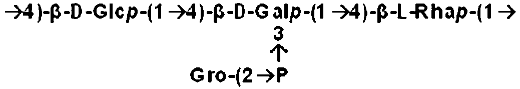

The rhamnosyltransferase WchW, which in 23F and 23A is responsible for the addition of the terminal α-Rha, is present in the 23B cps biosynthesis locus, but apparently inactive. The immunodominant terminal α-Rha of 23F is more sterically crowded in 23A compared to 23F and absent in 23B, which may explain the reported typing cross reactions for serotype 23F: slight with 23A and none with 23B. Determination of the 23B pneumococcal saccharide structure enables the verification of synthetically (e.g. recombinantly)-produced saccharide and allows the design of a glycoengineering strategy which might be needed for bioconjugation. The present invention is directed to synthetic (e.g. recombinant) pneumococcal saccharide comprising one or more repeat unit(s) of the 23B pneumococcal saccharide.

Thus, the present invention provides a synthetic (e.g. recombinant) pneumococcal saccharide comprising one or more repeat unit(s) →4)-β-D-Glc*p*-(1→4)-[Gro-(2→P→3)]-β-D-Gal*p*-(1→4)-β-L-Rha*p*-(1→.

According to a further aspect of the invention, there is provided a bioconjugate comprising a recombinant pneumococcal saccharide of the present invention conjugated to a carrier protein.

According to a further aspect of the invention, there is provided a host cell comprising:
(i) one or more nucleic acids that encode glycosyltransferase(s) sufficient for synthesis of the repeat unit →4)-β-D-Glc*p*-(1→4)-[Gro-(2→P→3)]-β-D-Gal*p*-(1→4)-β-L-Rha*p*-(1→;
(ii) nucleic acid that encodes an oligosaccharyl transferase; and optionally
(iii) nucleic acid that encodes a carrier protein comprising an N-glycosylation consensus sequence (e.g. detoxified exotoxin A from *P. aeruginosa*, CRM197, Diphtheria toxoid, tetanus toxoid, detoxified hemolysin A of *S. aureus*, clumping factor A, clumping factor B, *E. coli* FimH, *E. coli* FimHC, *E. coli* heat labile enterotoxin, detoxified variants of *E. coli* heat labile enterotoxin, Cholera toxin B subunit (CTB), cholera toxin, detoxified variants of cholera toxin, *E. coli* sat protein, the passenger domain of *E. coli* sat protein, *C. jejuni* AcrA, *C. jejuni* natural glycoproteins, *S. pneumoniae* pneumolysin, *S. pneumoniae* NOX, *S. pneumoniae* PspA, *S. pneumoniae* PcpA, *S. pneumoniae* PhtD, *S. pneumoniae* PhtE, *S. pneumoniae* ply (e.g. detoxified ply), or *S. pneumoniae* LytB); and optionally (iv) nucleic acid that encodes a polymerase (e.g. *wzy*).

According to a further aspect of the invention, there is provided a process of producing a bioconjugate that comprises a recombinant pneumococcal saccharide linked to a carrier protein, said process comprising (i) culturing the host cell of the present invention under conditions suitable for the production of glycoproteins and (ii) isolating the bioconjugate. There is further provided a bioconjugate produced by this process.

According to a further aspect of the invention, there is provided an immunogenic composition comprising a pneumococcal saccharide comprising one or more repeat unit(s) →4)-β-D-Glcp-(1→4)-[Gro-(2→P→3-β-D-Galp-(1→4)]-β-L-Rhap-(1→. There is also provided an immunogenic composition comprising a pneumococcal saccharide of the present invention, or a bioconjugate of the present invention. There is also provided an immunogenic composition comprising a pneumococcal saccharide comprising one or more repeat unit(s) →4)-β-D-Glcp-(1→4)-[Gro-(2→P→3-β-D-Galp-(1→4)]-β-L-Rhap-(1→ conjugated to a carrier protein (e.g. selected from detoxified exotoxin A from *P. aeruginosa* (EPA), TT, DT, CRM197, PhtD, detoxified pneumolysin and protein D, suitably exotoxin A from *P. aeruginosa* (EPA)).

According to a further aspect of the invention, there is provided a process of making the immunogenic composition of the present invention, which process comprises the step of mixing the pneumococcal saccharide or the bioconjugate with a pharmaceutically acceptable carrier, diluent or adjuvant.

According to a further aspect of the invention, there is provided a vaccine comprising an immunogenic composition of the present invention.

According to a further aspect of the invention, there is provided a kit comprising a pneumococcal saccharide of the present invention, a bioconjugate of the present invention, or an immunogenic composition of the present invention or a vaccine of the present invention and instructions for the use thereof.

According to a further aspect of the invention, there is provided a pneumococcal saccharide of the present invention, a bioconjugate of the present invention, an immunogenic composition of the present invention or a vaccine of the present invention, for use in the treatment or prevention of a disease caused by *Streptococcus pneumoniae* infection, e.g. pneumonia, invasive pneumococcal disease (IPD), exacerbations of chronic obstructive pulmonary disease (COPD), otitis media, meningitis, bacteraemia, pneumonia and/or conjunctivitis.

According to a further aspect of the invention, there is provided an pneumococcal saccharide of the present invention, a bioconjugate of the present invention, an immunogenic composition of the present invention, or a vaccine of the present invention for use in immunizing against infection by *Streptococcus pneumoniae*.

According to a further aspect of the invention, there is provided a pneumococcal saccharide of the present invention, a bioconjugate of the present invention, an immunogenic composition of the present invention, or a vaccine of the present invention, for inducing an immune response against *Streptococcus pneumoniae*.

According to a further aspect of the invention, there is provided the use of a pneumococcal saccharide of the present invention, a bioconjugate of the present invention, an immunogenic composition of the present invention, or a vaccine of the present invention in the manufacture of a medicament for the treatment or prevention of a disease caused by *Streptococcus pneumoniae* infection, e.g. pneumonia, invasive pneumococcal disease (IPD), exacerbations of chronic obstructive pulmonary disease (COPD), otitis media, meningitis, bacteraemia, pneumonia and/or conjunctivitis.

According to a further aspect of the invention, there is provided the use of a pneumococcal saccharide of the present invention, a bioconjugate of the present invention, an immunogenic composition of the present invention, or a vaccine of the present invention in the manufacture of a medicament for immunizing against infection by *Streptococcus pneumoniae*.

### Description of Figures

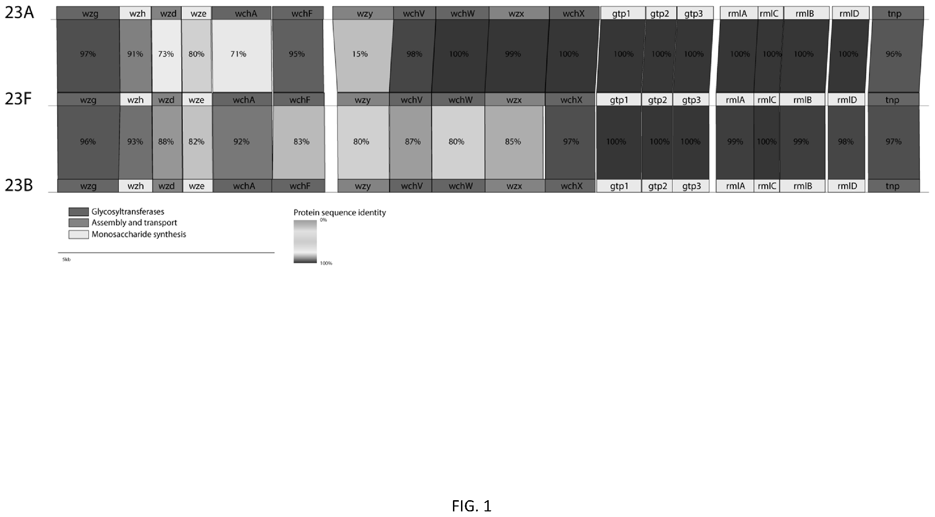

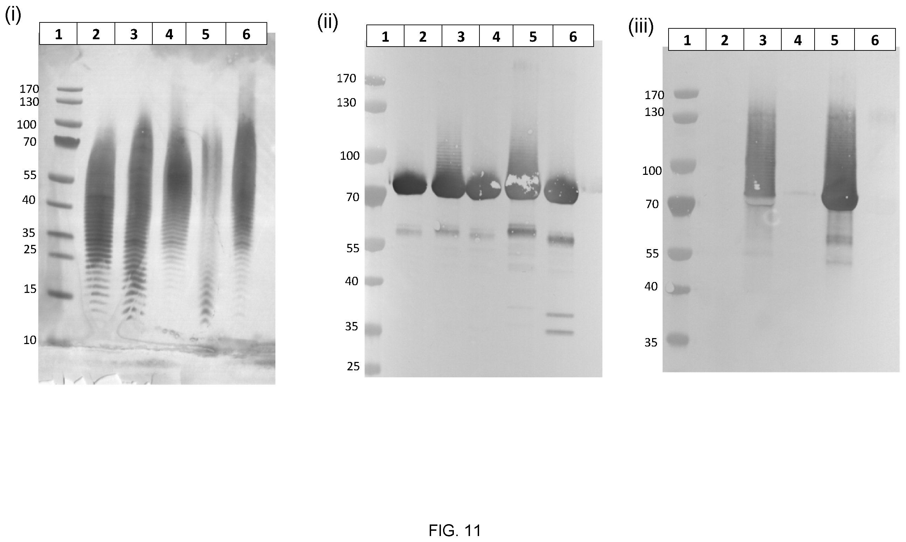

**FIG. 1****: Comparison of serogroup 23 cps loci.** The results of a pairwise BLASTp protein sequence comparison are shown.

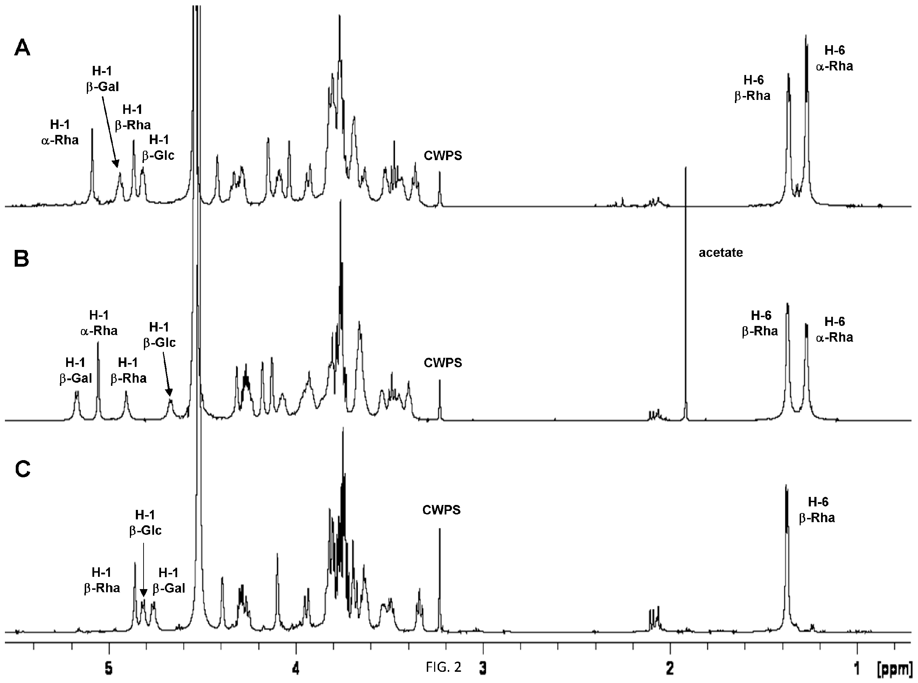

**FIG. 2****: 1D** **¹****H NMR spectra of pneumococcal serotype (A) 23F, (B) 23A and (C) 23B capsular polysaccharides.** Some signals including the diagnostic anomeric and methyl protons are labeled.

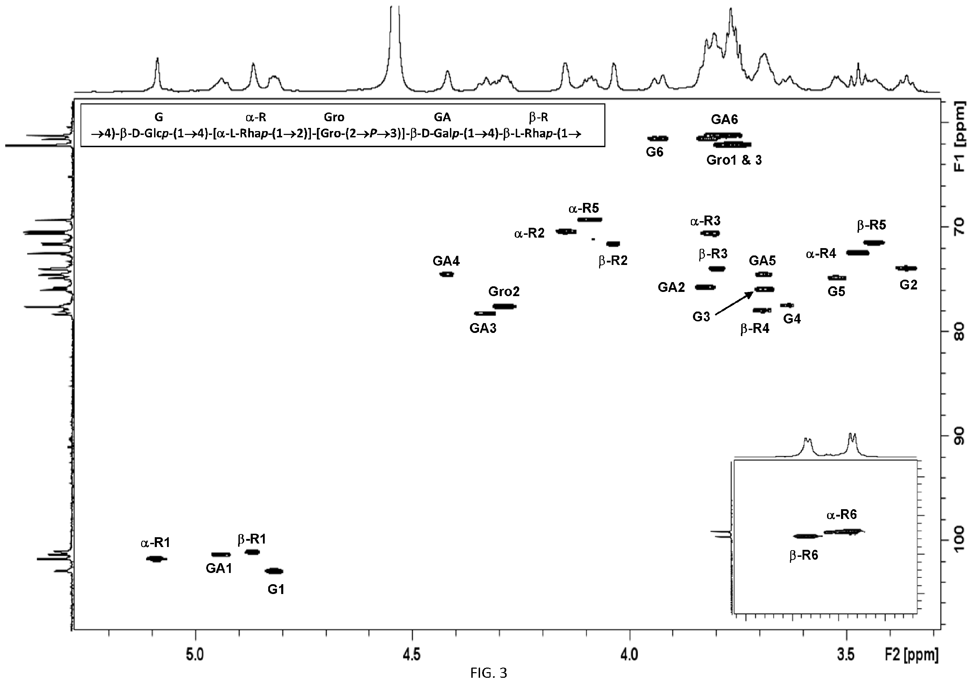

**FIG. 3****: Expansion of the HSQC spectrum of polysaccharide 23F recorded at 600 MHz, the crosspeaks from the methyl region of the spectrum are shown in the insert.** Key tetrasaccharide repeating unit proton/carbon crosspeaks have been labeled according to the carbon atom of the corresponding residue (α- and β-R = α- and β-Rha, G = Glc, GA = Gal and Gro = glycerol).

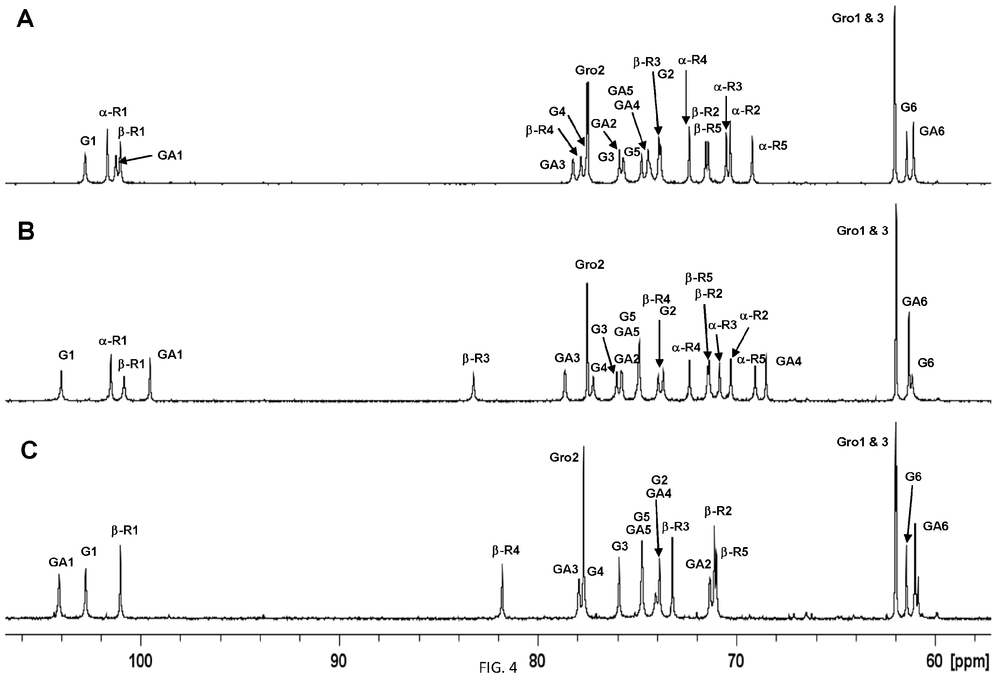

**FIG. 4****: Expansion of the 1D 13C NMR spectra of pneumococcal serotype (A) 23F, (B) 23A and (C) 23B capsular polysaccharides showing the anomeric and ring regions.** Carbon peaks have been labeled according to the corresponding residue (α- and β-R = α- and β-Rha, G = Glc, GA = Gal and Gro = glycerol).

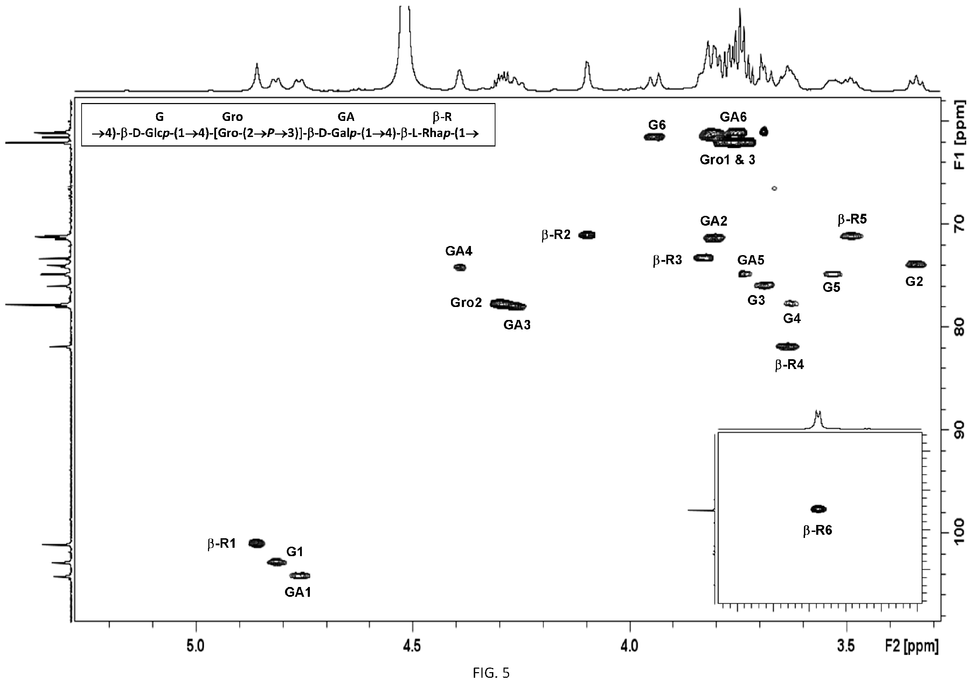

**FIG. 5****: Expansion of the HSQC spectrum of polysaccharide 23B recorded at 600 MHz, the crosspeaks from the methyl region of the spectrum are shown in the insert.** Key trisaccharide repeating unit proton/carbon crosspeaks have been labeled according to the carbon atom of the corresponding residue β-R = β-Rha, G = Glc, GA = Gal and Gro = glycerol).

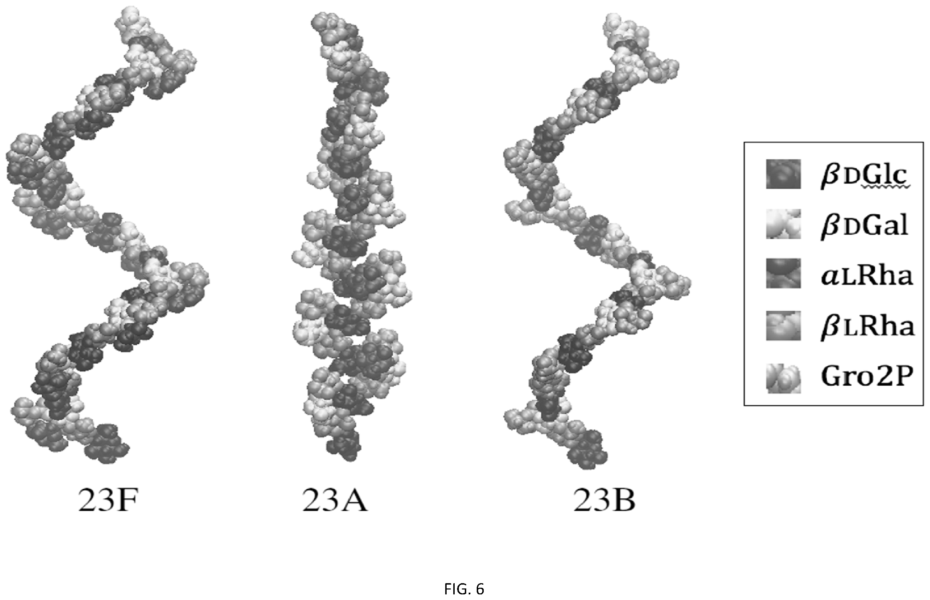

**FIG. 6****: Minimized molecular models for 10RU of 23F (left) 23A (middle) and 23B (right), shown in space-filling representation and colored according to residue type.** The models for 23F and 23B show a very similar loose helical conformation, the model for 23A is a slightly twisted ribbon, with clear steric crowding at the β-L-Rha branch point.

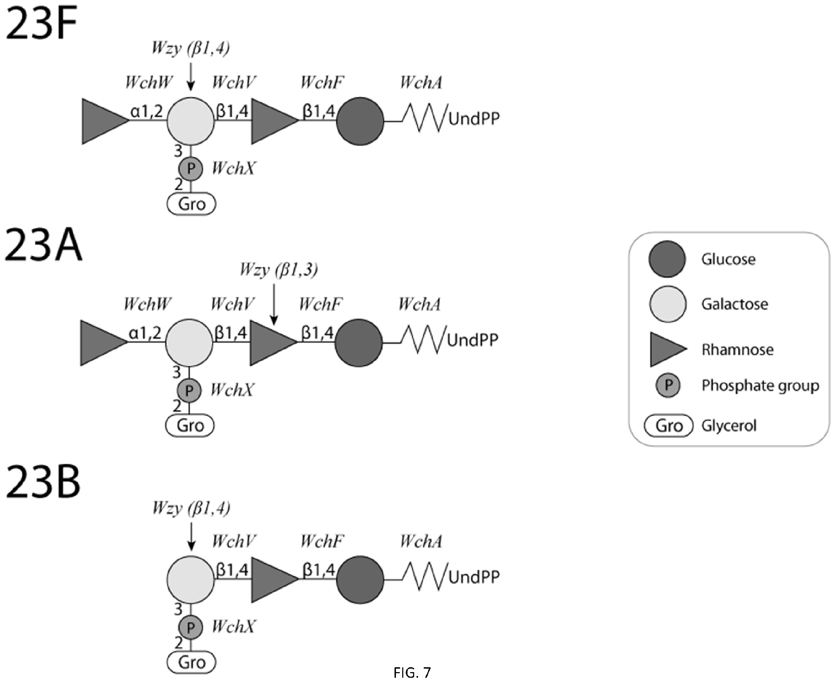

**FIG. 7****: Proposed glycosyltransferase and polymerase activity in serogroup 23 polysaccharides.** Glycosyltransferases responsible for each elongation step are listed above the respective glycosidic linkage in italics. The polymerization site is marked by an arrow.

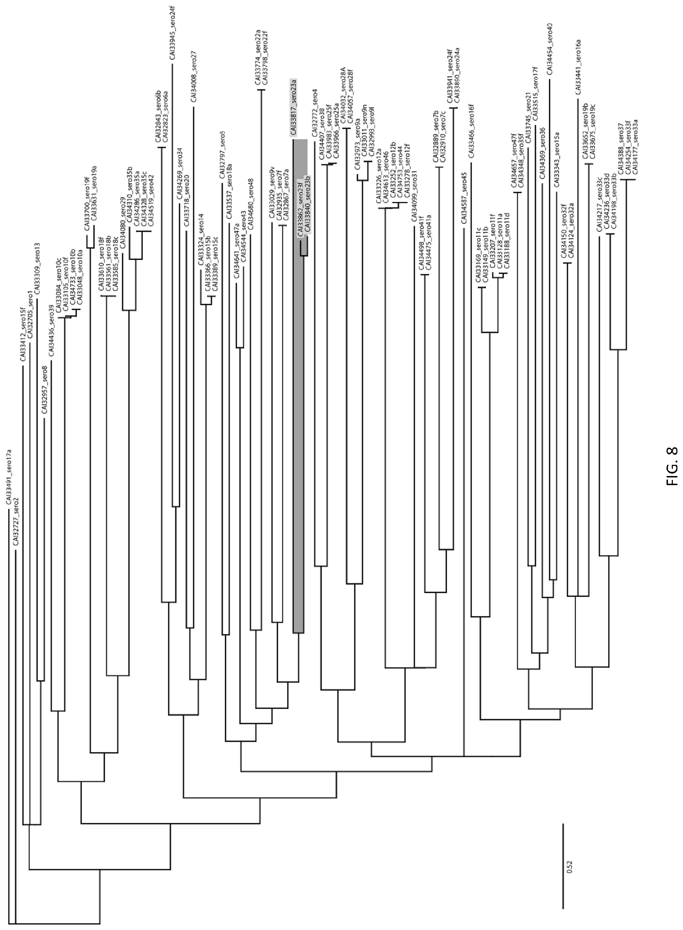

**FIG. 8****:** Unrooted phylogenetic tree of *S. pneumoniae* polymerase Wzy illustrating sequence diversity. Serogroup 23 clade marked in blue. Leaves are labelled with the protein accession number and serogroup.

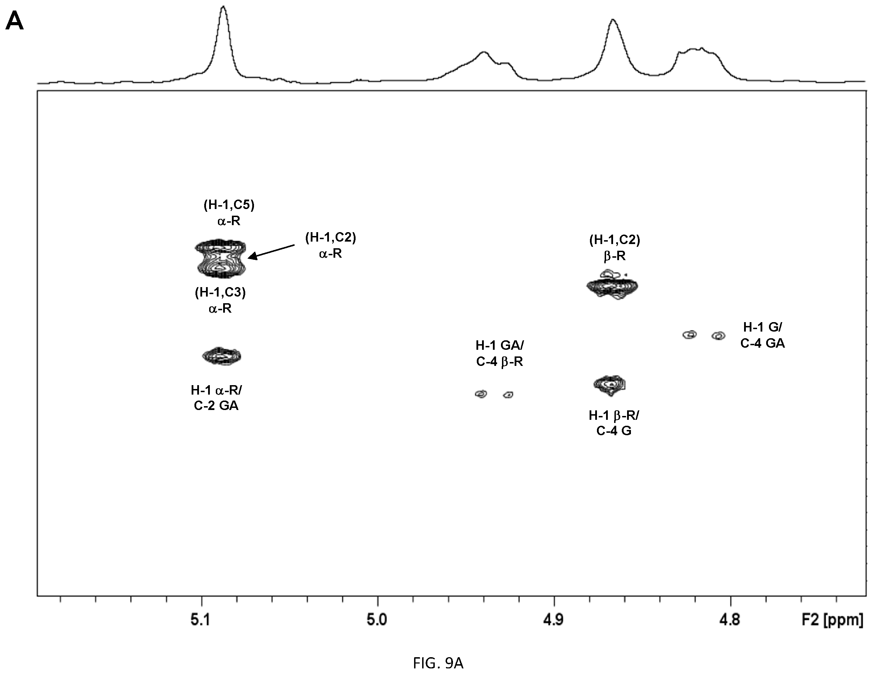

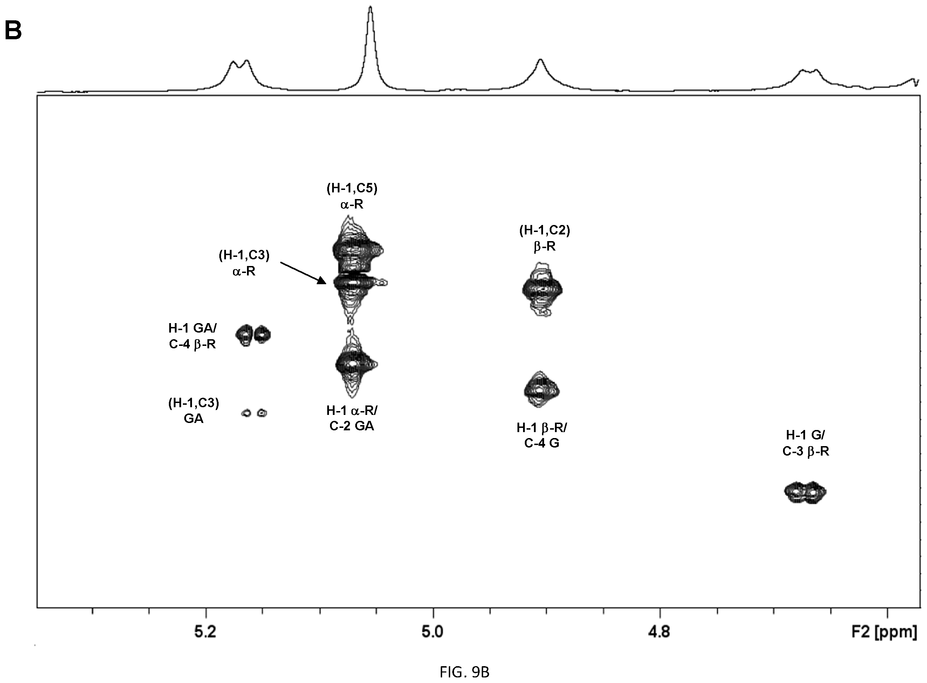

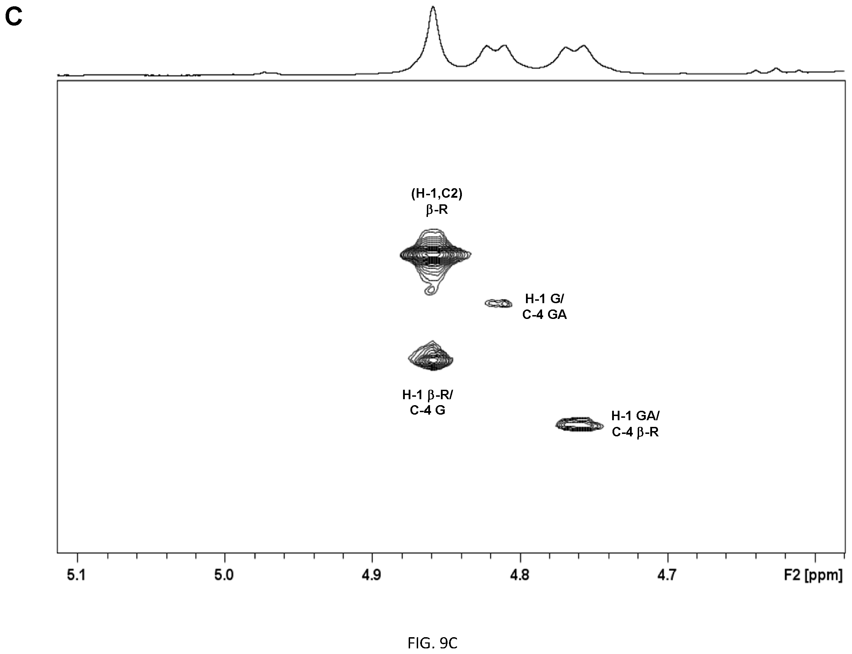

**FIG. 9****:** Expansion of the HMBC spectra of pneumococcal serotype (A) 23F, (B) 23A and (C) 23B capsular polysaccharides showing the anomeric H-1 correlations. Proton/carbon crosspeaks have been labeled according to the corresponding residue (a- and b-R = a- and b-Rha, G = Glc, GA = Gal and Gro = glycerol).

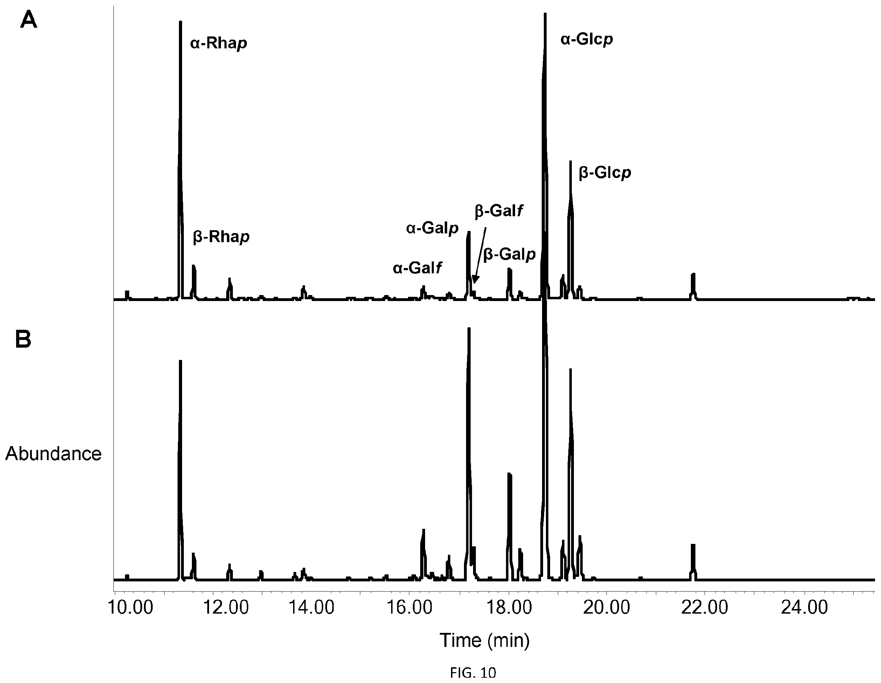

**FIG. 10****:** GC-MS chromatograms of the TMS methyl glycosides of polysaccharide (A) 23A and (B) 23B. Peak identity shown confirmed by retention time and MS.
**FIG 11****:** The LLO samples were analyzed via SDS-PAGE (4-12% NuPAGE Gel, MES Running Buffer, 55min at 200V) followed by transfer onto membrane and anti-23B Western Blot (i). The enriched samples were analyzed via SDS PAGE SDS-PAGE (4-12% NuPAGE Gel, MOPS Running Buffer, 55min at 200V) followed by transfer onto membrane and anti-His (ii) or anti-23A (ii) Western Blot. The volumes corresponding to 0.2 OD600 (i), 0.6 OD600 (ii) and (iii) of culture volume were mixed to loading buffer and loaded. Lane 1: Marker PageRuler from Thermo-Fischer Scientific; lane 2-6 stLMTB13743 transformed with pLMTB3130 (pLAFR^{tet}_Sp15Agtp123) and pLMTB6015 (pEXT22^{Kan}_EPA_rcsA_fepE). Lane 2: additional co-transformed plasmids pGVXN72 (empty pEXT21^{sp}), pLMTB4640 (pDISC^{tmp}_engineering cluster (23AwchX_23AwchV_E. coli O2 wegR_23Awzx)). Lane3: additional plasmids pLMTB4640 and pLMTB4623 (pEXT21^{sp}_pglB_homolog). Lane 4: additional plasmids pLMTB4623 and pGVXN1387 (empt pDISC). Lane 5: additional plasmids pLMTB4640 and pLMTB4620 (pEXT21^{sp}_pglB homolog). Lane 6 additional plasmids pGVYN1387 and pLMTB4620. 23B LLO's are produced in every combination (FIG. 11 (i)).

### Detailed description

### Terminology

Carrier protein: a protein covalently attached to an antigen (e.g. saccharide antigen) to create a conjugate (e.g. bioconjugate). A carrier protein activates T-cell mediated immunity in relation to the antigen to which it is conjugated.

Any amino acid apart from proline (pro, P): refers to an amino acid selected from the group consisting of alanine (ala, A), arginine (arg, R), asparagine (asn, N) , aspartic acid (asp,D), cysteine (cys, C) ,glutamine (gln, Q), glutamic acid (glu, E), glycine (gly, G), histidine (his, H), isoleucine (ile,I), leucine (leu, L), lysine (lys, K), methionine (met, M), phenylalanine (phe, F), serine (ser, S), threonine (thr, T), tryptophan (trp, W), tyrosine (tyr, Y), valine (val, V).
PLY or ply: Pneumolysin from *S. pneumoniae*
CP: Capsular polysaccharide
LPS: lipopolysaccharide.
*wzy*: the polysaccharide polymerase gene encoding an enzyme which catalyzes polysaccharide polymerization. The encoded enzyme transfers the reducing end of the elongating polysaccharide chain onto a single repeat unit forming a glycosidic linkage. .
*waaL*: the O antigen ligase gene encoding a membrane bound enzyme. The encoded enzyme transfers undecaprenyl-pirophosphate (UndPP)-bound O antigen to the lipid A core oligosaccharide, forming a lipopolysaccharide.
Und-PP: undecaprenyl pyrophosphate.
Und-P: undecaprenyl phosphate

Reducing end: the reducing end of an oligosaccharide or polysaccharide is the monosaccharide with a free anomeric carbon that is not involved in a glycosidic bond and is thus capable of converting to the open-chain form.

As used herein, the term "bioconjugate" refers to conjugate between a protein (*e.g.* a carrier protein) and an antigen (e.g. a saccharide) prepared in a host cell background, wherein host cell machinery links the antigen to the protein (*e.g.* N-links).

As used herein, the term "conjugation" is the coupling of carrier protein to saccharide.

As used herein, the term "effective amount," in the context of administering a therapy (*e.g.* an immunogenic composition or vaccine of the invention) to a subject refers to the amount of a therapy which has a prophylactic and/or therapeutic effect(s). In certain embodiments, an "effective amount" refers to the amount of a therapy which is sufficient to achieve one, two, three, four, or more of the following effects: (i) reduce or ameliorate the severity of a bacterial infection or symptom associated therewith; (ii) reduce the duration of a bacterial infection or symptom associated therewith; (iii) prevent the progression of a bacterial infection or symptom associated therewith; (iv) cause regression of a bacterial infection or symptom associated therewith; (v) prevent the development or onset of a bacterial infection, or symptom associated therewith; (vi) prevent the recurrence of a bacterial infection or symptom associated therewith; (vii) reduce organ failure associated with a bacterial infection; (viii) reduce hospitalization of a subject having a bacterial infection; (ix) reduce hospitalization length of a subject having a bacterial infection; (x) increase the survival of a subject with a bacterial infection; (xi) eliminate a bacterial infection in a subject; (xii) inhibit or reduce a bacterial replication in a subject; and/or (xiii) enhance or improve the prophylactic or therapeutic effect(s) of another therapy.

As used herein, the term "subject" refers to an animal, in particular a mammal such as a primate (e.g. human).

As used herein, the term "hexose monosaccharide derivative" refers to a derivative of a hexose monosaccharide that can be a substrate for oligosaccharyl transferase activity. In general, hexose monosaccharide derivatives comprise a monosaccharide comprising an acetamido group at position 2. Exemplary hexose monosaccharide derivatives include GlcNAc, HexNAc, deoxy HexNAc, or 2,4-diacetamido-2,4,6-trideoxyhexose.

As used herein, the term "hybrid oligosaccharide or polysaccharide" refers to an engineered oligosaccharide or polysaccharide that does not comprise a hexose at the reducing end of the first repeat unit, but instead comprises a hexose monosaccharide derivative at the reducing end of the first repeat unit.

As used herein, the term "immunogenic fragment" is a portion of an antigen smaller than the whole, that is capable of eliciting a humoral and/or cellular immune response in a host animal, e.g. human, specific for that fragment. Fragments of a protein can be produced using techniques known in the art, e.g. recombinantly, by proteolytic digestion, or by chemical synthesis. Internal or terminal fragments of a polypeptide can be generated by removing one or more nucleotides from one end (for a terminal fragment) or both ends (for an internal fragment) of a nucleic acid which encodes the polypeptide. Typically, fragments comprise at least 10, 20, 30, 40 or 50 contiguous amino acids of the full length sequence. Fragments may be readily modified by adding or removing 1, 2, 3, 4, 5, 6, 7, 8, 9, 10, 20, 30, 40 or 50 amino acids from either or both of the N and C termini.

As used herein, the term "conservative amino acid substitution" involves substitution of a native amino acid residue with a non-native residue such that there is little or no effect on the size, polarity, charge, hydrophobicity, or hydrophilicity of the amino acid residue at that position, and without resulting in decreased immunogenicity. For example, these may be substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Conservative amino acid modifications to the sequence of a polypeptide (and the corresponding modifications to the encoding nucleotides) may produce polypeptides having functional and chemical characteristics similar to those of a parental polypeptide.

As used herein, the term "deletion" is the removal of one or more amino acid residues from the protein sequence. Typically, no more than about from 1 to 6 residues (e.g. 1 to 4 residues) are deleted at any one site within the protein molecule.

As used herein, the term "insertion" is the addition of one or more non-native amino acid residues in the protein sequence. Typically, no more than about from 1 to 10 residues, (e.g. 1 to 7 residues, 1 to 6 residues, or 1 to 4 residues) are inserted at any one site within the protein molecule.

As used herein, the term "isolated" refers to pneumococcal capsular saccharides substantially free of bacterial cell material or extraction solvent when produced from growing bacterial strains of *Streptococcus pneumoniae*.

As used herein, the term "purified" or "substantially pure" refers to a preparation is at least about 75% pure, preferably at least about 80% pure, and more preferably more than about 80%, 90%, 95% or 99% pure.

As used herein, the term "synthetic" refers to saccharides produced synthetically to have the same or similar structure and/or composition of the *Streptococcus pneumoniae* saccharides disclosed herein, that are not produced in the native host cell (*i.e., S. pneumoniae*). The term "synthetic" includes for example, saccharides produced from techniques such as recombinant DNA technology (*i.e.,* recombinant) and/or chemical synthesis.

As used herein, the term "recombinant" means produced using recombinant DNA technology. In general, the term "recombinant saccharide" refers to a poly- or oligosaccharide produced in a host cell that does not naturally comprise the nucleic acid encoding the glycosyltransferases for producing said poly- or oligosaccharide (*i.e.,* in a host cell other the *Streptococcus pneumoniae*). In the context of the present invention, this term refers to a pnemococcal saccharide produced recombinantly in a prokaryotic host cell, for example, *Escherichia* spp., *Campylobacter* spp., *Salmonella* spp., *Shigella* spp., *Helicobacter* spp., *Pseudomonas* spp., *Bacillus* spp., and in further embodiments *Escherichia coli*, *Campylobacter jejuni*, *Salmonella typhimurium* etc., wherein the nucleic acid encoding the glycosyltransferases to produce said pneumococcal saccharide has been introduced into said host cell.

As used herein the "size" of, for example, a conjugate or pneumococcal saccharide means the Molecular weight (Mw) of the conjugate or pneumococcal saccharide, respectively. The Mw may be provided in kilodaltons (kDa). As used herein "in the range" and "between" are inclusive of the range's upper and lower limits (for example, "in the range 30-300kDa" includes 30 kDa and 300kDa).

### Pneumococcal Saccharide

The present invention provides a synthetic (e.g. recombinant) pneumococcal saccharide comprising one or more repeat unit(s) →4)-β-D-Glc*p*-(1→4)-[Gro-(2→P→3)]-β-D-Gal*p-*(1→4)-β-L-Rha*p*-(1→.

The present invention also provides a synthetic (e.g. recombinant) pneumococcal saccharide comprising one or more repeat unit(s):

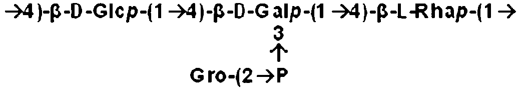

A synthetic (e.g. recombinant) pneumococcal saccharide of the invention may differ from a wild-type (WT) pneumococcal saccharide, e.g. having one or more of the following features: (a) a different number of repeat units (for example, the synthetic (e.g. recombinant pneumococcal saccharide may have fewer repeat units that a WT pneumococcal saccharide), (b) comprising one of more repeat units having a different saccharide structure (for example, the synthetic (e.g. recombinant) pneumococcal saccharide may have a hexose monosaccharide derivative at the reducing end of one or more of the repeat units).

In an embodiment, the pneumococcal saccharide of the present invention, comprises less than 500 repeat units, for example 1 to 200 repeat units (suitably 2 to 200, 1 to 100, 2 to 100, 2 to 50, 2 to 30, 2 to 20 repeat units).

The size (Mw) of the pneumococcal saccharide of the invention is less than 500kDa, for example in the range 1-500kDa, 10-450-kDa, 30-400kDa. In an embodiment, the present invention provides pneumococcal saccharide(s) wherein the size (Mw) of the pneumococcal saccharide is less than 300kDa, or less than 200kDa, for example between 1-150kDa (e.g. between 20-150kDa).

In an embodiment, the pneumococcal saccharide of the present invention is a hybrid oligosaccharide or polysaccharide having a structure:

(B)ₙ-A→

wherein A is an oligosaccharide containing at least 2, 3, 4, 5, 6, 7 or 8 monosaccharides (e.g. 2 to 10, 2 to 8, 3 to 6, 4 to 5 monosaccharides), with a hexose monosaccharide derivative at the reducing end (indicated by the arrow in the diagram), e.g. wherein the hexose monosaccharide derivative is N-acetylglucosamine (GlcNAc), N-acetylgalactoseamine (GalNAc), 2,4-Diacetamido-2,4,6-trideoxyhexose (DATDH). N-acetylfucoseamine (FucNAc), or N-acetylquinovosamine (*QuiNAc*) (e.g. N-acetylglucosamine (GlcNAc));
wherein B is an oligosaccharide repeat unit →4)-β-D-Glc*p*-(1→4)-[Gro-(2→P→3)]-β-D-Gal*p*-(1→4)-β-L-Rha*p*-(1→; and
wherein n is at least 2, 3, 4, 5, 6, 7, 8, 9, 10, 11, 12, 13, 14, 15, 16, 17, 18, 19, or at least 20 (e.g. n is 1 to 100, 2 to 100, 10 to 100, 20 to 100, or 25 to 100).

In an embodiment, A is an oligosaccharide containing no more than 20, 15, 12, 10, 9, 8, 7, 6, or 5 monosaccharides. In an embodiment n is no more than 500, 400, 300, 200, 100, 90, 80, 70, 60, 50, 40, 30, 20, 10 or 5. For example, n may be 1 to 100, 2 to 100, 10 to 100, 20 to 100, or 25 to 100.

In an embodiment, A is an oligosaccharide containing at least 3 monosaccharides. In an embodiment, A is an oligosaccharide containing at least 3 monosaccharides and n is at least 5. In an embodiment, A is an oligosaccharide containing at least 3 monosaccharides and n is at least 20.

In an embodiment, A is an oligosaccharide containing 4 monosaccharides. In an embodiment, A is an oligosaccharide containing 4 monosaccharides and n is at least 5. In an embodiment, A is an oligosaccharide containing 4 monosaccharides and n is at least 20.

In an embodiment, A is an oligosaccharide containing 2-8 monosaccharides. In an embodiment, A is an oligosaccharide containing 2-8 monosaccharides and n is at least 5 and no more than 500. In an embodiment, A is an oligosaccharide containing 2-8 monosaccharides and n is at least 20 and no more than 100.

In an embodiment, A is an oligosaccharide containing 2-10 monosaccharides. In an embodiment, A is an oligosaccharide containing 2-10 monosaccharides and n is at least 5 and no more than 500. In an embodiment, A is an oligosaccharide containing 2-10 monosaccharides and and n is at least 20 and no more than 100.

In an embodiment, A is an oligosaccharide identical to the repeat unit B with the exception that A comprises a hexose monosaccharide derivative at the reducing end of the oligosaccharide in place of the hexose monosaccharide at the reducing end of B (e.g. A is→4)-β-D-GlcNAc*p*-(1→4)-[Gro-(2→P→3)]-β-D-Gal*p*-(1→4)-β-L-Rha*p*-(1→.

In an embodiment, A is an oligosaccharide comprising →4)-β-D-Glc*p*-(1→4)-[Gro-(2→P→3)]-β-D-Gal*p*-(1→4)-β-L-Rha*p*-(1→ and in addition a hexose monosaccharide derivative at the reducing end of the oligosaccharide (e.g. GlcNAc).

In an embodiment, the hexose monosaccharide derivative is any monosaccharide in which C-2 position is modified with an acetamido group. In one aspect, the hexose monosaccharide is selected from the group consisting of glucose, galactose, rhamnose, arabinotol, fucose and mannose (e.g. galactose). Suitable hexose monosaccharide derivatives include N-acetylglucosamine (GlcNAc), N-acetylgalactoseamine (GalNAc), HexNAc, deoxy HexNAc, 2,4-Diacetamido-2,4,6-trideoxyhexose (DATDH), N-acetylfucoseamine (FucNAc), or N-acetylquinovosamine (QuiNAc). A suitable hexose monosaccharide derivative is N-acetylglucosamine (GlcNAc).

Chemical synthesis of the repeat unit →4)-β-D-Glc*p*-(1→4)-[Gro-(2→P→3)]-β-D-Gal*p-*(1→4)-β-L-Rha*p*-(1→ can be performed using synthetic protocols known to the man skilled in the art. For example see the methods for chemical synthesis of pneumococcal polysaccharides disclosed in WO15/004041, WO16/04640, WO16/091399, WO16/198170.

In an embodiment, pneumococcal saccharides of the invention are purified by extracting the pneumococcal saccharides from the bacterial cells by mild acid treatment (e.g. 0.1 % to 5%, suitably 2% acetic acid) under conditions to cleave the polysaccharides from the cell surface material, and purifying the extracted cell surface polysaccharides by centrifugation, size exclusion chromatography and/or anion exchange chromatography.

The present invention also provides a method of identifying a *Streptococcus pneumoniae* bacterium designated *S. pneumoniae* 23B comprising elucidating the chemical structure of the capsular polysaccharide characterized as having the repeat unit →4)-β-D-Glc*p*-(1→4)-[Gro-(2→P→3)]-β-D-Gal*p*-(1→4)-β-L-Rha*p*-(1→, e.g. using NMR.

### Conjugates

The present invention provides a pneumococcal saccharide comprising one or more repeat unit(s) →4)-β-D-Glc*p*-(1→4)-[Gro-(2→P→3)]-β-D-Gal*p*-(1→4)-β-L-Rha*p*-(1→ conjugated to a carrier protein. Suitably, the pneumococcal saccharide comprises less than 500 repeat units, for example 1 to 200 repeat units (suitably 2 to 200, 1 to 100, 2 to 100, 2 to 50, 2 to 30, 2 to 20 repeat units) conjugated to a carrier protein.

The present invention also provides a pneumococcal saccharide of the present invention conjugated to a carrier protein (e.g. detoxified exotoxin A from *P. aeruginosa*, CRM197, Diphtheria toxoid, tetanus toxoid, detoxified hemolysin A of *S. aureus*, clumping factor A, clumping factor B, *E. coli* FimH, *E. coli* FimHC, *E. coli* heat labile enterotoxin, detoxified variants of *E. coli* heat labile enterotoxin, Cholera toxin B subunit (CTB), cholera toxin, detoxified variants of cholera toxin, *E. coli* sat protein, the passenger domain of *E. coli* sat protein, *C. jejuni* AcrA, *C. jejuni* natural glycoproteins, *S. pneumoniae* pneumolysin, *S. pneumoniae* NOX, *S. pneumoniae* PspA, *S. pneumoniae* PcpA, *S. pneumoniae* PhtD, *S. pneumoniae* PhtE, *S. pneumoniae* ply (e.g. detoxified ply), or *S. pneumoniae* LytB). Thus, an aspect of the invention is a conjugate (e.g. bioconjugate) comprising (or consisting of) a pneumococcal saccharide of the invention linked (e.g. covalently linked) to a carrier protein. In an embodiment, the pneumococcal saccharide of the present invention is conjugated to a carrier protein selected from detoxified exotoxin A from *P. aeruginosa* (EPA), TT, DT, CRM197, PhtD, detoxified pneumolysin and protein D. In a further embodiment of the invention, the carrier protein is not an *S. pneumoniae* protein. In a further embodiment, the pneumococcal saccharide of the present invention is conjugated to a carrier protein selected from detoxified exotoxin A from *P. aeruginosa* (EPA), TT, DT, or CRM197. In a further embodiment, the pneumococcal saccharide of the present invention is conjugated to exotoxin A from *P. aeruginosa* (EPA).

In an embodiment, the carrier proteins which may be used in the present invention are TT, DT, CRM197, PhtD, detoxified pneumolysin and protein D. In a further embodiment, each *Streptococcus pneumoniae* capsular saccharide serotype in an immunogenic composition of the invention is conjugated to a carrier protein independently selected from the group consisting of detoxified exotoxin A from *P. aeruginosa* (EPA), TT, DT, CRM197 and protein D. In a further embodiment, each *Streptococcus pneumoniae* capsular saccharide serotype in an immunogenic composition of the invention is conjugated to a carrier protein independently selected from the group consisting of TT, DT, CRM197, PhtD and protein D. In a further embodiment, each *Streptococcus pneumoniae* capsular saccharide serotype is conjugated to a carrier protein independently selected from the group consisting of TT, DT, CRM197 and protein D. In another embodiment, the immunogenic composition of the invention comprises two or more different carrier proteins. In another embodiment, the immunogenic composition of the invention comprises 2, 3, 4, 5 or 6 different carrier proteins.

In an embodiment, the carrier protein is protein D from *Haemophilus influenzae* (PD), for example, protein D sequence from FIG. 9 of EP 0594610 (FIG. 9a and 9b together, 364 amino acids). Inclusion of this protein in the immunogenic composition may provide a level of protection against *Haemophilus influenzae* related otitis media (Pyrmula et. al. Lancet 367; 740-748 (2006)). The Protein D may be used as a full length protein or as a fragment (for example, Protein D may be as described in WO0056360). For example, a protein D sequence may comprise (or consist) a the protein D fragment as described in EP0594610 lacking the 19 N-terminal amino acids from Fig. 9 of EP0594610, optionally with the tripeptide MDP from NS1 fused to the N-terminal of said protein D fragment (348 amino acids). In one aspect, the protein D or fragment of protein D is unlipidated. The protein D could be present in the immunogenic composition as a free protein or as a carrier protein. In one aspect, protein D is present in the immunogenic composition as free protein. In another aspect, protein D is present both as a carrier protein and as free protein. In a further aspect, protein D is present as a carrier protein for one or more of the polysaccharides. In a further aspect, 2-9 of the capsular polysaccharides selected from different serotypes are conjugated to protein D. In a further aspect, protein D is present as a carrier protein for the majority of the polysaccharides, for example 6, 7, 8, 9 or more of the polysaccharides may be conjugated to protein D.

In an embodiment, the carrier protein is CRM197. CRM197 is a non-toxic form of the diphtheria toxin but is immunologically indistinguishable from the diphtheria toxin (DT). Genetically detoxified analogues of diphtheria toxin include CRM197 and other mutants described in US 4,709,017, US 5,843,711, US 5,601,827, and US 5,917,017. CRM197 is produced by *C. diphtheriae* infected by the nontoxigenic phase β197tox- created by nitrosoguanidine mutagenesis of the toxigenic carynephage b (Uchida et al Nature New Biology (1971) 233; 8-11). The CRM197 protein has the same molecular weight as the diphtheria toxin but differs from it by a single base change in the structural gene. This leads to a glycine to glutamine change of amino acid at position 52 which makes fragment A unable to bind NAD and therefore non-toxic (Pappenheimer 1977, Ann Rev, Biochem. 46; 69-94, Rappuoli Applied and Environmental Microbiology Sept 1983 p560-564).

In an embodiment, the carrier protein is Tetanus Toxoid (TT). Tetanus toxin is a single peptide of approximately 150 kDa, which consists of 1315 amino-acid residues. Tetanus-toxin may be cleaved by papain to yield two fragments; one of them, fragment C, is approximately 50 kDa. Fragment C of TT is described in Neubauer et al. Biochim. Biophys.Acta 1981, 27, 141-148.

In an embodiment, the carrier protein is dPly (detoxified pneumolysin). Pneumolysin (Ply) is a multifunctional toxin with a distinct cytolytic (hemolytic) and complement activation activities (Rubins et al., Am. Respi. Cit Care Med, 153:1339-1346 (1996)). The toxin is not secreted by pneumococci, but it is released upon lysis of pneumococci under the influence of autolysin. Its effects include e.g., the stimulation of the production of inflammatory cytokines by human monocytes, the inhibition of the beating of cilia on human respiratory epithelial, the decrease of bactericidal activity and migration of neutrophils, and in the lysis of red blood cells, which involves binding to cholesterol. Because it is a toxin, it needs to be detoxified (*i.e.,* non-toxic to a human when provided at a dosage suitable for protection) before it can be administered in vivo. Expression and cloning of wild-type or native pneumolysin is known in the art. See, for example, Walker et al. (Infect Immun, 55:1184-1189 (1987)), Mitchell et al. (Biochim Biophys Acta, 1007:67-72 (1989) and Mitchell et al (NAR, 18:4010 (1990)). Detoxification of Ply can be conducted by chemical means, e.g., subject to formalin or glutaraldehyde treatment or a combination of both (WO 04081515, PCT/EP2005/010258). Such methods are known in the art for various toxins. Alternatively, Ply can be genetically detoxified. Thus, the invention encompasses derivatives of pneumococcal proteins which may be, for example, mutated proteins. The term "mutated" is used herein to mean a molecule which has undergone deletion, addition or substitution of one or more amino acids using known techniques for site directed mutagenesis or any other conventional method. For example, as described above, a mutant Ply protein may be altered so that it is biologically inactive whilst still maintaining its immunogenic epitopes, see, for example, WO90/06951, Berry et al. (Infect Immun, 67:981-985 (1999)) and WO99/03884. As used herein, it is understood that the term "Ply" encompasses mutated pneumolysin and detoxified pneumolysin (dPly) suitable for medical use (*i.e.,* non toxic).

In an embodiment, the carrier protein is detoxified exotoxin A from *P. aeruginosa* (EPA). In an embodiment, the carrier protein is genetically detoxified Exotoxin A of *Pseudomonas aeruginosa* (US2011/0274720 A1; Ihssen, et al. , (2010) Microbial cell factories 9, 61). For producing a version of EPA that may be glycosylated, the nucleic acids encoding for EPA need to be modified by insertion of glycosylation sites (consensus sequence(s)) as discussed below.

The size (Mw) of the conjugate is greater than 30kDa, for example, a MW in the range 30-300kDa, 30-220kDa, 50-200kDa. In an embodiment, the size (Mw) of the conjugate is less than 300kDa, e.g. between 30-220kDa.

In an embodiment, the pneumococcal saccharide of the present invention is conjugated to a carrier protein wherein the carrier protein is attached to a glucose or hexose monosaccharide derivative (e.g. N-acetylglucosamine) of the pneumococcal saccharide (e.g. the glucose or hexose monosaccharide derivative (e.g. GlcNAc) at the reducing end of the first repeat unit of the pneumococcal saccharide).

In an embodiment, the pneumococcal saccharide of the present invention is covalently coupled to a carrier protein, either directly (e.g. via an N-linked glycosidic bond or via an O-linked glycosidic bond) or via a linker. In an embodiment, the pneumococcal saccharide of the present invention is directly coupled to a carrier protein (e.g. via an N-linked glycosidic bond or via an O-linked glycosidic bond).

In an embodiment, the pneumococcal saccharide of the present invention is coupled to a carrier protein via an O-linked glycosidic bond. O-linked glycosylation is a form of glycosylation that occurs in eukaryotes, archaea, and bacteria. It consists of the attachment of a sugar molecule to a side-chain hydroxyl group of an amino acid residue in the protein target (Faridmoayer et al. JOURNAL OF BACTERIOLOGY, Nov. 2007, Vol. 189, No. 22, p. 8088-8098).

In an embodiment, the pneumococcal saccharide of the present invention is coupled to a carrier protein via an N-linked glycosidic bond. For example, the pneumococcal saccharide of the present invention may be N-linked to a carrier protein. X can be any amino acid except proline. In a conjugate (e.g. bioconjugate) comprising a carrier protein containing a Asn-X-Ser/Thr consensus sequence (e.g. within D/E-X-N-Z-S/T (SEQ ID NO: 1) or K-D/EX-N-Z-S/T-K (SEQ ID NO: 3)), the asparagine residue may be linked to the pneumococcal saccharide. X can be any amino acid except proline. In an embodiment, the consensus sequence is D/E-X-N-Z-S/T (SEQ ID NO: 1), wherein X is Q (glutamine) and Z is A (alanine), e.g. D-Q-N-A-T (SEQ ID NO: 2) also referred to as "DQNAT". In an embodiment, the consensus sequence is K-D/E-X-N-Z-S/T-K (SEQ ID NO: 3), wherein X is Q (glutamine) and Z is A (alanine), e.g. K-D-Q-N-A-T-K (SEQ ID NO: 4) also referred to as "KDQNATK". A further aspect of the invention is a conjugate (e.g. bioconjugate) comprising a carrier protein N-linked to a hybrid oligosaccharide or polysaccharide of the invention, wherein said hydrid oligosaccharide or polysaccharide is identical to a 23B oligosaccharide or polysaccharide, with the exception of the fact that the hybrid oligosaccharide or polysaccharide comprises a hexose monosaccharide derivative at the reducing end of the first repeat unit in addition to comprising all of the monosaccharides of the 23B oligosaccharide or polysaccharide. In an embodiment, a conjugate (e.g. bioconjugate) is provided comprising a carrier protein containing a Asn-X-Ser/Thr consensus sequence (e.g. within D/E-X-N-Z-S/T (SEQ ID NO: 1) or K-D/E-X-N-Z-S/T-K (SEQ ID NO: 3)), the asparagine residue of which is linked to a hybrid oligosaccharide or polysaccharide of the invention, wherein said hybrid oligosaccharide or polysaccharide contains at least 2, 3, 4, 5, 6, 7, 8, 9, 10, 20, 30 , 40 or 50 saccharide repeat units of a 23B oligosaccharide or polysaccharide and a further repeat unit N-linked to the carrier protein in which a hexose monosaccharide derivative is at the reducing end of said further repeat unit.

In an embodiment of the invention, one or more amino acids (e.g. 1-7 amino acids, e.g. one amino acid) of the carrier protein sequence have been substituted by a five amino acid D/E-X-N-Z-S/T (SEQ ID NO: 1) or by a seven amino acid K-D/E-X-N-Z-S/T-K (SEQ ID NO: 3) (e.g. K-D-Q-N-A-T-K (SEQ ID NO: 4) also referred to as "KDQNATK") consensus sequence. For example, a single amino acid in the carrier protein amino acid sequence may be replaced with a D/E-X-N-Z-S/T (SEQ ID NO: 1) or K-D/E-X-N-Z-S/T-K (SEQ ID NO: 3) (e.g. K-D-Q-N-A-T-K (SEQ ID NO: 4)) consensus sequence. Alternatively, 2, 3, 4, 5, 6 or 7 amino acids in the carrier protein amino acid sequence may be replaced with a D/E-X-N-Z-S/T (SEQ ID NO: 1) or K-D/E-X-N-Z-S/T-K (SEQ ID NO: 3) (e.g. K-D-Q-N-A-T-K (SEQ ID NO: 4)) consensus sequence.

In an embodiment, the pneumococcal saccharide of the invention is linked to an amino acid on the carrier protein selected from asparagine, aspartic acid, glutamic acid, lysine, cysteine, tyrosine, histidine, arginine or tryptophan (e.g. asparagine). In another embodiment, the amino acid residue on the carrier protein to which the a pneumococcal saccharide of the invention is linked is an asparagine residue. In another embodiment, the amino acid residue on the carrier protein to which the a pneumococcal saccharide of the invention is linked is part of the D/E-X-N-Z-S/T (SEQ ID NO: 1) and K-D/E-X-N-Z-S/T-K (SEQ ID NO: 3) consensus sequence (e.g. the asparagine in the D/E-X-N-Z-S/T (SEQ ID NO: 1) and K-D/E-X-N-Z-S/T-K (SEQ ID NO: 3) consensus sequence).

In an embodiment, the amino acid residue on the carrier protein to which the pneumococcal saccharide of the invention is linked is not an asparagine residue and in this case, the conjugate is typically produced by chemical conjugation. In an embodiment, the amino acid residue on the carrier protein to which the pneumococcal saccharide of the invention is linked is selected from the group consisting of: Ala, Arg, Asp, Cys, Gly, Glu, Gln, His, Ile, Leu, Lys, Met, Phe, Pro, Ser, Thr, Trp, Tyr, and Val. Optionally, the amino acid is: an amino acid containing a terminal amine group, a lysine, an arginine, a glutaminic acid, an aspartic acid, a cysteine, a tyrosine, a histidine or a tryptophan. Optionally, the pneumococcal saccharide of the invention is covalently linked to amino acid on the carrier protein selected from: aspartic acid, glutamic acid, lysine, cysteine, tyrosine, histidine, arginine or tryptophan.

In an embodiment, the amino acid residue on the carrier protein to which the pneumococcal saccharide of the invention is linked is not part of the D/E-X-N-Z-S/T (SEQ ID NO: 1) and K-D/E-X-N-Z-S/T-K (SEQ ID NO: 3) consensus sequence pneumococcal saccharide of the invention is linked is not the asparagine residue in the D/E-X-N-Z-S/T (SEQ ID NO: 1) and K-D/E-X-N-Z-S/T-K (SEQ ID NO: 3) consensus sequence.

In an embodiment, the pneumococcal saccharide of the present invention is covalently linked (either directly or through a linker) to an amino acid residue of a carrier protein. In an embodiment, the pneumococcal saccharide is covalently linked to a carrier protein through a chemical linkage obtainable using a chemical conjugation method, optionally selected from the group consisting of carbodiimide chemistry, reductive animation, cyanylation chemistry (for example CDAP chemistry), maleimide chemistry, hydrazide chemistry, ester chemistry, and N-hydroxysuccinimide chemistry either directly or via a linker. As used herein, the term "directly linked" means that the two entities are connected via a chemical bond, preferably a covalent bond. As used herein, the term "indirectly linked" means that the two entities are connected via a linking moiety (as opposed to a direct covalent bond). In certain embodiments the linker is adipic acid dihydrazide.

In an embodiment, the chemical conjugation method is selected from the group consisting of carbodiimide chemistry, reductive animation, cyanylation chemistry (for example CDAP chemistry), maleimide chemistry, hydrazide chemistry, ester chemistry, and N-hydroysuccinimide chemistry. Conjugates can be prepared by direct reductive amination methods as described in, US200710184072 (Hausdorff) US 4365170 (Jennings) and US 4673574 (Anderson). Other methods are described in EP-0-161-188, EP-208375 and EP-0-477508. The conjugation method may alternatively rely on activation of the pneumococcal saccharide with 1-cyano-4-dimethylamino pyridinium tetrafluoroborate (CDAP) to form a cyanate ester. Such conjugates are described in PCT published application WO 93/15760 Uniformed Services University and WO 95/08348 and WO 96/29094. See also Chu C. et al. Infect. Immunity, 1983, pages 245-256.

In general the following types of chemical groups on a carrier protein can be used for conjugation:
A) Carboxyl (for instance via aspartic acid or glutamic acid). In one embodiment this group is linked to amino groups on saccharides directly or to an amino group on a linker with carbodiimide chemistry e.g. with EDAC.
B) Amino group (for instance via lysine). In one embodiment this group is linked to carboxyl groups on saccharides directly or to a carboxyl group on a linker with carbodiimide chemistry e.g. with EDAC. In another embodiment this group is linked to hydroxyl groups activated with CDAP or CNBr on saccharides directly or to such groups on a linker; to saccharides or linkers having an aldehyde group; to saccharides or linkers having a succinimide ester group.
C) Sulphydryl (for instance via cysteine). In one embodiment this group is linked to a bromo or chloro acetylated saccharide or linker with maleimide chemistry. In one embodiment this group is activated/modified with bis diazobenzidine.
D) Hydroxyl group (for instance via tyrosine). In one embodiment this group is activated/modified with bis diazobenzidine.
E) Imidazolyl group (for instance via histidine). In one embodiment this group is activated/modified with bis diazobenzidine.
F) Guanidyl group (for instance via arginine).
G) Indolyl group (for instance via tryptophan).

On a saccharide, in general the following groups can be used for a coupling: OH, COOH or NH₂. Aldehyde groups can be generated after different treatments such as: periodate, acid hydrolysis, hydrogen peroxide, etc.

### Direct coupling approaches:

Saccharide-OH + CNBr or CDAP -----> cyanate ester + NH₂-Protein ----> conjugate
Saccharide-aldehyde + NH₂-Protein ----> Schiff base + NaCNBH3 ----> conjugate
Saccharide-COOH + NH₂-Protein + EDAC ----> conjugate
Saccharide-NH₂ + COOH-Protein + EDAC ----> conjugate

### Indirect coupling via spacer (linker) approaches:

Saccharide-OH + CNBr or CDAP ---> cyanate ester + NH₂----NH₂ ----> saccharide----NH₂ + COOH-Protein + EDAC -----> conjugate
Saccharide-OH + CNBr or CDAP ----> cyanate ester + NH₂-----SH -----> saccharide----SH + SH-Protein (native Protein with an exposed cysteine or obtained after modification of amino groups of the protein by SPDP for instance) -----> saccharide-S-S-Protein
Saccharide-OH + CNBr or CDAP ---> cyanate ester + NH₂----SH -------> saccharide----SH + maleimide-Protein (modification of amino groups) ----> conjugate
Saccharide-OH + CNBr or CDAP ---> cyanate ester + NH₂-----SH ---> Saccharide-SH + haloacetylated-Protein ----> Conjugate
Saccharide-COOH + EDAC + NH₂-----NH₂ ---> saccharide------NH₂ + EDAC + COOH-Protein ----> conjugate
Saccharide-COOH + EDAC+ NH₂----SH -----> saccharide----SH + SH-Protein (native Protein with an exposed cysteine or obtained after modification of amino groups of the protein by SPDP for instance) -----> saccharide-S-S-Protein
Saccharide-COOH + EDAC+ NH₂----SH -----> saccharide----SH + maleimide-Protein (modification of amino groups) ----> conjugate
Saccharide-COOH + EDAC + NH₂----SH ---> Saccharide-SH + haloacetylated-Protein ----> Conjugate
Saccharide-Aldehyde + NH₂-----NH₂ ----> saccharide---NH2 + EDAC + COOH-Protein ----> conjugate
Note: instead of EDAC above, any suitable carbodiimide may be used.

In an embodiment, the pneumococcal saccharide of the invention is attached to the carrier protein via a linker. Optionally, the linker is selected from the group consisting of linkers with 4-12 carbon atoms, bifunctional linkers, linkers containing 1 or 2 reactive amino groups at the end, B-proprionamido, nitrophenyl-ethylamine, haloacyl halides, 6-aminocaproic acid and ADH. The activated saccharide may thus be coupled directly or via a spacer (linker) group to an amino group on the carrier protein. For example, the spacer could be cystamine or cysteamine to give a thiolated polysaccharide which could be coupled to the carrier protein via a thioether linkage obtained after reaction with a maleimide-activated carrier protein (for example using GMBS (4-Maleimidobutyric acid N-hydroxysuccinimide ester)) or a haloacetylated carrier protein (for example using SIAB (succinimidyl (4-iodoacetyl)aminobenzoate), or SIA (succinimidyl iodoacetate), or SBAP (succinimidyl-3-(bromoacetamide)propionate)). In an embodiment, the cyanate ester (optionally made by CDAP chemistry) is coupled with hexane diamine or ADH (adipic acid dihydrazide) and the amino-derivatised saccharide is conjugated to the carrier protein using carbodiimide (e.g. 1-Ethyl-3-(3-dimethylaminopropyl)carbodiimide (EDAC or EDC)) chemistry via a carboxyl group on the protein modified pneumolysin. Such conjugates are described in PCT published application WO 93/15760 Uniformed Services University and WO 95/08348 and WO 96/29094.

### Host cell

The present invention provides a host cell comprising:
(i) one or more nucleic acids that encode glycosyltransferase(s) sufficient for synthesis of the repeat unit →4)-β-D-Glc*p*-(1→4)-[Gro-(2→P→3)]-β-D-Gal*p*-(1→4)-β-L-Rha*p*-(1→;
(ii) nucleic acid that encodes an oligosaccharyl transferase (optionally a heterologous oligosaccharyltransferase); and optionally
(iii) nucleic acid that encodes a carrier protein comprising an N-glycosylation consensus sequence (e.g. detoxified exotoxin A from *P. aeruginosa*, CRM197, Diphtheria toxoid, tetanus toxoid, detoxified hemolysin A of *S. aureus*, clumping factor A, clumping factor B, *E. coli* FimH, *E. coli* FimHC, *E. coli* heat labile enterotoxin, detoxified variants of *E. coli* heat labile enterotoxin, Cholera toxin B subunit (CTB), cholera toxin, detoxified variants of cholera toxin, *E. coli* sat protein, the passenger domain of *E. coli* sat protein, *C. jejuni* AcrA, *C. jejuni* natural glycoproteins, *S. pneumoniae* pneumolysin, *S. pneumoniae* NOX, *S. pneumoniae* PspA, *S. pneumoniae* PcpA, *S. pneumoniae* PhtD, *S. pneumoniae* PhtE, *S. pneumoniae* ply (e.g. detoxified ply), or *S. pneumoniae* LytB); and optionally (iv) nucleic acid that encodes a polymerase (e.g. *wzy*).

Host cells that can be used to produce the bioconjugates of the invention, include archea, prokaryotic host cells, and eukaryotic host cells. Exemplary prokaryotic host cells for use in production of the bioconjugates of the invention, without limitation, *Escherichia* species, *Shigella* species, *Klebsiella* species, *Xhantomonas* species, *Salmonella* species, *Yersinia* species, *Lactococcus* species, *Lactobacillus* species, *Pseudomonas* species, *Corynebacterium* species, *Streptomyces* species, *Streptococcus* species, *Staphylococcus* species, *Bacillus* species, and *Clostridium* species. In an embodiment, the host cell is a gram-negative bacterium, optionally selected from the group consisting of *Escherichia* species, *Shigella* species, *Klebsiella* species, *Salmonella* species, *Yersinia* species, *Neisseria* species, *Vibrio* species and *Pseudomonas* species. In an embodiment, the host cell is *E. coli* (e.g. *E. coli* W3110).

In an embodiment, the host cells used to produce the bioconjugates of the invention are engineered to comprise heterologous nucleic acids, e.g. heterologous nucleic acids that encode one or more carrier proteins and/or heterologous nucleic acids that encode one or more proteins, e.g. genes encoding one or more proteins. Thus, a host cell of the invention is not a wild-type (WT) *Steptococcus pneumoniae* host cell. In an embodiment, heterologous nucleic acids that encode proteins involved in glycosylation pathways (e.g. prokaryotic and/or eukaryotic glycosylation pathways) may be introduced into the host cells of the invention. Such nucleic acids may encode proteins including, without limitation, oligosaccharyl transferases, epimerases, flippases, polymerases, and/or glycosyltransferases. Heterologous nucleic acids (e.g. nucleic acids that encode carrier proteins and/or nucleic acids that encode other proteins, e.g. proteins involved in glycosylation) can be introduced into the host cells of the invention using methods such as electroporation, chemical transformation by heat shock, natural transformation, phage transduction, and conjugation. In an embodiment, heterologous nucleic acids are introduced into the host cells of the invention using a plasmid, e.g. the heterologous nucleic acids are expressed in the host cells by a plasmid (e.g. an expression vector). In an embodiment, heterologous nucleic acids are introduced into the host cells of the invention using the method of insertion described in International Patent application NO: PCT/EP2013/068737 (published as WO 14/037585).

In an embodiment, additional modifications may be introduced (e.g. using recombinant techniques) into the host cells of the invention. For example, host cell nucleic acids (e.g. genes) that encode proteins that form part of a possibly competing or interfering glycosylation pathway (e.g. compete or interfere with one or more heterologous genes involved in glycosylation that are recombinantly introduced into the host cell) can be deleted or modified in the host cell background (genome) in a manner that makes them inactive/dysfunctional (*i.e.,* the host cell nucleic acids that are deleted/modified do not encode a functional protein or do not encode a protein whatsoever). In an embodiment, when nucleic acids are deleted from the genome of the host cells of the invention, they are replaced by a desirable sequence, e.g. a sequence that is useful for glycoprotein production.

Exemplary genes that can be deleted in host cells (and, in some cases, replaced with other desired nucleic acid sequences) include genes of host cells involved in glycolipid biosynthesis, such as *waaL* (see, e.g. Feldman et al. 2005, PNAS USA 102:3016-3021), the lipid A core biosynthesis cluster (*waa*), galactose cluster (*gal*), arabinose cluster (*ara*), colanic acid cluster (*wca*), capsular polysaccharide cluster, undecaprenyl-pyrophosphate biosynthesis genes (e.g*. uppS* (Undecaprenyl pyrophosphate synthase)*, uppP* (Undecaprenyl diphosphatase)), Und-P recycling genes, metabolic enzymes involved in nucleotide activated sugar biosynthesis, enterobacterial common antigen cluster, and prophage O antigen modification clusters like the *gtrABS* cluster. In an embodiment, the *waaL* gene is deleted. In another embodiment, colanic acid cluster (*wca*) genes (e.g. the complete colanic acid cluster from *wza* to *wcaM*) are replaced by the glycosyltransferase(s) sufficient for synthesis of the repeat unit →4)-β-D-Glc*p*-(1→4)-[Gro-(2→P→3)]-β-D-Gal*p*-(1→4)-β-L-Rha*p*-(1→. In another embodiment, the *yeaS* gene of *E. coli* W3110 is replaced with a gene encoding a glycosyltransferase which transfers a β-L-rhamnose on position 4 of UndPP-D-Glc*p*NAc, e.g. *wegR* from the O-antigen cluster of *E. coli* O2(e.g. SEQ ID NO: 59). WegR can be added to a strain as a plasmid-encoded gene, but in order to obtain a more stable production strain, *wegR* may be integrated into the host cell genome replacing the gene *yeaS*.

Such a modified prokaryotic host cell comprises nucleic acids encoding enzymes capable of producing a bioconjugate comprising a pneumococcal saccharide of the invention attached to a carrier protein. Such host cells may naturally express nucleic acids specific for production of a saccharide antigen, or the host cells may be made to express such nucleic acids, *i.e.,* in certain embodiments said nucleic acids are heterologous to the host cells. In certain embodiments, one or more of said nucleic acids specific for production of a saccharide antigen are heterologous to the host cell and intergrated into the genome of the host cell. In certain embodiments, the host cells of the invention comprise nucleic acids encoding additional enzymes active in the N-glycosylation of proteins, *e.g.* the host cells of the invention further comprise nucleic acid encoding an oligosaccharyl transferase and/or one or more nucleic acids encoding other glycosyltransferases.

Nucleic acid sequences comprising capsular polysaccharide gene clusters can be inserted into the host cells of the invention. In an embodiment, the capsular polysaccharide gene cluster inserted into a host cell of the invention is a capsular polysaccharide gene cluster from an *E. coli* strain, a *Streptococcus* strain (e.g. *S. pneumoniae*, *S. pyrogenes*, *S. agalacticae*), a *Staphylococcus* strain (e.g. *S. aureus*), or a *Burkholderia* strain (e.g. *B mallei, B. pseudomallei, B. thailandensis*). Disclosures of methods for making such host cells which are capable of producing bioconjugates are found in WO 06/119987, WO 09/104074, WO 11/62615, WO 11/138361, WO 14/57109, WO14/72405 and WO16/20499*.*

In an embodiment, the host cell is capable of producing a hybrid oligosaccharide or polysaccharide, wherein said hybrid oligosaccharide or polysaccharide is identical to *S. pneumoniae* CP23B, with the exception of the fact that said hybrid oligosaccharide or polysaccharide comprises a hexose monosaccharide derivative at the reducing end of the first repeat unit in place of the hexose monosaccharide normally present at the reducing end of the first repeat unit of *S. pneumoniae* CP23B, e.g. wherein said hexose monosaccharide derivative is N-acetylglucosamine (GlcNAc), N-acetylgalactoseamine (GalNAc), 2,4-Diacetamido-2,4,6-trideoxyhexose (DATDH), N-acetylfucoseamine (FucNAc), N-acetylquinovosamine (QuiNAc) (e.g. N-acetylglucosamine (GlcNAc)).

### Glycosylation Machinery

The host cells of the invention comprise, and/or can be modified to comprise, nucleic acids that encode genetic machinery (e.g. glycosyltransferases, flippases, polymerases, and/or oligosaccharyltransferases) capable of producing hybrid oligosaccharides and/or polysaccharides, as well as genetic machinery capable of linking a pneumococcal saccharide of the invention to a carrier protein.

The capsular polysaccharide gene cluster maps between dexB and aliA in the pneumococcal chromosome (Llull et al., 1999, J. Exp. Med. 190, 241-251). There are typically four relatively conserved genes: (*wzg*), (*wzh*), (*wzd*), (*wze*) at the 5' end of the capsular polysaccharide gene cluster (Jiang et al., 2001, Infect. Immun. 69, 1244-1255). Also included in the capsular polysaccharide gene cluster of *S. pneumoniae* are wzx (polysaccharide flippase gene) and *wzy* (polysaccharide polymerase gene). The CP gene clusters of all 90 *S. pneumoniae* serotypes have been sequenced by Sanger Institute (WorldWideWeb(www).sanger.ac.uk/Projects/S_pneumoniae/CPS/), and *wzx* and *wzy* of 89 serotypes have been annotated and analyzed (Kong et al., 2005, J. Med. Microbiol. 54, 351-356). The capsular biosynthetic genes of *S. pneumoniae* are further described in Bentley et al. (PLoS Genet. 2006 Mar; 2(3): e31 and the sequences are provided in GenBank (Genbank CR931632-CR931722). Serotype 23A from Streptococcus pneumoniae strain 1196/45 (serotype 23A) is accession number: CR931683.1. Serotype 23B from Streptococcus pneumoniae strain 1039/41 is accession number: CR931684.1. Serotype 23F from Streptococcus pneumoniae strain Dr. Melchior is accession number: CR931685.1.

### Phosphotransferases and Glycosyltransferases

The host cells of the invention comprise nucleic acids that encode phosphotransferases and glycosyltransferases that produce an oligosaccharide or polysaccharide repeat unit. In an embodiment, said repeat unit does not comprise a hexose at the reducing end, and said oligosaccharide or polysaccharide repeat unit is derived from a →4)-β-D-Glc*p*-(1→4)-[Gro-(2→P→3)]-β-D-Gal*p*-(1→4)-β-L-Rha*p*-(1→ repeat unit that comprises a hexose (*i.e.,* Glc; glucose) at the reducing end.

In an embodiment, the host cells of the invention may comprise nucleic acid that encodes an enzyme, e.g. a phosphotransferase that assembles a hexose monosaccharide derivative onto undecaprenyl monophopshpate (Und-P) to form a hexose monosaccharide derivative assembled on Und-PP (Und-PP precursor). In one aspect, the phosphotransferase that assembles a hexose monosaccharide derivative onto Und-PP is heterologous to the host cell and/or heterologous to one or more of the genes that encode the glycosyltransferase(s). Said phosphotransferase can be derived from, e.g. *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species*.* For example, GlcNAc may be assembled on UndP from UDP-GlcNAc by a phosphotransferase, e.g. *wecA* (which exists in all Gram-negative bacteria that synthesize ECA and Gram-positive bacteria that makes Teichoic acid) (Annu Rev Microbiol. 2013;67:313-36; Glycobiology. 2011 Feb;21(2):138-51) to make a β (1,3) linkage and form Und-PP-GlcNAc. In an embodiment, the phosphotransferase that assembles a hexose monosaccharide derivative onto Und-PP is *wecA*, optionally from *E. coli* (Genbank Accession AAA82970 (nucleotide sequence) AAA24526 (amino acid sequence) SEQ IDNO:63).

In an embodiment, the host cell comprises nucleic acids that encode (a) an enzyme, e.g. a phophotransferase that assembles a hexose monosaccharide derivative onto undecaprenyl monophosphate (Und-P), optionally wherein said phosphotransferaseis wecA, optionally from *E. coli*, and (b) one or more glycosyltransferases capable of adding a monosaccharide to the hexose monosaccharide derivative assembled on Und-PP.

In an embodiment, the host cells of the invention may comprise nucleic acids that encode one or more glycosyltransferases capable of adding a monosaccharide to the Und-PP precursor, (the hexose monosaccharide derivative assembled on Und-PP), for example a rhamnosyltransferase (e.g. 4-linked β-L-rhamnose), for example the rhamnosyltransferase (*wegR*) from *E. coli* O2 (e.g. SEQ ID NO: 59) or the rhamnosyltransferase (*wbuV*) from E. *coli* O149 (e.g. SEQ ID NO: 60) or the rhamnosyltransferase (*wepI*) from *Cronobacter sakazakii* O5 (e.g. SEQ ID NO: 61). In an embodiment, said one or more glycosyltransferases capable of adding a monosaccharide to the hexose monosaccharide derivative comprise the rhamnosyltransferase (*wegR*) from *E. coli* O2 having an amino acid sequence of SEQ ID NO: 59 (GenBank: EU549863.1) or an amino acid sequence at least 80%, 85%, 90%, 92%, 95%, 96%, 97%, 98% or 99% identical to SEQ ID NO: 59, for example comprising at least 100, 150 or 200 contiguous amino acids of the full length sequence. In an embodiment, said one or more glycosyltransferases capable of adding a monosaccharide to the hexose monosaccharide derivative comprise the rhamnosyltransferase (*wbuV*) from *E. coli* O149 having an amino acid sequence of SEQ ID NO: 60 (GenBank: DQ868764.1) or an amino acid sequence at least 80%, 85%, 90%, 92%, 95%, 96%, 97%, 98% or 99% identical to SEQ ID NO: 60, for example comprising at least 100, 150 or 200 contiguous amino acids of the full length sequence. In an embodiment, said one or more glycosyltransferases capable of adding a monosaccharide to the hexose monosaccharide derivative comprise the rhamnosyltransferase (*wepI*) from *Cronobacter sakazakii* O5 having an amino acid sequence of SEQ ID NO: 61 (GenBank: JQ674748.1) or an amino acid sequence at least 80%, 85%, 90%, 92%, 95%, 96%, 97%, 98% or 99% identical to SEQ ID NO: 61, for example comprising at least 100, 150 or 200 contiguous amino acids of the full length sequence.

In an embodiment, a host cell of the invention comprises nucleic acids encoding glycosyltransferases for synthesis of the repeat unit from the capsular polysaccharide gene clusters of *S. pneumoniae* CP23A, CP23B, and/or CP23F. In an embodiment, a host cell of the invention comprises nucleic acids encoding glycosyltransferases for synthesis of the →4)-β-D-Glc*p*-(1→4)-[Gro-(2→P→3)]-β-D-Gal*p*-(1→4)-β-L-Rha*p*-(1→repeat unit from the capsular polysaccharide gene clusters of *S. pneumoniae* CP23B. In an embodiment, said glycosyltransferases for synthesis of the repeat units comprise *WchA* (glucosyltransferase) (e.g. from *S. pneumoniae* CP23A, CP23B or CP23F, in particular from CP23A), *WchF* (rhamnosyltransferase) (e.g. from *S. pneumoniae* CP23A, CP23B or CP23F, in particular from CP23B), *WchV* (galactosyltransferase) (e.g. from *S. pneumoniae* CP23A, CP23B or CP23F, in particular from CP23B), and *WchX* (e.g. from *S. pneumoniae* CP23A, CP23B or CP23F, in particular from CP23B). In an embodiment, said glycosyltransferases for synthesis of the repeat units comprise *WchA* (glucosyltransferase) (e.g. from *S. pneumoniae* CP23A, CP23B or CP23F, in particular from CP23B), e.g. having an amino acid sequence of SEQ ID NO: 27 (GenBank: CR931684.1) or an amino acid sequence at least 80%, 85%, 90%, 92%, 95%, 96%, 97%, 98% or 99% identical to SEQ ID NO: 27, for example comprising at least 100, 150 or 200 contiguous amino acids of the full length sequence. In an embodiment, said glycosyltransferases for synthesis of the repeat units comprise *WchF* (rhamnosyltransferase) (e.g. from *S. pneumoniae* CP23A, CP23B or CP23F, in particular from CP23B), e.g. from *S. pneumoniae* CP23B having an amino acid sequence of SEQ ID NO: 28 (GenBank: CR931684.1) or an amino acid sequence at least 80%, 85%, 90%, 92%, 95%, 96%, 97%, 98% or 99% identical to SEQ ID NO: 28, for example comprising at least 100, 150 or 200 contiguous amino acids of the full length sequence. In an embodiment, said glycosyltransferases for synthesis of the repeat units comprise *WchV (galactosyltransferase)*, (e.g. from *S. pneumoniae* CP23A, CP23B or CP23F, in particular from CP23B), e.g. from *S. pneumoniae* CP23B having an amino acid sequence of SEQ ID NO: 30 (GenBank: CR931684.1) or an amino acid sequence at least 80%, 85%, 90%, 92%, 95%, 96%, 97%, 98% or 99% identical to SEQ ID NO: 30, for example comprising at least 100, 150 or 200 contiguous amino acids of the full length sequence. In an embodiment, said glycosyltransferases for synthesis of the repeat units comprise *WchW* (rhamnosyltransferase), (e.g. from *S. pneumoniae* CP23A, CP23B or CP23F, in particular from CP23B), e.g. from *S. pneumoniae* CP23B having an amino acid sequence of SEQ ID NO: 31 (GenBank: CR931684.1) or an amino acid sequence at least 80%, 85%, 90%, 92%, 95%, 96%, 97%, 98% or 99% identical to SEQ ID NO: 31, for example comprising at least 100, 150 or 200 contiguous amino acids of the full length sequence. In an embodiment, said glycosyltransferases for synthesis of the repeat units do not comprise *WchW* (rhamnosyltransferase), (e.g. from *S. pneumoniae* CP23A, CP23B or CP23F, in particular from CP23B), e.g. from *S. pneumoniae* CP23B having an amino acid sequence of SEQ ID NO: 31 (GenBank: CR931684.1) or an amino acid sequence at least 80%, 85%, 90%, 92%, 95%, 96%, 97%, 98% or 99% identical to SEQ ID NO: 31, for example comprising at least 100, 150 or 200 contiguous amino acids of the full length sequence. In an embodiment, said glycosyltransferases for synthesis of the repeat units comprise *WchX* , (e.g. from *S. pneumoniae* CP23A, CP23B or CP23F, in particular from CP23B), e.g. from *S. pneumoniae* CP23B having an amino acid sequence of SEQ ID NO: 33 (GenBank: CR931684.1) or an amino acid sequence at least 80%, 85%, 90%, 92%, 95%, 96%, 97%, 98% or 99% identical to SEQ ID NO: 33, for example comprising at least 100, 150 or 200 contiguous amino acids of the full length sequence.

In one embodiment, the glycosyltransferases that assemble the →4)-β-D-Glc*p*-(1→4)-[Gro-(2→P→3-β-D-Gal*p*-(1→4)]-β-L-Rhap-(1→ repeat unit onto the hexose monosaccharide derivative comprise a glycosyltransferase that is capable of adding the monosaccharide that is adjacent to the hexose monosaccharide present at the reducing end of the first repeat unit of the donor oligosaccharide or polysaccharide to the hexose monosaccharide present at the reducing end of the first repeat unit of the pneumococcal saccharide.

In an embodiment, a host cell of the invention also comprises nucleic acids encoding *wzg*, *wzh*, *wzd* and/or *wze* from *S. pneumoniae* CP23A, CP23B or CP23F, in particular from CP23B, e.g. having amino acid sequences of SEQ ID NOs. 23, 24, 25 and 26 respectively (GenBank: CR931684.1) or amino acid sequences at least 80%, 85%, 90%, 92%, 95%, 96%, 97%, 98% or 99% identical to SEQ ID NOs. 23, 24, 25 and 26, for example comprising at least 100, 150 or 200 contiguous amino acids of the full length sequence. The genes *wzg*, *wzh*, *wzd* and *wze* from *S. pneumoniae* are regulatory genes.

In another embodiment, the host cells of the invention lack one or more of the regulatory genes from *Streptococcus pnuemoniae* that are involved in biosynthesis of capsular polysaccharide 23B. Thus, in an embodiment, the host cells of the invention do not comprise nucleic acid that encodes one or more of these regulatory gene from a *Streptococcus pneumoniae* that are involved in polysaccharide biosynthesis of capsular polysaccharide 23B. The present inventors have found that sufficient yield of capsular polysaccharide 23B may be produced without these regulatory genes.

In an embodiment, a host cell of the invention does not comprise nucleic acids encoding *wzg*, *wzh*, *wzd* or *wze* from *S. pneumoniae* CP23A, CP23B or CP23F, in particular from CP23B, e.g. amino acid sequences of SEQ ID NOs. 23, 24, 25 and 26 respectively (GenBank: CR931684.1). In an embodiment, a host cell of the invention does not comprise nucleic acid encoding wzg from *S. pneumoniae* CP23A, CP23B or CP23F, in particular from CP23B, e.g. amino acid sequence of SEQ ID NO. 23 (GenBank: CR931684.1). In an embodiment, a host cell of the invention does not comprise nucleic acid encoding wzh from *S. pneumoniae* CP23A, CP23B or CP23F, in particular from CP23B, e.g. amino acid sequence of SEQ ID NO. 24 respectively (GenBank: CR931684.1). In an embodiment, a host cell of the invention does not comprise nucleic acid encoding *wzd* from *S. pneumoniae* CP23A, CP23B or CP23F, in particular from CP23B, e.g. amino acid sequence of SEQ ID NO. 25 respectively (GenBank: CR931684.1). In an embodiment, a host cell of the invention does not comprise nucleic acids encoding *wze* from *S. pneumoniae* CP23A, CP23B or CP23F, in particular from CP23B, e.g. amino acid sequence of SEQ ID NO. 26 respectively (GenBank: CR931684.1).

In an embodiment, the host cell of the invention may comprise nucleic acid sequence encoding genes from the CDP-glycerol pathway for biosynthesis of capsular polysaccharide 23B: *gtp1*, *gtp2* and *gtp3*. In an embodiment, the host cell of the invention may comprise nucleic acid sequence encoding a *gtp1* (e.g. from *S. pneumoniae* CP23B) having an amino acid sequence of SEQ ID NO: 34 (GenBank: CR931684.1) or an amino acid sequence at least 80%, 85%, 90%, 92%, 95%, 96%, 97%, 98% or 99% identical to SEQ ID NO: 34, for example comprising at least 100, 150 or 200 contiguous amino acids of the full length sequence. In an embodiment, the host cell of the invention may comprise nucleic acid sequence encoding a *gtp2* (e.g. from *S. pneumoniae* CP23B) having an amino acid sequence of SEQ ID NO: 35 (GenBank: CR931684.1) or an amino acid sequence at least 80%, 85%, 90%, 92%, 95%, 96%, 97%, 98% or 99% identical to SEQ ID NO: 35, for example comprising at least 100, 150 or 200 contiguous amino acids of the full length sequence. In an embodiment, the host cell of the invention may comprise nucleic acid sequence encoding a *gtp3* (e.g. from *S. pneumoniae* CP23B) having an amino acid sequence of SEQ ID NO: 36 (GenBank: CR931684.1) or an amino acid sequence at least 80%, 85%, 90%, 92%, 95%, 96%, 97%, 98% or 99% identical to SEQ ID NO: 36, for example comprising at least 100, 150 or 200 contiguous amino acids of the full length sequence. In an embodiment, the host cell of the invention may comprise nucleic acid sequence encoding genes from the dTDP-L-rhamnose pathway for biosynthesis of capsular polysaccharide 23B: *rmlA*, *rmlB*, *rmlC* and *rmlD*. In an embodiment, the host cell of the invention may comprise nucleic acid sequence encoding a *rmlA* (e.g. from *S. pneumoniae* CP23B) having an amino acid sequence of SEQ ID NO: 37 (GenBank: CR931684.1) or an amino acid sequence at least 80%, 85%, 90%, 92%, 95%, 96%, 97%, 98% or 99% identical to SEQ ID NO: 37, for example comprising at least 100, 150 or 200 contiguous amino acids of the full length sequence. In an embodiment, the host cell of the invention may comprise nucleic acid sequence encoding a *rmlC* (e.g. from *S. pneumoniae* CP23B) having an amino acid sequence of SEQ ID NO: 38 (GenBank: CR931684.1) or an amino acid sequence at least 80%, 85%, 90%, 92%, 95%, 96%, 97%, 98% or 99% identical to SEQ ID NO: 38, for example comprising at least 100, 150 or 200 contiguous amino acids of the full length sequence. In an embodiment, the host cell of the invention may comprise nucleic acid sequence encoding a *rmlB* (e.g. from *S. pneumoniae* CP23B) having an amino acid sequence of SEQ ID NO: 39 (GenBank: CR931684.1) or an amino acid sequence at least 80%, 85%, 90%, 92%, 95%, 96%, 97%, 98% or 99% identical to SEQ ID NO: 39, for example comprising at least 100, 150 or 200 contiguous amino acids of the full length sequence. In an embodiment, the host cell of the invention may comprise nucleic acid sequence encoding a *rmlD* (e.g. from *S. pneumoniae* CP23B) having an amino acid sequence of SEQ ID NO: 40 (GenBank: CR931684.1) or an amino acid sequence at least 80%, 85%, 90%, 92%, 95%, 96%, 97%, 98% or 99% identical to SEQ ID NO: 40, for example comprising at least 100, 150 or 200 contiguous amino acids of the full length sequence.

In a embodiment, the host cell of the present invention comprises *S. pneumoniae* 23B capsular polysaccharide genes from wchA to rmlD (see GenBank: CR931683). The *S. pneumoniae* 23B capsular polysaccharide genes from wchA to rmlD (GenBank: CR931683) may be cloned into a plasmid, e.g. pDOC, which acts as a donor for the replacement of the complete colanic acid cluster (from wza to wcaM) with the 23B cluster. For the replacement a helper plasmid may be used, e.g. pTKRED (GenBank: GU327533.1), which has been mutagenized so that nucleotides A9477 and G9478 have been replaced by C and A, respectively, translating in a V to A mutation in the RepA protein to ensure better temperature sensitivity of the replicon.

In an embodiment, the host cell of the present invention is engineered so that it produces a repeat unit with a trisaccharide backbone (Glc-Gal-βRha) without a terminal α-Rha. In an embodiment, *wchA* attaches a D-glucose-P onto UndP, followed by *WchF* attaching a L-rhamnose onto UndPP-Glucose, followed by *WchV* attaching a D-galactose, followed by *WchX* attaching a glycerol-2-phosphate to the galactose. In an embodiment *Wzx* (e.g. *wzx* flippase from *S. pneumoniae* CP23B) flips a single repeat unit into the periplasm, followed by the *Wzy* polymerase attaching the D-glucose at reducing end of the growing chain to the D-galactose of the single repeating unit, to produce a polysaccharide.

In an embodiment, a plasmid may be used to produce the CP23B engineered subunit in the cytoplasm, from which it may be translocated into the periplasm by the flippase of CP23B wherein wild type oligosaccharide or polysaccharide can be assembled on it by action of CP23B polymerase (*wzy*).

Further details on the synthesis of capsular polysaccharides can be found in WO2014/072405A1 and further details on glycoengineering can be found in WO2016/020499A2.

### Oligosaccharyltransferases

Oligosaccharyltransferases (OSTs) are membrane-embedded enzymes that transfer oligosaccharides from a lipid carrier to a nascent polypeptide (a type of glycosyltransferase).

N-linked protein glycosylation, the addition of carbohydrate molecules to an asparagine residue in the polypeptide chain of the target protein, is the most common type of post-translational modification occurring in the endoplasmic reticulum of eukaryotic organisms. The process is accomplished by the enzymatic oligosaccharyltransferase complex (OST) responsible for the transfer of a preassembled oligosaccharide from a lipid carrier (e.g. undecaprenyl pyrophosphate or dolichyl pyrophosphate) to an asparagine residue of a nascent protein within the conserved sequence Asn-X-Ser/Thr (where X is any amino acid except proline) in the Endoplasmic reticulum.

In N-linked protein glycosylation, oligosaccharyl transferases transfer lipid-linked oligosaccharides to asparagine residues of nascent polypeptide chains that comprise a N-glycosylation consensus motif, e.g. Asn-X-Ser(Thr), wherein X can be any amino acid except Pro; or Asp(Glu)-X-Asn-Z-Ser(Thr) (SEQ ID NO: 1), wherein X and Z are independently selected from any natural amino acid except Pro (see WO 2006/119987). See, e.g. WO 2003/074687 and WO 2006/119987, the disclosures of which are herein incorporated by reference in their entirety. It has been shown that a bacterium, the food-borne pathogen *Campylobacter jejuni*, can also N-glycosylate its proteins (Wacker et al. Science. 2002; 298(5599):1790-3) due to the fact that it possesses its own glycosylation machinery. The machinery responsible of this reaction is encoded by a cluster called "pgl" (for protein glycosylation).

The *C. jejuni* glycosylation machinery can be transferred to *E. coli* to allow for the glycosylation of recombinant proteins expressed by the *E. coli* cells. Previous studies have demonstrated how to generate *E. coli* strains that can perform N-glycosylation (see, e.g. Wacker et al. Science. 2002; 298 (5599):1790-3; Nita-Lazar et al. Glycobiology. 2005; 15(4):361-7; Feldman et al. Proc Natl Acad Sci U S A. 2005; 102(8):3016-21; Kowarik et al. EMBO J. 2006; 25(9):1957-66; Wacker et al. Proc Natl Acad Sci U S A. 2006; 103(18):7088-93; International Patent Application Publication Nos. WO2003/074687, WO2006/119987, WO 2009/104074, and WO/2011/06261, and WO2011/138361).

In an embodiment, the host cells of the invention comprise nucleic acid that encodes an oligosaccharyl transferase. The nucleic acid that encodes an oligosaccharyl transferase can be native to the host cell, or can be introduced into the host cell using genetic approaches, as described above. In an embodiment, the oligosaccharyl transferase is heterologous to the host cell. In an embodiment, the oligosaccharyl transferase is an oligosaccharyl transferase from *Campylobacter*, e.g. *Campylobacter jejuni* (*i.e., pglB*; see, e.g. Wacker et al. 2002, Science 298:1790-1793; see also, e.g. NCBI Gene ID: 3231775, UniProt Accession NO: O86154). In an embodiment, the oligosaccharyl transferase is an oligosaccharyl transferase from *Campylobacter lari* (see, e.g. NCBI Gene ID: 7410986)*.* In an embodiment, the host cell of the present invention comprises nucleic acid encoding *pglB*, for example from from *C. jejuni*, in particular from *pglB* having amino acid sequence of SEQ ID NOs. 62 (GenBank: AF108897.1) or amino acid sequences at least 80%, 85%, 90%, 92%, 95%, 96%, 97%, 98% or 99% identical to SEQ ID NO: 62, for example comprising at least 100, 150 or 200 contiguous amino acids of the full length sequence. In another embodiment, the host cell of the present invention comprises nucleic acid encoding pglB, for example from from *C. jejuni*, in particular from pglB having amino acid sequence of SEQ ID NOs. 65 (GenBank: WP_087705088.1) or amino acid sequences at least 80%, 85%, 90%, 92%, 95%, 96%, 97%, 98% or 99% identical to SEQ ID NO: 65, for example comprising at least 100, 150 or 200 contiguous amino acids of the full length sequence. In another embodiment, the host cell of the present invention comprises nucleic acid encoding pglB, for example a homolog of pglB from *C. jejuni*, in particular a pglB homolog having amino acid sequence of SEQ ID NOs. 65 (GenBank: WP_087705088.1) or amino acid sequences at least 30% identical to SEQ ID NO: 65.

Optionally, the nucleic acid encoding the oligosaccharyl transferase is integrated into the host cell genome and optionally at least one gene of the host cell has been functionally inactivated or deleted, optionally the *waaL* gene of the host cell has been functionally inactivated or deleted, optionally the *waaL* gene of the host cell has been replaced by nucleic acid encoding an oligosaccharyltransferase.

In an embodiment, the host cells of the invention comprise nucleic acid sequence encoding an oligosaccharyl transferase, wherein said nucleic acid sequence encoding an oligosaccharyl transferase (*e.g. pglB*) is integrated into the genome of the host cell.

In an embodiment, provided herein is a modified prokaryotic host cell comprising (i) a glycosyltransferase derived from an capsular polysaccharide cluster from *S. pneumoniae*, wherein said glycosyltransferase is integrated into the genome of said host cell; (ii) nucleic acid encoding an oligosaccharyl transferase (*e.g. pglB*), wherein said nucleic acid encoding an oligosaccharyl transferase is integrated into the genome of the host cell; and (iii) a carrier protein, wherein said carrier protein is either plasmid-borne or integrated into the genome of the host cell. There is also provided a method of making a modified prokaryotic host cell comprising (i) integrating a glycosyltransferase derived from an capsular polysaccharide cluster from *S. pneumoniae* into the genome of said host cell; (ii) integrating nucleic acid encoding an oligosaccharyl transferase (*e.g. pglB* from *Campylobacter jejuni*) into the genome of the host cell; and (iii) integrating into a host cell a carrier protein either plasmid-borne or integrated into the genome of the host cell.

### Polymerases

In an embodiment, a polymerase (*e.g. wzy*) is introduced into a host cell of the invention (*i.e.,* the polymerase is heterologous to the host cell). Thus a host cell of the invention may comprise nucleic acid encoding a heterologous polymerase. In an embodiment, the polymerase is a bacterial polymerase. In an embodiment, the polymerase is a capsular polysaccharide polymerase (*e.g. wzy*) or an O antigen polymerase (*e.g. wzy*). In an embodiment, the polymerase is a capsular polysaccharide polymerase e.g. from *S. pneumoniae*.

In an embodiment, the polymerase introduced into the host cells of the invention is the *wzy* gene from a capsular polysaccharide gene cluster of *S. pneumoniae* CP1, CP2, CP4, CP5, CP6(A,B,C,D), CP7 (A,B, C), CP8, CP9(A,L,N,V), CP10(A,B,C,F), CP11(A, B,C,D,F), CP12(A,B,F), CP13, CP14 CP15(A,B,C,F), CP16(A,F), CP17(A,F), CP18(A,B,C,F), CP19(A,B,C,F), CP20, CP21, CP22(A,F), CP23(A,B,F), CP24(A,B,F), CP25(A,F), CP26, CP27,CP28(A,F), CP29, CP31, CP32(A,F), CP33(A,B,C,D,F), CP34, CP35(A,B,C,D,F), CP36, CP38, CP39, CP40, CP41(A,F), CP42, CP43, CP44, CP45, CP46, CP47(A,F) or CP48. For example, a host cell of the invention may comprise nucleic acid encoding a *wzy* polymerase from *S. pneumoniae* CP23A, CP23B or CP23F, in particular from CP23B having an amino acid sequence of SEQ ID NO: 29 (GenBank: CR931684.1) or an amino acid sequence at least 80%, 85%, 90%, 92%, 95%, 96%, 97%, 98% or 99% identical to SEQ ID NO: 29, for example comprising at least 100, 150 or 200 contiguous amino acids of the full length sequence.

Other polymerases that can introduced into the host cells of the invention are from *S. pneumoniae* described in Bentley SD, Aanensen DM, Mavroidi A, Saunders D, Rabbinowitsch E, Collins M, Donohoe K, Harris D, Murphy L, Quail MA et al: Genetic analysis of the capsular biosynthetic locus from all 90 pneumococcal serotypes. PLoS genetics 2006, 2(3):e31) (Genbank CR931632-CR931722).

In an embodiment, said *wzy* polymerase is incorporated (e.g. inserted into the genome of or plasmid expressed by) in said host cell as part of a *S. pneumoniae* capsular polysaccharide cluster, wherein said *S. pneumoniae* capsular polysaccharide cluster has been modified to comprise the *wzy* polymerase. In an embodiment, nucleic acid encoding the *S. pneumoniae wzy* polymerase is inserted into and expressed by the host cells of the invention.

### Flippases

In an embodiment, a flippase (*wzx*) is introduced into a host cell of the invention (*i.e.,* the flippase is heterologous to the host cell). Thus, a host cell of the invention may comprise nucleic acid encoding a heterologous flippase. In an embodiment, the flippase is a bacterial flippase. Flippases translocate lipid-linked wild type repeat units and/or their corresponding engineered (hybrid) repeat units from the cytoplasm into the periplam of host cells (e.g. *E. coli*). Thus, a host cell of the invention may comprise nucleic acid that encodes a flippase (*wzx*).

In an embodiment, a flippase of a capsular polysaccharide biosynthetic pathway of *S. pneumoniae* is introduced into a host cell of the invention. In certain embodiments, the flippase introduced into the host cells of the invention is the *wzx* gene from a capsular polysaccharide gene cluster of *S. pneumoniae* CP1, CP2, CP4, CP5, CP6(A,B,C,D), CP7(A,B,C), CP8, CP9(A,L,N,V), CP10(A,B,C,F), CP11(A,B,C,D,F), CP12(A,B,F), CP13, CP14 CP15(A,B,C,F), CP16(A,F), CP17(A,F), CP18(A,B,C,F), CP19(A,B,C,F), CP20, CP21, CP22(A,F), CP23(A,B,F), CP24(A,B,F), CP25(A,F), CP26, CP27, CP28(A,F), CP29, CP31, CP32(A,F), CP33(A,B,C,D,F), CP34, CP35(A,B,C,D,F), CP36, CP38, CP39, CP40, CP41(A,F), CP42, CP43, CP44, CP45, CP46, CP47(A,F), or CP48. In an embodiment, the flippase introduced into the host cells of the invention is the *wzx* gene from *S. pneumoniae* CP23A, CP23B or CP23F, in particular from CP23B having an amino acid sequence of SEQ ID NO: 32 or an amino acid sequence at least 80%, 85%, 90%, 92%, 95%, 96%, 97%, 98% or 99% identical to SEQ ID NO: 32, for example comprising at least 100, 150 or 200 contiguous amino acids of the full length sequence.

In an embodiment, the flippase (e.g. Wzx flippase from *S. pneumoniae* CP23B) flips a single repeat unit into the periplasm, followed by the polymerase attaching the D-glucose at reducing end of the growing chain to the β-L-rhamnose of the single repeat unit, to produce an oligosaccharide or polysaccharide.

Other flippases that can be introduced into the host cells of the invention are for example from *Campylobacter jejuni* (e.g. *pglK*). Other flippases that can introduced into the host cells of the invention are from *S. pneumoniae* described in Bentley SD, Aanensen DM, Mavroidi A, Saunders D, Rabbinowitsch E, Collins M, Donohoe K, Harris D, Murphy L, Quail MA et al. "Genetic analysis of the capsular biosynthetic locus from all 90 pneumococcal serotypes" PLoS genetics 2006, 2(3):e31) (Genbank CR931632-CR931722).

### Enzymes That Modify Monosaccharides

### Accessory Enzymes

In an embodiment, nucleic acids encoding one or more accessory enzymes are introduced into the host cells of the invention. Thus, a host cell of the invention may further comprise one or more of these accessory enzymes. Such nucleic acids encoding one or more accessory enzymes can be either plasmid-borne or integrated into the genome of the host cells of the invention. Exemplary accessory enzymes include, without limitation, epimerases, branching, modifying (e.g. to add cholins, glycerolphosphates, pyruvates), amidating, chain length regulating, acetylating, formylating, polymerizing enzymes. For example, in one embodiment the the host cell of the present invention comprises nucleic acid encoding a chain length regulator, for example *fepE* (ferric enterobactin (Enterochelin) transporter) from *Salmonella typhimurium* having amino acid sequence of SEQ ID NO. 66 (WP_001139584.1) or amino acid sequences at least 80%, 85%, 90%, 92%, 95%, 96%, 97%, 98% or 99% identical to SEQ ID NO: 66, for example comprising at least 100, 150 or 200 contiguous amino acids of the full length sequence.

In certain embodiments, enzymes that are capable of modifying monosaccharides are introduced into a host cell of the invention (*i.e.,* the enzymes that are capable of modifying monosaccharides are heterologous to the host cell). Such enzymes include, e.g. epimerases and racemases. Thus, a host cell of the invention may further comprise nucleic acid encoding an epimerase and/or racemase.

In an embodiment, the epimerases and racemases are from bacteria. In certain embodiments, the epimerases and/or racemases introduced into the host cells of the invention are from *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species. In certain embodiments, the epimerase inserted into a host cell of the invention is an epimerase described in International Patent Application Publication No. WO2011/062615.

In an embodiment, a host cell of the invention further comprises RcsA (an activator of CP synthesis). RcsA is an unstable positive regulator required for the synthesis of colanic acid capsular polysaccharide in *Escherichia coli*. (GenBank Accession M58003 (nucleotide sequence), AAA82970 (amino acid sequence) SEQ ID NO:64)

### Genetic Background

Exemplary host cells that can be used to generate the host cells of the invention include, without limitation, *Escherichia* species, *Shigella* species, *Klebsiella* species, *Xhantomonas* species, *Salmonella* species, *Yersinia* species, *Lactococcus* species, *Lactobacillus* species, *Pseudomonas* species, *Corynebacterium* species, *Streptomyces* species, *Streptococcus* species, *Staphylococcus* species, *Bacillus* species, and *Clostridium* species. In an embodiment, the host cell used herein is *E. coli*.

In an embodiment, the host cell genetic background is modified by, e.g. deletion of one or more genes. Exemplary genes that can be deleted in host cells (and, in some cases, replaced with other desired nucleic acid sequences) include genes of host cells involved in glycolipid biosynthesis, such as *waaL* (see, e.g. Feldman *et al.* 2005, PNAS USA 102:3016-3021), the O antigen cluster (*rfb* or *wb*), enterobacterial common antigen cluster (*wec*), the lipid A core biosynthesis cluster (*waa*), and prophage O antigen modification clusters like the *gtrABS* cluster. In an embodiment, one or more of the *waaL* gene, *gtrA* gene, *gtrB* gene, *gtrS* gene, or a gene or genes from the *wec* cluster or a gene or genes from the *rfb* gene cluster are deleted or functionally inactivated from the genome of a prokaryotic host cell of the invention. In one embodiment, a host cell used herein is *E. coli*, wherein the *waaL* gene, *gtrA* gene, *gtrB* gene, *gtrS* gene are deleted or functionally inactivated from the genome of the host cell. In another embodiment, a host cell used herein is *E. coli*, wherein the *waaL* gene and *gtrS* gene are deleted or functionally inactivated from the genome of the host cell. In another embodiment, a host cell used herein is *E. coli*, wherein the *waaL* gene and genes from the wec cluster are deleted or functionally inactivated from the genome of the host cell.

### Benefits

The host cells of the invention are of particular commercial importance and relevance, as they allow for large scale fermentation of bioconjugates comprising saccharide, for example, *Streptococcus* antigens that can be used as therapeutics (e.g. in immunogenic compositions, vaccines), at a lower risk due to the increased stability of the chromosomally inserted DNA and thus expression of the DNA of interest during fermentation. The host cells of the invention are advantageous over host cells that rely on plasmid borne expression of nucleic acids required for generation of the bioconjugates of the invention because, *inter alia*, antibiotic selection during fermentation is not required once the heterologous DNA is inserted into the host cell genome. That is, when the insert DNA is inserted in the chromosome, it doesn't need to be selected for, because it is propagated along with replication of the host genome. Further, it is a disadvantage in plasmid borne systems that with every generation (*i.e.,* cycle of host cell replication) the risk for losing the plasmid increases. This loss of plasmid is due to the sometimes inappropriate distribution of plasmids to daughter cells at the stage of cell separation during cell division. At large scale, bacterial cell cultures duplicate more often than in smaller fermentation scales to reach high cell densities. Thus, higher cell stability and insert DNA expression leads to higher product yields, providing a distinct advantage. Cell stability is furthermore a process acceptance criteria for approval by regulatory authorities, while antibiotic selection is generally not desired during fermentation for various reasons, e.g. antibiotics present as impurities in the final medicial products and bear the risk of causing allergic reactions, and antibiotics may promote antibiotic resistance (e.g. by gene transfer or selection of resistant pathogens).

The present application provides host cells for use in making bioconjugates comprising saccharide antigens that can be used as therapeutics (e.g. in immunogenic compositions, vaccines), wherein certain genetic elements required to drive the production of bioconjugates are integrated stably into the host cell genome. Consequently the host cell can contain a reduced number of plasmids, just a single plasmid or no plasmids at all. In some embodiments, the presence of a single plasmid can result in greater flexibility of the production strain and the ability to change the nature of the conjugation (in terms of its saccharide or carrier protein content) easily leading to greater flexibility of the production strain.

In general, a reduction in the use of plasmids leads to a production strain which is more suited for use in the production of medicinal products. A drawback of essential genetic material being present on plasmids is the requirement for selection pressure to maintain the episomal elements in the host cell. The selection pressure requires the use of antibiotics, which is undesirable for the production of medicinal products due to, e.g. the danger of allergic reactions against the antibiotics and the additional costs of manufacturing. Furthermore, selection pressure is often not complete, resulting in inhomogeneous bacterial cultures in which some clones have lost the plasmid and thus are not producing the bioconjugate. The host cells of the invention therefore are able to produce a safer product that can be obtained in high yields.

### Bioconjugates

The host cells of the invention can be used to produce bioconjugates comprising a pneumococcal saccharide of the invention, for example a pneumococcal saccharide of the invention linked to a carrier protein. Methods of producing bioconjugates using host cells are described for example in WO 2003/074687 and WO 2006/119987. Bioconjugates, as described herein, have advantageous properties over chemical conjugates of antigen-carrier protein, in that they require less chemicals in manufacture and are more consistent in terms of the final product generated.

In an embodiment, provided herein is a bioconjugate comprising a recombinant pneumococcal saccharide of the invention linked to a carrier protein.

The bioconjugates of the invention can be purified (to remove host cell impurities and unglycosylated carrier protein) e.g. by chromatography (e.g. ion exchange, anionic exchange, affinity, and sizing column chromatography), centrifugation, differential solubility, or by any other standard technique for the purification of proteins. See, e.g. Saraswat et al. 2013, Biomed. Res. Int. ID#312709 (p. 1-18); see also the methods described in WO 2009/104074. For example, affinity column IMAC (Immobilized metal ion affinity chromatography) may be used to bind the poly-histidine tag of the carrier protein, followed by two anion exchange chromatography and a size exclusion chromatography (SEC). Further, the bioconjugates may be fused to heterologous polypeptide sequences described herein or otherwise known in the art to facilitate purification. The actual conditions used to purify a particular bioconjugate will depend, in part, on the synthesis strategy and on factors such as net charge, hydrophobicity, and/or hydrophilicity of the bioconjugate, and will be apparent to those having skill in the art.

A further aspect of the invention is a process for producing a bioconjugate that comprises (or consists of) a recombinant pneumococcal saccharide of the invention linked to a carrier protein, said process comprising (i) culturing the host cell of the invention under conditions suitable for the production of proteins (and optionally under conditions suitable for the production of saccharides) and (ii) isolating the bioconjugate produced by said host cell.

A further aspect of the invention is a bioconjugate produced by the process of the invention, wherein said bioconjugate comprises a recombinant pneumococcal saccharide of the invention linked to a carrier protein.

### Analytical Methods

Various methods can be used to analyze the structural compositions and sugar chain lengths of the bioconjugates of the invention.

In one embodiment, hydrazinolysis can be used to analyze glycans. First, polysaccharides are released from their protein carriers by incubation with hydrazine according to the manufacturer's instructions (Ludger Liberate Hydrazinolysis Glycan Release Kit, Oxfordshire, UK). The nucleophile hydrazine attacks the glycosidic bond between the polysaccharide and the carrier protein and allows release of the attached glycans. N-acetyl groups are lost during this treatment and have to be reconstituted by re-N-acetylation. The free glycans are purified on carbon columns and subsequently labeled at the reducing end with the fluorophor 2-amino benzamide. See Bigge JC, Patel TP, Bruce JA, Goulding PN, Charles SM, Parekh RB: Nonselective and efficient fluorescent labeling of glycans using 2-amino benzamide and anthranilic acid. Anal Biochem 1995, 230(2):229-238. The labeled polysaccharides are separated on a GlycoSep-N column (GL Sciences) according to the HPLC protocol of Royle *et al.*. See Royle L, Mattu TS, Hart E, Langridge JI, Merry AH, Murphy N, Harvey DJ, Dwek RA, Rudd PM: An analytical and structural database provides a strategy for sequencing O-glycans from microgram quantities of glycoproteins. Anal Biochem 2002, 304(1):70-90. The resulting fluorescence chromatogram indicates the polysaccharide length and number of repeat units. Structural information can be gathered by collecting individual peaks and subsequently performing MS/MS analysis. Thereby the monosaccharide composition and sequence of the repeat unit could be confirmed and additionally in homogeneity of the polysaccharide composition could be identified.

In another embodiment, SDS-PAGE or capillary gel electrophoresis can be used to assess glycans and bioconjugates. Polymer length for the O antigen glycans is defined by the number of repeat units that are linearly assembled. This means that the typical ladder like pattern is a consequence of different repeat unit numbers that compose the glycan. Thus, two bands next to each other in SDS PAGE or other techniques that separate by size differ by only a single repeat unit. These discrete differences are exploited when analyzing glycoproteins for glycan size. The unglycosylated carrier protein and the bioconjugate with different polymer chain lengths separate according to their electrophoretic mobilities. The first detectable repeat unit number (n₁) and the average repeat unit number (n_{average}) present on a bioconjugate are measured. These parameters can be used to demonstrate batch to batch consistency or polysaccharide stability.

In another embodiment, high mass MS and size exclusion HPLC could be applied to measure the size of the complete bioconjugates.

In another embodiment, an anthrone-sulfuric acid assay can be used to measure polysaccharide yields. See Leyva A, Quintana A, Sanchez M, Rodriguez EN, Cremata J, Sanchez JC: Rapid and sensitive anthrone-sulfuric acid assay in microplate format to quantify carbohydrate in biopharmaceutical products: method development and validation. Biologicals : journal of the International Association of Biological Standardization 2008, 36(2):134-141. In another embodiment, a Methylpentose assay can be used to measure polysaccharide yields. See, e.g. Dische et al. J Biol Chem. 1948 Sep;175(2):595-603.

### Change in glycosylation site usage

To show that the site usage in a specific protein is changed in a multiple plasmid system as opposed to an inserted system, the glycosylation site usage must be quantified. Methods to do so are listed below.

Glycopeptide LC-MS/MS: bioconjugates are digested with protease(s), and the peptides are separated by a suitable chromatographic method (C18, Hydrophilic interaction HPLC HILIC, GlycoSepN columns, SE HPLC, AE HPLC), and the different peptides are identified using MS/MS. This method can be used with our without previous sugar chain shortening by chemical (smith degradation) or enzymatic methods. Quantification of glycopeptide peaks using UV detection at 215 to 280 nm allow relative determination of glycosylation site usage.

Size exclusion HPLC: Higher glycosylation site usage is reflected by a earlier elution time from a SE HPLC column.

### Homogeneity

Bioconjugate homogeneity (*i.e.,* the homogeneity of the attached sugar residues) can be assessed using methods that measure glycan length and hydrodynamic radius.

### Analytical Methods for Testing Benefit

*Yield.* Yield is measured as carbohydrate amount derived from a liter of bacterial production culture grown in a bioreactor under controlled and optimized conditions. After purification of bioconjugate, the carbohydrate yields can be directly measured by either the anthrone assay or ELISA using carbohydrate specific antisera. Indirect measurements are possible by using the protein amount (measured by BCA, Lowry, or bardford assays) and the glycan length and structure to calculate a theoretical carbohydrate amount per gram of protein. In addition, yield can also be measured by drying the glycoprotein preparation from a volatile buffer and using a balance to measure the weight.

*Homogeneity.* Homogeneity means the variability of glycan length and possibly the number of glycosylation sites. Methods listed above can be used for this purpose. SE-HPLC allows the measurement of the hydrodynamic radius. Higher numbers of glycosylation sites in the carrier lead to higher variation in hydrodynamic radius compared to a carrier with less glycosylation sites. However, when single glycan chains are analyzed, they may be more homogenous due to the more controlled length. Glycan length is measured by hydrazinolysis, SDS PAGE, and CGE. In addition, homogeneity can also mean that certain glycosylation site usage patterns change to a broader/narrower range. These factors can be measured by Glycopeptide LC-MS/MS.

*Strain stability and reproducibility.* Strain stability during bacterial fermentation in absence of selective pressure is measured by direct and indirect methods that confirm presence or absence of the recombinant DNA in production culture cells. Culture volume influence can be simulated by elongated culturing times meaning increased generation times. The more generations in fermentation, the more it is likely that a recombinant element is lost. Loss of a recombinant element is considered instability. Indirect methods rely on the association of selection cassettes with recombinant DNA, e.g. the antibiotic resistance cassettes in a plasmid. Production culture cells are plated on selective media, e.g. LB plates supplemented with antibiotics or other chemicals related to a selection system, and resistant colonies are considered as positive for the recombinant DNA associated to the respective selection chemical. In the case of a multiple plasmid system, resistant colonies to multiple antibiotics are counted and the proportion of cells containing all three resistances is considered the stable population. Alternatively, quantitative PCR can be used to measure the amount of recombinant DNA of the three recombinant elements in the presence, absence of selection, and at different time points of fermentation. Thus, the relative and absolute amount of recombinant DNA is measured and compared. Reproducibility of the production process is measured by the complete analysis of consistency batches by the methods stated in this application.

### Immunogenic Compositions

The pneumococcal saccharides and conjugates (e.g. bioconjugate), of the invention are particularly suited for inclusion in immunogenic compositions and vaccines. The present invention provides an immunogenic composition comprising the pneumococcal saccharide of the invention, or the conjugate of the invention, or the bioconjugate of the invention.

Immunogenic compositions comprise an immunologically effective amount of the pneumococcal saccharide or conjugate (e.g. bioconjugate) of the invention, as well as any other components. By "immunologicaly effective amount", it is meant that the administration of that amount to an individual, either as a single dose or as part of a series is effective for treatment or prevention. This amount varies depending on the health and physical condition of the individual to be treated, age, the degree of protection desired, the formulation of the vaccine and other relevant factors. It is expected that the amount will fall in a relatively broad range that can be determined through routine trials.

In an embodiment, the immunogenic composition of the invention further comprises a pharmaceutically acceptable excipient, carrier or diluent. Also provided is a method of making the immunogenic composition of the invention comprising the step of mixing the pneumococcal saccharide or the conjugate (e.g. bioconjugate) of the invention with a pharmaceutically acceptable excipient, carrier or diluent.

Pharmaceutically acceptable excipients and carriers can be selected by those of skill in the art. For example, the pharmaceutically acceptable excipient or carrier can include a buffer, such as Tris (trimethamine), phosphate (e.g. sodium phosphate), acetate, borate (e.g. sodium borate), citrate, glycine, histidine and succinate (e.g. sodium succinate), suitably sodium chloride, histidine, sodium phosphate or sodium succinate. The pharmaceutically acceptable excipient may include a salt, for example sodium chloride, potassium chloride or magnesium chloride. Optionally, the pharmaceutically acceptable excipient contains at least one component that stabilizes solubility and/or stability. Examples of solubilizing/stabilizing agents include detergents, for example, laurel sarcosine and/or polysorbate (e.g. TWEEN 80 (Polysorbate-80)). Examples of stabilizing agents also include poloxamer (e.g. poloxamer 124, poloxamer 188, poloxamer 237, poloxamer 338 and poloxamer 407). The phamaceutically acceptable excipient may include a non-ionic surfactant, for example polyoxyethylene sorbitan fatty acid esters, TWEEN 80 (Polysorbate-80), TWEEN 60 (Polysorbate-60), TWEEN 40 (Polysorbate-40) and TWEEN 20 (Polysorbate-20), or polyoxyethylene alkyl ethers (suitably polysorbate-80). Alternative solubilizing/stabilizing agents include arginine, and glass forming polyols (such as sucrose, trehalose and the like). The pharmaceutically excipient may be a preservative, for example phenol, 2-phenoxyethanol, or thiomersal. Other pharmaceutically acceptable excipients include sugars (e.g. lactose, sucrose), and proteins (e.g. gelatine and albumin). Pharmaceutically acceptable carriers include water, saline solutions, aqueous dextrose and glycerol solutions. Numerous pharmaceutically acceptable excipients and carriers are described, for example, in Remington's Pharmaceutical Sciences, by E. W. Martin, Mack Publishing Co. Easton, PA, 5th Edition (1975).

Immunogenic compositions if the invention may also contain diluents such as water, saline, glycerol etc. Additionally, auxiliary substances, such as wetting or emulsifying agents, pH buffering substances, polyols and the like may be present.

In an embodiment, the immunogenic composition of the invention additionally comprises one or more buffers, e.g. phosphate buffer and/or sucrose phosphate glutamate buffer. In other embodiments, the immunogenic composition of the invention does not comprise a buffer.

In an embodiment, the immunogenic composition of the invention additionally comprises one or more salts, e.g. sodium chloride, calcium chloride, sodium phosphate, monosodium glutamate, and aluminum salts (e.g. aluminum hydroxide, aluminum phosphate, alum (potassium aluminum sulfate), or a mixture of such aluminum salts). In other embodiments, the immunogenic composition of the invention does not comprise a salt.

In an embodiment, the immunogenic composition of the invention additionally comprises a preservative, e.g. a mercury derivative thimerosal or 2-phenoxyethanol. In an embodiment, the immunogenic composition of the invention comprises 0.001% to 0.01% thimerosal. In an embodiment, the immunogenic composition of the invention comprises 0.001% to 0.01% 2-phenoxyethanol. In other embodiments, the immunogenic composition or vaccine of the invention do not comprise a preservative.

In an embodiment, the immunogenic composition of the invention additionally comprises a detergent e.g. polysorbate, such as TWEEN 80 (Polysorbate-80). Detergents may be present at low levels e.g. <0.01%, but higher levels have been suggested for stabilising antigen formulations e.g. up to 10%.

The immunogenic compositions comprising the pneumococcal saccharide of the invention or conjugates (or bioconjugates) may comprise any additional components suitable for use in pharmaceutical administration. In an embodiment, the immunogenic compositions of the invention are monovalent formulations. In other embodiments, the immunogenic compositions of the invention are multivalent formulations, e.g. bivalent, trivalent, and tetravalent formulations. For example, a multivalent formulation comprises more than one antigen for example more than one conjugate.

In an embodiment, the immunogenic composition of the invention comprises 10 or more, 11 or more, 12 or more, 13 or more, 14 or more, 15 or more, 16 or more, 17 or more, 18 or more, 19 or more, 20 or more capsular saccharide conjugates from different *S. pneumoniae* serotypes. The immunogenic composition of the invention may comprise 10, 11, 12, 13, 14, 15, 16, 17, 18, 19, 20, 21, 22, 23, 24, or 25 capsular saccharide conjugates from different *S. pneumoniae* serotypes.

The immunogenic compositions of the invention may also comprise *S. pneumoniae* capsular saccharides (suitably conjugated to a carrier protein), for example as described in WO2007/071707A2. The *S. pneumoniae* capsular saccharides (suitably conjugated to a carrier protein) may be selected from a *Streptococcus pneumoniae* serotypes 1, 2, 3, 4, 5, 6A, 6B, 7A, 7B, 7C, 8, 9A, 9L, 9N, 9V, 10A, 10B, 10C, 10F, 11A, 11B, 11C, 11D, 11F, 12A, 12B, 12F, 13, 14, 15A, 15B, 15C, 15F, 16A, 16F, 17A, 17F, 18A, 18B, 18C, 18F, 19A, 19B, 19C, 19F, 20, 21, 22A, 22F, 23A, 23B, 23F, 24A, 24B, 24F, 25A, 25F, 26, 27, 28A, 28F, 29, 31, 32A, 32F, 33A, 33B, 33C, 33D, 33F, 34, 35A, 35B, 35C, 35D, 35F, 36, 37, 38, 39, 40, 41A, 41F, 42, 43, 44, 45, 46, 47A, 47F or 48 capsular saccharide. For example, the *S. pneumoniae* capsular saccharides (suitably conjugated to a carrier protein) may be selected from a *Streptococcus pneumoniae* serotypes 1, 2, 3, 4, 5, 6A, 6B, 7F, 8, 9N, 9V, 10A, 11A, 12F, 14, 15B, 17F, 18C, 19A, 19F, 20, 22F, 23F and 33F. In an embodiment, at least four serotypes are included in the composition, e.g. 6B, 14, 19F and 23F (suitably conjugated to a carrier protein). In another embodiment, at least 7 serotypes are included in the composition, e.g. 4, 6B, 9V, 14, 18C, 19F and 23F (suitably conjugated to a carrier protein). In another embodiment the immunogenic composition comprises 10 or more, 11 or more, 12 or more, 13 or more, 14 or more, 15 or more, 16 or more, 17 or more, 19 or more, or 20 capsular saccharides from different *S. pneumoniae* serotypes (suitably conjugated to a carrier protein). In an embodiment the immunogenic composition comprises 10 to 23 capsular saccharides from different *S. pneumoniae* serotypes (suitably conjugated to a carrier protein). In an embodiment, the vaccine may be an 11-valent vaccine. For example, a 11-valent vaccine may comprise saccharides from serotypes 1, 4, 5, 6A, 6B, 7F, 9V, 14, 18C, 19F and 23F. In an embodiment, the vaccine may be an 12-valent or 13-valent vaccine. A 12 or 13-valent paediatric (infant) vaccine may also include the 11 valent formulation supplemented with serotypes 19A, or 22F, whereas a 13-valent elderly vaccine may include the 11 valent formulation supplemented with serotypes 19A and 22F, 8 and 12F, or 8 and 15, or 8 and 19A, or 8 and 22F, or 12F and 15, or 12F and 19A, or 12F and 22F, or 15 and 19A, or 15 and 22F. In an embodiment, the vaccine may be a 14-valent or 15-valent vaccine. A 14 or 15-valent paediatric vaccine may include the 11 valent formulation described above supplemented with serotypes 3, 19A and 22F; serotypes 8, 19A and 22F; serotypes 12F, 19A and 22F; serotypes 15, 19A and 22F; serotypes 3, 8, 19A and 22F; serotypes 3, 12F, 19A and 22F; serotypes 3, 15, 19A and 22F. In an embodiment, the vaccine may be a 16-valent vaccine. A 16 valent vaccine may include the 11 valent formulation described above supplemented with serotypes 3, 15B, 19A, 22F and 23F. A 16 valent vaccine may include the 11 valent formulation described above supplemented with serotypes 3, 15B, 19A, 22F and 33F. In an embodiment, the vaccine may be a 19-valent vaccine. A 19 valent vaccine may include the 11 valent formulation described above supplemented with serotypes 8, 10A, 11A, 12F, 15B, 19A, 22F and 23F. A 19 valent vaccine may include the 11 valent formulation described above supplemented with serotypes 8, 10A, 11A, 12F, 15B, 19A, 22F and 33F. In an embodiment, the vaccine may be a 20-valent vaccine. A 20 valent vaccine may include the 11 valent formulation described above supplemented with serotypes 3, 8, 10A, 11A, 12F, 15B, 19A, 22F and 23F. A 20 valent vaccine may include the 11 valent formulation described above supplemented with serotypes 3, 8, 10A, 11A, 12F, 15B, 19A, 22F and 33F. In an embodiment, the vaccine may be a 21-valent vaccine. In an embodiment, the vaccine may be a 22-valent vaccine. In an embodiment, the vaccine may be a 23-valent vaccine. In an embodiment, the vaccine may be a 24-valent vaccine. In an embodiment, the vaccine may be a 25-valent vaccine.

The immunogenic composition of the invention may optionally further comprise additional antigens. Examples of such additional antigens are *S. pneumoniae* antigens selected from the following categories, such as proteins having a Type II Signal sequence motif of LXXC (where X is any amino acid, e.g. the polyhistidine triad family (PhtX)), choline binding proteins (e.g. CbpX (choline binding protein family), PcpA (pneumococcal choline-binding protein A)), proteins having a Type I Signal sequence motif (e.g. Sp101), and proteins having a LPXTG motif (where X is any amino acid, e.g. Sp128, Sp130). Thus, the immunogenic composition of the invention may comprise one or more *S. pneumoniae* proteins selected from polyhistidine triad family (PhtX), Choline Binding Protein family (CbpX), CbpX truncates, pneumococcal autolysin family (LytX) (e.g. LytA (*N-*acetylmuramoyl-l-alanine amidase), LytB, LytC), LytX truncates, CbpX truncate-LytX truncate chimeric proteins, PspA (pneumococcal surface protein A), PsaA (pneumococcal surface adhesion A), Sp128, Sp101, Sp130, Sp125 and Sp133. In a further embodiment, the immunogenic composition of the invention comprises 2 or more proteins selected from the group consisting of the polyhistidine triad family (PhtX), Choline Binding Protein family (CbpX), CbpX truncates, LytX family, LytX truncates, CbpXtruncate-LytXtruncate chimeric proteins (or fusions), PspA (pneumococcal surface protein A), PsaA (pneumococcal surface adhesion A), and Sp128. In a further embodiment, the immunogenic composition comprises 2 or more proteins selected from the group consisting of the polyhistidine triad family (PhtX) e.g. PhtD, Choline Binding Protein family (CbpX), CbpX truncates, LytX family, LytX truncates, CbpX truncate-LytX truncate chimeric proteins (or fusions), and Sp128.

In an embodiment, the *S. pneumoniae* antigen selected from member(s) of the polyhistidine triad family is PhtD. The term "PhtD" as used herein includes the full length protein with the signal sequence attached or the mature full length protein with the signal peptide (for example 20 amino acids at N-terminus) removed, and immunogenic fragments, variants and/or fusion proteins thereof, e.g. SEQ ID NO: 4 of WO00/37105. In one aspect, PhtD is the full length protein with the signal sequence attached e.g. SEQ ID NO: 4 of WO00/37105. In another aspect, PhtD is a sequence comprising the mature full length protein with the signal peptide (for example 20 amino acids at N-terminus) removed, e.g. amino acids 21-838 of SEQ ID NO: 4 of WO00/37105. Suitably, the PhtD sequence comprises an N-terminal methionine. The present invention also includes PhtD polypeptides which are immunogenic fragments of PhtD, variants of PhtD and/or fusion proteins of PhtD. For example, as described in WO00/37105, WO00/39299, US6699703 and WO09/12588.

Immunogenic compositions of the present invention may comprise additional antigens capable of eliciting an immune response against a human or animal pathogen. These additional antigens include, for example, additional *S. pneumoniae* antigens, e.g. *S. pneumoniae* protein antigens. Such proteins may be used as carrier proteins, or may be present as a free protein (unconjugated), or may be present both as a carrier protein and a free protein. Where the additional antigen is a pneumococcal protein, the protein may be conjugated for example to a saccharide. In an embodiment, the immunogenic composition of the invention further comprises one or more unconjugated *S. pneumoniae* proteins, for example, unconjugated pneumococcal polyhistidine triad protein D (PhtD). In another embodiment, the immunogenic composition of the invention further comprises one or more conjugated *S. pneumoniae* proteins, for example, conjugated pneumococcal polyhistidine triad protein D (PhtD).

The additional *Streptococcus pneumoniae* antigens are either surface exposed, at least during part of the life cycle of the pneumococcus, or are proteins which are secreted or released by the pneumococcus. In an embodiment, the *S. pneumoniae* antigens are selected from the following categories, such as proteins having a Type II Signal sequence motif of LXXC (where X is any amino acid, e.g. the polyhistidine triad family (PhtX)), choline binding proteins (e.g. CbpX (choline binding protein family), PcpA (pneumococcal choline-binding protein A)), proteins having a Type I Signal sequence motif (e.g. Sp101), and proteins having a LPXTG motif (where X is any amino acid, e.g., Sp128, Sp130). Preferred examples within these categories (or motifs) are the following proteins, or immunologically functional equivalents thereof. Thus, the immunogenic composition of the invention may comprise one or more *S. pneumoniae* proteins selected from polyhistidine triad family (PhtX), Choline Binding Protein family (CbpX), CbpX truncates, pneumococcal autolysin family (LytX) (e.g. LytA (*N*-acetylmuramoyl-l-alanine amidase), LytB, LytC), LytX truncates, CbpX truncate-LytX truncate chimeric proteins, PspA (pneumococcal surface protein A), PsaA (pneumococcal surface adhesion A), Sp128, Sp101, Sp130, Sp125 and Sp133. In a further embodiment, the immunogenic composition of the invention comprises 2 or more proteins selected from the group consisting of the polyhistidine triad family (PhtX), Choline Binding Protein family (CbpX), CbpX truncates, LytX family, LytX truncates, CbpXtruncate-LytXtruncate chimeric proteins (or fusions), PspA (pneumococcal surface protein A), PsaA (pneumococcal surface adhesion A), and Sp128. In a further embodiment, the immunogenic composition comprises 2 or more proteins selected from the group consisting of the polyhistidine triad family (PhtX), Choline Binding Protein family (CbpX), CbpX truncates, LytX family, LytX truncates, CbpX truncate-LytX truncate chimeric proteins (or fusions), and Sp128.

The Pht (polyhistidine triad) family comprises proteins PhtA, PhtB, PhtD, and PhtE. The family is characterized by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and Neisseria. In one embodiment of the invention, the immunogenic composition comprises PhtD. It is understood, however, that the terms Pht A, B, D, and E refer to proteins having sequences disclosed in the citations below as well as variants thereof that have a sequence homology that is at least 90% identical to the proteins described below, e.g. amino acids 21-838 of SEQ ID NO: 4 of WO00/37105. In an embodiment it is at least 95% identical and in another embodiment it is 97% identical to the proteins described below, e.g. amino acids 21-838 of SEQ ID NO: 4 of WO00/37105.

With regards to the PhtX proteins, PhtA is disclosed in WO 98/18930, and is also referred to Sp36. As noted herein, it is a protein from the polyhistidine triad family and has the type II signal motif of LXXC. PhtD is disclosed in WO 00/37105, and is also referred to Sp036D. As noted herein, it also is a protein from the polyhistidine triad family and has the type II LXXC signal motif. PhtB is disclosed in WO 00/37105, and is also referred to Sp036B. Another member of the PhtB family is the C3-Degrading Polypeptide, as disclosed in WO 00/17370. This protein also is from the polyhistidine triad family and has the type II LXXC signal motif. A preferred immunologically functional equivalent is the protein Sp42 disclosed in WO 98/18930. A PhtB truncate (a "truncate" being part of a protein having an N-terminal and/or C-terminal deletion) (approximately 79kD) is disclosed in WO99/15675 which is also considered a member of the PhtX family. PhtE is disclosed in WO00/30299 and is referred to as BVH-3. Where any Pht protein is referred to herein, it is meant that immunogenic fragments or fusions thereof of the Pht protein can be used.

In one embodiment, the *S. pneumoniae* antigen selected from member(s) of the polyhistidine triad family is PhtD. The term "PhtD" as used herein includes the full length protein with the signal sequence attached or the mature full length protein with the signal peptide (for example 20 amino acids at N-terminus) removed, and immunogenic fragments, variants and/or fusion proteins thereof, e.g. SEQ ID NO: 4 of WO00/37105. In one aspect, PhtD is the full length protein with the signal sequence attached e.g. SEQ ID NO: 4 of WO00/37105. In another aspect, PhtD is a sequence comprising the mature full length protein with the signal peptide (for example 20 amino acids at N-terminus) removed, e.g. amino acids 21-838 of SEQ ID NO: 4 of WO00/37105. Suitably, the PhtD sequence comprises an N-terminal methionine. The present invention also includes PhtD polypeptides which are immunogenic fragments of PhtD, variants of PhtD and/or fusion proteins of PhtD. For example, as described in WO00/37105, WO00/39299, US6699703 and WO09/12588.

Where immunogenic fragments of PhtD proteins are used (separately or as part of a fusion protein), these immunogenic fragments will be at least about 15, at least about 20, at least about 40, or at least about 60 contiguous amino acid residues in length, e.g. from a PhtD amino acid sequence in WO00/37105 or WO00/39299, such as SEQ ID NO: 4 of WO00/37105. In an embodiment of the invention, immunogenic fragments of PhtD protein comprise at least about 15, at least about 20, at least about 40, or at least about 60 contiguous amino acid residues of the sequence shown in SEQ ID NO: 4 of WO00/37105, wherein said polypeptide is capable of eliciting an immune response specific for said amino acid sequence. In an embodiment, the immunogenic composition of the invention comprises an immunogenic fragment of PhtD, for example described in WO09/12601, WO01/98334 and WO09/12588. Where immunogenic fragments of PhtD proteins are used (separately or as part of a fusion protein), each immunogenic fragment optionally contains one or more histidine triad motif(s) of such polypeptides. A histidine triad motif is the portion of polypeptide that has the sequence HxxHxH where H is histidine and x is an amino acid other than histidine. In an embodiment of the present invention, the or each immunogenic fragment contains exactly or at least 2, 3, 4 or 5 histidine triad motifs (optionally, with native PhtD sequence between the 2 or more triads, or intra-triad sequence) where the immunogenic fragment is more than 50, 60, 70, 80, 90 or 100% identical to a native pneumococcal intra-triad PhtD sequence (e.g. the intra-triad sequence shown in SEQ ID NO: 4 of WO00/37105). Immunogenic fragments of PhtD proteins optionally contain one or more coiled coil regions of such polypeptides. A coiled coil region is a region predicted by "Coils" algorithm Lupus, A et al (1991) Science 252; 1162-1164. In an embodiment of the present invention, each immunogenic fragment contains exactly or at least 2, 3 or 4 coiled coil regions. In an embodiment of the present invention, the or each immunogenic fragment contains exactly or at least 2, 3 or 4 coiled coil regions where the immunogenic fragment is more than 50, 60, 70, 80, 90, 95, 96 or 100% identical to a native pneumococcal PhtD sequence (e.g. the sequence shown in SEQ ID NO: 4 of WO00/37105). In another embodiment of the present invention, the immunogenic fragment includes one or more histidine triad motif as well as at least 1, 2, 3 or 4 coiled coil regions.

In the case where the PhtD polypeptide is a variant, the variation is generally in a portion thereof other than the histidine triad residues and the coiled-coil region, although variations in one or more of these regions may be made. In accordance with the present invention, a variant is a protein in which the native pneumolysin is mutated. Amino acid substitution may be conservative or non-conservative. In one aspect, amino acid substitution is conservative. Substitutions, deletions, insertions or any combination thereof may be combined in a single variant so long as the variant is an immunogenic polypeptide. Variants typically include polypeptides which share at least 80, 90, 94, 95, 98, or 99% amino acid sequence identity with a wild-type sequence. Variants of PhtD typically include any immunogenic fragment or variation of PhtD which shares at least 80, 90, 95, 96, 98, or 99% amino acid sequence identity with a wild-type PhtD sequence, e.g. SEQ ID NO: 4 of WO00/37105. In an embodiment, the present invention includes immunogenic fragments and/or variants in which several, 5 to 10, 1 to 5, 1 to 3, 1 to 2 or 1 amino acid(s) are substituted, deleted, or added in any combination. In another embodiment, the present invention includes immunogenic fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

In an embodiment of the invention, PhtD and its immunogenic fragments, variants and/or fusion proteins thereof comprise an amino acid sequence sharing at least 80, 85, 90, 95, 96, 97, 98, 99 or 100% identity with amino acid sequence 21 to 838 of SEQ ID NO:4 of WO00/37105. In another embodiment of the invention, PhtD and its immunogenic fragments, variants and/or fusion proteins thereof have an amino acid sequence sharing at least 80, 85, 90, 95, 96, 97, 98, 99 or 100% identity with amino acid sequence 21 to 838 of SEQ ID NO:4 of WO00/37105. Suitably, PhtD and its immunogenic fragments, variants and/or fusion proteins thereof comprise an amino acid sequence having an N-terminal methionine. In another embodiment of the invention, PhtD and its immunogenic fragments, variants and/or fusion proteins thereof comprise at least about 15, at least about 20, at least about 40, or at least about 60 or at least about 100, or at least about 200, or at least about 400 or at least about 800 contiguous amino acid residues of the sequence shown in SEQ ID NO: 4 of WO00/37105.

In one aspect the PhtD is conjugated to a saccharide, e.g. a capsular saccharide of *S. pneumoniae*. For example, PhtD may be conjugated to a capsular saccharide of *S. pneumoniae* selected from serotypes 1, 2, 3, 4, 5, 6A, 6B, 7F, 8, 9N, 9V, 10A, 11A, 12F, 14, 15, 17F, 18C, 19A, 19F, 20, 22F, 23F and 33F. In particular, PhtD may be conjugated to a capsular saccharide of *S. pneumoniae* serotype 22F. In another aspect, PhtD is unconjugated or present in the immunogenic composition as a free protein. In an aspect of the invention, more than 80% (e.g. more than 82%, 85%, 90%, 91%, 92%, 93%, 94%, 95%, 96%, 97%, 98% or 99%) of the PhtD is adsorbed onto aluminium phosphate. In another aspect of the invention, greater than 80% (e.g. more than 81%, 82%, 83%, 84%, 85%, 86%, 87%, 88%, 89% or 90%) of the particles of PhtD (e.g. unconjugated PhtD) adsorbed onto aluminium phosphate have a size less than 10µm.

The present invention also provides an immunogenic composition comprising PhtD adsorbed onto aluminium phosphate, wherein more than 85% (e.g. more than 90%, 91%, 92%, 93%, 94%, 95%, 96%, 97%, 98% or 99%) of the PhtD is adsorbed onto aluminium phosphate. The present invention also provides an immunogenic composition wherein greater than 80% (e.g. more than 81%, 82%, 83%, 84%, 85%, 86%, 87%, 88%, 89% or 90%) of the particles of PhtD adsorbed onto aluminium phosphate have has a particle a size less than 10µm.

Concerning the Choline Binding Protein family (CbpX), members of that family were originally identified as pneumococcal proteins that could be purified by choline-affinity chromatography. All of the choline-binding proteins are non-covalently bound to phosphorylcholine moieties of cell wall teichoic acid and membrane-associated lipoteichoic acid. Structurally, they have several regions in common over the entire family, although the exact nature of the proteins (amino acid sequence, length, etc.) can vary. In general, choline binding proteins comprise an N terminal region (N), conserved repeat regions, a proline rich region (P) and a conserved choline binding region (C), made up of multiple repeats, that comprises approximately one half of the protein. As used in this application, the term "Choline Binding Protein family (CbpX)" is selected from the group consisting of Choline Binding Proteins as identified in WO97/41151, Choline binding protein A, CbpA (also referred to as PbcA (C3-binding protein A), SpsA (*Streptococcus pneumoniae* secretory IgA binding protein), PspC (pneumococcal surface protein C)), Choline binding protein D (CbpD), and Choline binding protein G (CbpG). CbpA is disclosed in WO97/41151. CbpD and CbpG are disclosed in WO00/29434. PspC is disclosed in WO97/09994. PbcA is disclosed in WO98/21337. SpsA is a Choline binding protein disclosed in WO 98/39450. In an embodiment, the Choline Binding Proteins is CbpA. Another Choline Binding Protein is pneumococcal choline-binding protein A (PcpA) (Sanchez-Beato et al FEMS Microbiology Letters 164 (1998) 207-214).

Another preferred embodiment is CbpX truncates wherein "CbpX" is CbpA, CbpD or CbpG and "CbpX truncates" refers to CbpX proteins lacking 50% or more of the Choline binding region (C). Another preferred embodiment is PcpA truncates wherein "PcpA truncates" refers to PcpA proteins lacking 50% or more of the Choline binding region (C). In an embodiment, CbpX truncates or PcpA truncates lack the entire choline binding region. In another embodiment, the CbpX truncates or PcpA truncates lack (i) the choline binding region and (ii) a portion of the N-terminal half of the protein as well, yet retain at least one repeat region. In another embodiment, the truncate has at least 2 repeat regions. Examples of such preferred embodiments are illustrated in WO99/51266 or WO99/51188, however, other choline binding proteins lacking a similar choline binding region are also contemplated within the scope of this invention.

The LytX family is membrane associated proteins associated with cell lysis. The N-terminal domain comprises choline binding domain(s), however the LytX family does not have all the features found in the CbpA family noted herein and thus for the present invention, the LytX family is considered distinct from the CbpX family. In contrast with the CbpX family, the C-terminal domain contains the catalytic domain of the LytX protein family. The family comprises LytA, LytB and LytC. With regards to the LytX family, LytA is disclosed in Ronda et al., Eur J Biochem, 164:621-624 (1987). LytB is disclosed in WO 98/18930, and is also referred to as Sp46. LytC is also disclosed in WO 98/18930, and is also referred to as Sp91. A preferred member of that family is LytC.

Another preferred embodiment are LytX truncates wherein "LytX" is LytA, LytB or LytC and " LytX truncates" refers to LytX proteins lacking 50% or more of the Choline binding region. Suitably such proteins lack the entire choline binding region. Yet another preferred embodiment of this invention are CbpX truncate-LytX truncate chimeric proteins (or fusions). In an embodiment, the CbpX truncate-LytX truncate chimeric protein comprises the repeat regions of CbpX and the C-terminal portion (Cterm, *i.e.,* lacking the choline binding domains) of LytX (e.g., LytCCterm or Sp91Cterm). In another embodiment, CbpX is selected from the group consisting of CbpA, PbcA, SpsA and PspC. In another embodiment, it is CbpA. In an embodiment, LytX is LytC (also referred to as Sp91). Another embodiment of the present invention is a PspA (pneumococcal surface protein A) or PsaA (pneumococcal surface adhesion A) truncates lacking the choline binding domain (C) and expressed as a fusion protein with LytX. In an embodiment, LytX is LytC.

PsaA (pneumococcal surface adhesion A) and transmembrane deletion variants thereof have been described by Berry & Paton, Infect Immun 1996 Dec;64(12):5255-62. PspA (pneumococcal surface protein A) and transmembrane deletion variants thereof have been disclosed in, for example, US 5804193, WO 92/14488, and WO 99/53940.

Sp128 and Sp130 are disclosed in WO00/76540. Sp125 is an example of a pneumococcal surface protein with the Cell Wall Anchored motif of LPXTG (*i.e.,* leucine-*proline*-X-threonine-glycine where X is any amino acid). Any protein within this class of pneumococcal surface protein with this motif has been found to be useful within the context of this invention, and is therefore considered a further protein of the invention. Sp125 itself is disclosed in WO 98/18930, and is also known as ZmpB - a zinc metalloproteinase. Sp101 is disclosed in WO 98/06734 (where it has the reference # y85993). It is characterized by a Type I signal sequence. Sp133 is disclosed in WO 98/06734 (where it has the reference # y85992). It is also characterized by a Type I signal sequence.

The *S. pneumoniae* antigens may also be beneficially combined. By combined is meant that the immunogenic composition comprises all of the proteins from within the combination, either as carrier proteins or as free proteins or a mixture of the two. For example, in a combination of two proteins as set out hereinafter, both proteins may be used as carrier proteins, or both proteins may be present as free proteins, or both may be present as carrier and as free protein, or one may be present as a carrier protein and a free protein whilst the other is present only as a carrier protein or only as a free protein, or one may be present as a carrier protein and the other as a free protein. Where a combination of three proteins is given, similar possibilities exist. Preferred combinations include, but are not limited to PhtD + CbpX repeat regions, PhtD + dPly, PhtD + Sp128, PhtD + PsaA, PhtD + PspA, PhtA + CbpX repeat regions , PhtA + CbpX repeat regions -Sp91Cterm chimeric or fusion proteins, PhtA + dPly, PhtA + Sp128, PhtA + PsaA, PhtA + PspA, CbpX repeat regions + LytC, CbpX repeat regions + PspA, CbpX repeat regions + PsaA, CbpX repeat regions + Sp128, CbpX repeat regions + LytC, CbpX repeat regions + PspA, CbpX repeat regions + PsaA, CbpX repeat regions + Sp128, CbpX repeat regions + PhtD, CbpX repeat regions + PhtA. In an embodiment, CbpX repeat regionsis from CbpA. In another embodiment, it is from CbpA. Other combinations include 3 protein combinations such as PhtD + CbpX repeat regions + dPly, and PhtA + CbpX repeat regions + PhtD. In one embodiment, the immunogenic composition comprises detoxified pneumolysin and PhtD as carrier proteins. In a further embodiment, the immunogenic composition comprises detoxified pneumolysin and PhtD as free proteins.

### Adjuvants

In an embodiment, the immunogenic composition of the invention further comprises an adjuvant (suitably a pharmaceutically acceptable adjuvant). Also provided is a method of making the immunogenic composition of the invention comprising the step of mixing the pneumococcal saccharide or the conjugate (e.g. bioconjugate) of the invention with an adjuvant.

In an embodiment, the immunogenic composition of the invention comprises, or is administered in combination with, an adjuvant. The adjuvant for administration in combination with an immunogenic composition of the invention may be administered before, concomitantly with, or after administration of said immunogenic composition or vaccine. In some embodiments, the term "adjuvant" refers to a compound that when administered in conjunction with or as part of an immunogenic composition of the invention augments, enhances and/or boosts the immune response to a bioconjugate, but when the compound is administered alone does not generate an immune response to the pneumococcal saccharide/conjugate/bioconjugate. In some embodiments, the adjuvant generates an immune response to the pneumococcal saccharide, conjugate or bioconjugate and does not produce an allergy or other adverse reaction.

Adjuvants can enhance an immune response by several mechanisms including, e.g. lymphocyte recruitment, stimulation of B and/or T cells, and stimulation of macrophages. Specific examples of adjuvants include, but are not limited to, aluminum salts (alum) (such as aluminum hydroxide, aluminum phosphate, and aluminum sulfate), 3 De-O-acylated monophosphoryl lipid A (MPL) (see United Kingdom Patent GB2220211), MF59 (Novartis), AS03 (GlaxoSmithKline), AS04 (GlaxoSmithKline), polysorbate 80 (TWEEN 80; ICL Americas, Inc.), imidazopyridine compounds (see International Application NO: PCT/US2007/064857, published as International Publication NO: WO2007/109812), imidazoquinoxaline compounds (see International Application NO: PCT/US2007/064858, published as International Publication NO: WO2007/109813) and saponins, such as QS21 (see Kensil et al. in Vaccine Design: The Subunit and Adjuvant Approach (eds. Powell & Newman, Plenum Press, NY, 1995); U.S. Pat. NO: 5,057,540). In some embodiments, the adjuvant is Freund's adjuvant (complete or incomplete). Other adjuvants are oil in water emulsions (such as squalene or peanut oil), optionally in combination with immune stimulants, such as monophosphoryl lipid A (see Stoute et al. N. Engl. J. Med. 336, 86-91 (1997)). Another adjuvant is CpG (Bioworld Today, Nov. 15, 1998).

In one aspect of the invention, the adjuvant is an aluminum salt such as aluminum hydroxide gel (alum) or aluminium phosphate.

In another aspect of the invention, the adjuvant is selected to be a preferential inducer of either a TH1 or a TH2 type of response. High levels of Th1-type cytokines tend to favor the induction of cell mediated immune responses to a given antigen, whilst high levels of Th2-type cytokines tend to favour the induction of humoral immune responses to the antigen. It is important to remember that the distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of Il-4, IL-5, IL-6, IL-10. Suitable adjuvant systems which promote a predominantly Th1 response include: Monophosphoryl lipid A or a derivative thereof, particularly 3-de-O-acylated monophosphoryl lipid A (3D-MPL) (for its preparation see GB 2220211 A); and a combination of monophosphoryl lipid A, for example 3-de-O-acylated monophosphoryl lipid A, together with either an aluminium salt (for instance aluminium phosphate or aluminium hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP 689454-B1]. Unmethylated CpG containing oligonucleotides (WO 96/02555) are also preferential inducers of a TH1 response and are suitable for use in the present invention.

The immunogenic composition of the invention may contain an oil in water emulsion, since these have been suggested to be useful as adjuvant compositions (EP 399843; WO 95/17210). Oil in water emulsions such as those described in WO95/17210 (which discloses oil in water emulsions comprising from 2 to 10% squalene, from 2 to 10% alpha tocopherol and from 0.3 to 3% Tween 80 (Polysorbate 80) and their use alone or in combination with QS21 and/or 3D-MPL), WO99/12565 (which discloses oil in water emulsion compositions comprising a metabolisable oil, a saponin and a sterol and MPL) or WO99/11241 may be used. Further oil in water emulsions such as those disclosed in WO 09/127676 and WO 09/127677 are also suitable. A particularly potent adjuvant formulation involving QS21, 3D-MPL and tocopherol in an oil in water emulsion is described in WO 95/17210. In an embodiment, the immunogenic composition or vaccine additionally comprises a saponin, for example QS21. The immunogenic composition may also comprise an oil in water emulsion and tocopherol (WO 95/17210).

### Vaccines

The present invention also provides a vaccine comprising an immunogenic composition of the invention.

The immunogenic compositions or vaccines of the invention can be included in a container, pack, or dispenser together with instructions for administration.

The immunogenic compositions or vaccines of the invention can be stored before use, *e.g.* the compositions can be stored frozen (*e.g.* at about -20°C or at about -70°C); stored in refrigerated conditions (*e.g.* at about 4°C); or stored at room temperature.

The immunogenic compositions or vaccines of the invention may be stored in solution or lyophilized. In an embodiment, the solution is lyophilized in the presence of a sugar such as sucrose, trehalose or lactose. In another embodiment, the vaccines of the invention are lyophilized and extemporaneously reconstituted prior to use.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

The present invention also provides a kit comprising a pneumococcal saccharide of the invention, a bioconjugate of the invention, an immunogenic composition of the invention or a vaccine of the invention and instructions for the use thereof.

### Method of Administration

Immunogenic compositions or vaccines of the invention may be used to protect or treat a mammal susceptible to infection, by means of administering said immunogenic composition or vaccine via systemic or mucosal route. These administrations may include injection via the intramuscular (IM), intraperitoneal, intradermal (ID) or subcutaneous routes; or *via* mucosal administration to the oral/alimentary, respiratory, genitourinary tracts. For example, intranasal (IN) administration may be used for the treatment of pneumonia or otitis media (as nasopharyngeal carriage of pneumococci can be more effectively prevented, thus attenuating infection at its earliest stage). Although the immunogenic composition or vaccine of the invention may be administered as a single dose, components thereof may also be co-administered together at the same time or at different times (for instance pneumococcal saccharides could be administered separately, at the same time or 1-2 weeks after the administration of any bacterial protein component of the vaccine for optimal coordination of the immune responses with respect to each other). For co-administration, the optional Th1 adjuvant may be present in any or all of the different administrations, however in one particular aspect of the invention it is present in combination with the pneumococcal saccharide component of the immunogenic composition or vaccine. In addition to a single route of administration, 2 different routes of administration may be used. For example, saccharides may be administered IM (or ID) and bacterial proteins may be administered IN (or ID). In addition, the vaccines of the invention may be administered IM for priming doses and IN for booster doses.

In one aspect, the immunogenic composition or vaccine of the invention is administered by the intramuscular delivery route. Intramuscular administration may be to the thigh or the upper arm. Injection is typically via a needle (e.g. a hypodermic needle), but needle-free injection may alternatively be used. A typical intramuscular dose is 0.5 ml.

In another aspect, the immunogenic composition or vaccine of the invention is administered by the intradermal administration. Human skin comprises an outer "horny" cuticle, called the stratum corneum, which overlays the epidermis. Underneath this epidermis is a layer called the dermis, which in turn overlays the subcutaneous tissue. The conventional technique of intradermal injection, the "mantoux procedure", comprises steps of cleaning the skin, and then stretching with one hand, and with the bevel of a narrow gauge needle (26 to 31 gauge) facing upwards the needle is inserted at an angle of between 10 to 15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

In another aspect, the immunogenic composition or vaccine of the invention is administered by the intranasal administration. Typically, the immunogenic composition or vaccine is administered locally to the nasopharyngeal area, e.g. without being inhaled into the lungs. It is desirable to use an intranasal delivery device which delivers the immunogenic composition or vaccine formulation to the nasopharyngeal area, without or substantially without it entering the lungs. Suitable devices for intranasal administration of the vaccines according to the invention are spray devices.

In an embodiment, spray devices for intranasal use are devices for which the performance of the device is not dependent upon the pressure applied by the user. These devices are known as pressure threshold devices. Liquid is released from the nozzle only when a threshold pressure is applied. These devices make it easier to achieve a spray with a regular droplet size. Pressure threshold devices suitable for use with the present invention are known in the art and are described for example in WO91/13281 and EP311863 and EP516636, incorporated herein by reference. Such devices are commercially available from Pfeiffer GmbH and are also described in Bommer, R. Pharmaceutical Technology Europe, Sept 1999.

In another embodiment, intranasal devices produce droplets (measured using water as the liquid) in the range 1 to 200 µm, e.g. 10 to 120 µm. Below 10 µm there is a risk of inhalation, therefore it is desirable to have no more than about 5% of droplets below 10 µm. Droplets above 120 µm do not spread as well as smaller droplets, so it is desirable to have no more than about 5% of droplets exceeding 120 µm.

Following an initial vaccination, subjects may receive one or several booster immunizations adequately spaced.

The immunogenic composition or vaccine of the present invention may be used to protect or treat a mammal, e.g. human, susceptible to infection, by means of administering said immunogenic composition or vaccine via a systemic or mucosal route. These administrations may include injection *via* the intramuscular (IM), intraperitoneal (IP), intradermal (ID) or subcutaneous (SC) routes; or *via* mucosal administration to the oral/alimentary, respiratory, genitourinary tracts. Although the vaccine of the invention may be administered as a single dose, components thereof may also be co-administered together at the same time or at different times (for instance pneumococcal saccharide conjugates could be administered separately, at the same time or 1-2 weeks after the administration of the any pneumococcal saccharide, conjugate or bioconjugate of the invention for optimal coordination of the immune responses with respect to each other). For co-administration, the optional adjuvant may be present in any or all of the different administrations. In addition to a single route of administration, 2 different routes of administration may be used. For example, the pneumococcal saccharide, conjugate or bioconjugate of the invention may be administered IN (or ID). In addition, the immunogenic compositions or vaccines of the invention may be administered IM for priming doses and IN for booster doses.

### Dosage

The amount of conjugate antigen in each immunogenic composition or vaccine dose is selected as an amount which induces an immunoprotective response without significant, adverse side effects in typical vaccines. Such amount will vary depending upon which specific immunogen is employed and how it is presented. The content of penumococcal saccharide will typically be in the range 1-100µg, suitably 5-50µg. The content of saccharide will typically be in the range 0.1-10µg, suitably 1-5 µg.

A dose which is in a volume suitable for human use is generally between 0.25 and 1.5 ml, although, for administration to the skin a lower volume of between 0.05 ml and 0.2 ml may be used. In one embodiment, a human dose is 0.5 ml. In a further embodiment, a human dose is higher than 0.5 ml, for example 0.6, 0.7, 0.8, 0.9 or 1 ml. In a further embodiment, a human dose is between 1 ml and 1.5 ml. In another embodiment, in particular when the immunogenic composition is for the paediatric population, a human dose may be less than 0.5 ml such as between 0.25 and 0.5 ml.

### Prophylactic and Therapeutic Uses

The present invention also provides methods of treating and/or preventing bacterial infections of a subject comprising administering to the subject a pneumococcal saccharide, conjugate or bioconjugate of the invention. The pneumococcal saccharide, conjugate or bioconjugate may be in the form of an immunogenic composition or vaccine. In an embodiment, the immunogenic composition or vaccine of the invention is used in the prevention of infection of a subject (e.g. human subjects) by *Streptococcus* species (e.g. *Streptococcus pneumoniae*). In an embodiment, the immunogenic composition or vaccine of the invention is used to treat or prevent an infection by *Streptococcus* species (e.g. *Streptococcus pneumoniae*).

Also provided herein are methods of inducing an immune response in a subject against a bacterium, comprising administering to the subject a pneumococcal saccharide, or conjugate or bioconjugate of the invention (or immunogenic composition or vaccine). In one embodiment, said subject has bacterial infection at the time of administration. In another embodiment, said subject does not have a bacterial infection at the time of administration. The pneumococcal saccharide, conjugate or bioconjugate of the invention can be used to induce an immune response against *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species. In an embodiment, pneumococcal saccharide, or conjugate or bioconjugate of the invention is used to induce an immune response against *Streptococcus* species (e.g. *Streptococcus pneumoniae*).

Also provided herein are methods of inducing the production of opsonophagocytic antibodies in a subject against a bacterium, comprising administering to the subject a pneumococcal saccharide, or conjugate or bioconjugate of the invention (or immunogenic composition or vaccine). In one embodiment, said subject has bacterial infection at the time of administration. In another embodiment, said subject does not have a bacterial infection at the time of administration. The pneumococcal saccharide, or conjugate or bioconjugate of the invention (or immunogenic composition or vaccine) provided herein can be used to induce the production of opsonophagocytic antibodies against *Streptococcus pneumoniae*.

In an embodiment, the present invention is an improved method to elicit an immune response in infants (defined as 0-2 years old in the context of the present invention) by administering a therapeutically effective amount of an immunogenic composition or vaccine of the invention (a paediatric vaccine). In an embodiment, the vaccine is a paediatric vaccine.

In an embodiment, the present invention is an improved method to elicit an immune response in the elderly population (in the context of the present invention a patient is considered elderly if they are 50 years or over in age, typically over 55 years and more generally over 60 years) by administering a therapeutically effective amount of the immunogenic composition or vaccine of the invention. In an embodiment, the vaccine is a vaccine for the elderly.

The disease caused by *Streptococcus pneumoniae* infection may be selected from pneumonia, invasive pneumococcal disease (IPD), exacerbations of chronic obstructive pulmonary disease (eCOPD), otitis media, meningitis, bacteraemia, pneumonia and/or conjunctivitis. Where the human host is an infant (defined as 0-2 years old in the context of the present invention), the disease may be selected from otitis media, meningitis, bacteraemia, pneumonia and/or conjunctivitis. In one aspect, where the human host is an infant (defined as 0-2 years old in the context of the present invention), the disease is selected from otitis media and/or pneumonia. Where the human host is elderly (*i.e.,* 50 years or over in age, typically over 55 years and more generally over 60 years), the disease may be selected from pneumonia, invasive pneumococcal disease (IPD), and/or exacerbations of chronic obstructive pulmonary disease (eCOPD). In one aspect, where the human host is elderly, the disease is invasive pneumococcal disease (IPD). In another aspect, where the human host is elderly, the disease is exacerbations of chronic obstructive pulmonary disease (eCOPD).

The present invention provides a pneumococcal saccharide of the invention, or the conjugate of the invention, or the bioconjugate of the invention, or the immunogenic composition or vaccine of the invention for use in the treatment or prevention of a disease caused by *S. pneumoniae* infection. In an embodiment, the disease is pneumonia, invasive pneumococcal disease (IPD), exacerbations of chronic obstructive pulmonary disease (COPD), otitis media, meningitis, bacteraemia, pneumonia and/or conjunctivitis.

The present invention provides provides a pneumococcal saccharide of the invention, or the conjugate of the invention, or the bioconjugate of the invention, or the immunogenic composition or vaccine of the invention, for use in immunizing against infection by *Streptococcus pneumoniae*.

The present invention provides a pneumococcal saccharide of the invention, or the conjugate of the invention, or the bioconjugate of the invention, or the immunogenic composition or vaccine of the invention for inducing an immune response against *Streptococcus pneumoniae*.

The present invention provides use of the pneumococcal saccharide of the invention, or the conjugate of the invention, or the bioconjugate of the invention, or the immunogenic composition or vaccine of the invention in the manufacture of a medicament for the treatment or prevention of a disease caused by *Streptococcus pneumoniae* infection. In an embodiment, the disease is pneumonia, invasive pneumococcal disease (IPD), exacerbations of chronic obstructive pulmonary disease (COPD), otitis media, meningitis, bacteraemia, pneumonia and/or conjunctivitis.

The present invention provides use of a pneumococcal saccharide of the invention, or a conjugate of the invention, or a bioconjugate of the invention, or a immunogenic composition or a vaccine of the invention, in the manufacture of a medicament for immunizing against infection by *Streptococcus pneumoniae*.

The present invention provides use of a pneumococcal saccharide of the invention, or a conjugate of the invention, or a bioconjugate of the invention, or a immunogenic composition or a vaccine of the invention, in the manufacture of a medicament for inducing an immune response against *Streptococcus pneumoniae*.

The present invention provides a method for the treatment or prevention of *Streptococcus pneumoniae* infection in a subject (e.g. human) in need thereof comprising administering to said subject a therapeutically effective amount of the pneumococcal saccharide of the invention, or the conjugate of the invention, or the bioconjugate of the invention, or the immunogenic composition or vaccine of the invention. In an embodiment, the disease is pneumonia, invasive pneumococcal disease (IPD), exacerbations of chronic obstructive pulmonary disease (COPD), otitis media, meningitis, bacteraemia, pneumonia and/or conjunctivitis.

The present invention provides a method of immunizing a subject (e.g. human) against infection by *Streptococcus pneumoniae* comprising administering to the host an immunoprotective amount of the pneumococcal saccharide of the invention, or the conjugate of the invention, or the bioconjugate of the invention, or the immunogenic composition or vaccine of the invention.

The present invention provides a method of inducing an immune response to *Streptococcus pneumoniae* in a subject (e.g. human), comprising administering a therapeutically or prophylactically effective amount of the pneumococcal saccharide of the invention, or the conjugate of the invention, or the bioconjugate of the invention, or the immunogenic composition or vaccine of the invention.

All references or patent applications cited within this patent specification are incorporated by reference herein.

Aspects of the invention are summarized in the following numbered paragraphs:
1. A synthetic (e.g. recombinant) pneumococcal saccharide comprising one or more repeat unit(s) →4)-β-D-Glc*p*-(1→4)-[Gro-(2→P→3)]-β-D-Gal*p*-(1→4)-β-L-Rha*p-*(1→.
2. The pneumococcal saccharide according to paragraph 1, comprising less than 500 repeat units, for example 1 to 200 repeat units (suitably 2 to 200, 1 to 100, 2 to 100, 2 to 50, 2 to 30, 2 to 20 repeat units).
3. The pneumococcal saccharide according to paragraph 1 or paragraph 2 wherein the size (Mw) of the pneumococcal saccharide is less than 300kDa, e.g. between 1-150kDa.
4. The pneumococcal saccharide according to any one of paragraphs 1 to 3 which is a hybrid oligosaccharide or polysaccharide having a structure:

   (B)ₙ-A→

   wherein A is an oligosaccharide containing at least 2, 3, 4, 5, 6, 7 or 8 monosaccharides (e.g. 2 to 10, 2 to 8, 3 to 6, 4 to 5 monosaccharides), with a hexose monosaccharide derivative at the reducing end (indicated by the arrow in the diagram), e.g. wherein the hexose monosaccharide derivative is N-acetylglucosamine (GlcNAc), N-acetylgalactoseamine (GalNAc), 2,4-Diacetamido-2,4,6-trideoxyhexose (DATDH). N-acetylfucoseamine (FucNAc), or N-acetylquinovosamine (*QuiNAc*) (e.g. N-acetylglucosamine (GlcNAc));
   wherein B is an oligosaccharide repeat unit →4)-β-D-Glc*p*-(1→4)-[Gro-(2→P→3)]-β-D-Gal*p*-(1→4)-β-L-Rha*p*-(1→; and
   wherein n is at least 2, 3, 4, 5, 6, 7, 8, 9, 10, 11, 12, 13, 14, 15, 16, 17, 18, 19, or at least 20 (e.g. n is 1 to 100, 2 to 100, 10 to 100, 20 to 100, or 25 to 100).
5. The pneumococcal saccharide according to paragraph 4 wherein A is an oligosaccharide identical to the repeat unit B with the exception that A comprises a hexose monosaccharide derivative at the reducing end of the oligosaccharide in place of the hexose monosaccharide at the reducing end of B (e.g. A is →4)-β-D-GlcNAc*p*-(1→4)-[Gro-(2→P→3)]-β-D-Gal*p*-(1→4)-β-L-Rha*p*-(1→).
6. The pneumococcal saccharide according to paragraph 5 wherein A is an oligosaccharide comprising →4)-β-D-Glc*p*-(1→4)-[Gro-(2→P→3)]-β-D-Gal*p-*(1→4)-β-L-Rha*p*-(1→ and in addition a hexose monosaccharide derivative at the reducing end of the repeat unit (e.g. GlcNAc).
7. A pneumococcal saccharide comprising one or more repeat unit(s) →4)-β-D-Glc*p-*(1→4)-[Gro-(2→P→3)]-β-D-Gal*p*-(1→4)-β-L-Rha*p*-(1→ conjugated to a carrier protein.
8. The pneumococcal saccharide according to any of paragraphs 1 to 7 conjugated to a carrier protein.
9. The pneumococcal saccharide according to paragraph 7 or paragraph 8 conjugated to a carrier protein selected from CRM197, Diphtheria toxoid, tetanus toxoid, detoxified exotoxin A from *P. aeruginosa*, detoxified hemolysin A of *S. aureus*, clumping factor A, clumping factor B, *E. coli* FimH, *E. coli* FimHC, *E. coli* heat labile enterotoxin, detoxified variants of *E. coli* heat labile enterotoxin, Cholera toxin B subunit (CTB), cholera toxin, detoxified variants of cholera toxin, *E. coli* sat protein, the passenger domain of *E. coli* sat protein, *C. jejuni* AcrA, *C. jejuni* natural glycoproteins, *S. pneumoniae* pneumolysin, *S. pneumoniae* NOX, *S. pneumoniae* PspA, *S. pneumoniae* PcpA, *S. pneumoniae* PhtD, *S. pneumoniae* PhtE, *S. pneumoniae* ply (e.g. detoxified ply), or *S. pneumoniae* LytB.
10. The pneumococcal saccharide conjugated to a carrier protein according to any of paragraphs 7-9 wherein the size (Mw) of the conjugate is less than 300kDa, e.g. between 30-220kDa.
11. The pneumococcal saccharide conjugated to a carrier protein according to any of paragraphs 7-10 wherein the pneumococcal saccharide is directly coupled to the carrier protein (e.g. via an N-linked glycosidic bond or via an O-linked glycosidic bond).
12. The pneumococcal saccharide conjugated to a carrier protein according to any of paragraphs 7-11 wherein the carrier protein is attached to a glucose or hexose monosaccharide derivative (e.g. N-acetylglucosamine) of the pneumococcal saccharide.
13. The pneumococcal saccharide according to any of paragraphs 7 to 10, wherein the pneumococcal saccharide (e.g. recombinant pneumococcal saccharide) is covalently linked to the carrier protein through a chemical linkage obtainable using a chemical conjugation method, optionally selected from the group consisting of carbodiimide chemistry, reductive animation, cyanylation chemistry (for example CDAP chemistry), maleimide chemistry, hydrazide chemistry, ester chemistry, and N-hydroysuccinimide chemistry either directly or via a linker.
14. A bioconjugate comprising a pneumococcal saccharide (e.g. recombinant pneumococcal saccharide) according to any one of paragraphs 1 to 6 conjugated to a carrier protein.
15. A host cell comprising:
   (i) one or more nucleic acids that encode glycosyltransferase(s) sufficient for synthesis of the repeat unit →4)-β-D-Glc*p*-(1→4)-[Gro-(2→P→3)]-β-D-Gal*p*-(1→4)-β-L-Rha*p*-(1→;
   (ii) nucleic acid that encodes an oligosaccharyl transferase; and optionally
   (iii) nucleic acid that encodes a carrier protein comprising an N-glycosylation consensus sequence (e.g. detoxified exotoxin A from *P. aeruginosa*, CRM197, Diphtheria toxoid, tetanus toxoid, detoxified hemolysin A of *S. aureus*, clumping factor A, clumping factor B, *E. coli* FimH, *E. coli* FimHC, *E. coli* heat labile enterotoxin, detoxified variants of *E. coli* heat labile enterotoxin, Cholera toxin B subunit (CTB), cholera toxin, detoxified variants of cholera toxin, *E. coli* sat protein, the passenger domain of *E. coli* sat protein, *C. jejuni* AcrA, *C. jejuni* natural glycoproteins, *S. pneumoniae* pneumolysin, *S. pneumoniae* NOX, *S. pneumoniae* PspA, *S. pneumoniae* PcpA, *S. pneumoniae* PhtD, *S. pneumoniae* PhtE, *S. pneumoniae* ply (e.g. detoxified ply), or *S. pneumoniae* LytB); and optionally (iv) nucleic acid that encodes a polymerase (e.g. *wzy*).
16. The host cell according to paragraph 15 wherein said host cell is capable of producing a hybrid oligosaccharide or polysaccharide, wherein said hybrid oligosaccharide or polysaccharide is identical to *S. pneumoniae* CP23B, with the exception of the fact that said hybrid oligosaccharide or polysaccharide comprises a hexose monosaccharide derivative at the reducing end of the first repeat unit in place of the hexose monosaccharide normally present at the reducing end of the first repeat unit of *S. pneumoniae* CP23B, e.g. wherein said hexose monosaccharide derivative is N-acetylglucosamine (GlcNAc), N-acetylgalactoseamine (GalNAc), 2,4-Diacetamido-2,4,6-trideoxyhexose (DATDH), N-acetylfucoseamine (FucNAc), N-acetylquinovosamine (*QuiNAc*) (e.g. N-acetylglucosamine (GlcNAc)).
17. The host cell according to paragraph 16, wherein said host cell comprises nucleic acid encoding (a) an enzyme, e.g. phophotransferase that assembles a hexose monosaccharide derivative onto undecaprenyl monophosphate (Und-P) , optionally wherein said phosphotransferase is *wecA*, optionally from *E. coli*, and (b) one or more glycosyltransferases capable of adding a monosaccharide to the hexose monosaccharide derivative assembled on Und-PP.
18. The host cell according to paragraph 17, wherein said one or more glycosyltransferases capable of adding a monosaccharide to the hexose monosaccharide derivative assembled on Und-PP is a rhamnosyltransferase (e.g. 4-linked β-L-rhamnose), for example the rhamnosyltransferase (*wegR*) from *E. coli* O2 or the rhamnosyltransferase (*wbuV*) from *E. coli* O149 or the rhamnosyltransferase (*wepl*) from *Cronobacter sakazakii* O5.
19. The host cell according to any one of paragraphs 15 to 18 wherein said glycosyltransferases for synthesis of the repeat units comprise *WchA* (glucosyltransferase) (e.g. from *S. pneumoniae* CP23B), *WchF* (rhamnosyltransferase) (e.g. from *S. pneumoniae* CP23B), *WchV* (galactosyltransferase) (e.g. from *S. pneumoniae* CP23B), and *WchX* (e.g. from *S. pneumoniae* CP23B), optionally wherein the glycosyltransferases for synthesis of the repeat units do not comprising *WchW* (rhamnosyltransferase) (e.g. from *S. pneumoniae* CP23B).
20. The host cell according to any one of paragraphs 15 to 19 wherein the oligosaccharyl transferase is heterologous to the host cell, optionally wherein the heterologous oligosaccharyl transferase is integrated into the host cell genome and optionally wherein at least one gene of the host cell has been functionally inactivated or deleted, optionally wherein the *waaL* gene of the host cell has been functionally inactivated or deleted, optionally wherein the *waaL* gene of the host cell has been replaced by nucleic acid encoding an oligosaccharyltransferase.
21. The host cell according to any one of paragraphs 15 to 20, wherein said host cell comprises nucleic acid that encodes a capsular polysaccharide polymerase (e.g. *wzy*) or an O antigen polymerase (e.g. *wzy)*), optionally said capsular polysaccharide polymerase is from *Streptococcus pneumoniae*, optionally from *S. pneumoniae* CP1, CP2, CP4, CP5, CP6 (A, B, C, D), CP7 (A,B,C), CP8, CP9(A,L,N,V), CP10(A,B,C,F), CP11 (A,B,C,D,F), CP12(A,B,F), CP13, CP14 CP15(A,B,C,F), CP16(A,F), CP17(A,F), CP18(A,B,C,F), CP19(A,B,C,F), CP20, CP21, CP22(A,F), CP23(A,B,F), CP24(A,B,F), CP25(A,F), CP26, CP27,CP28(A,F), CP29, CP31, CP32(A,F), CP33(A,B,C,D,F), CP34, CP35(A,B,C,D,F), CP36, CP38, CP39, CP40, CP41(A,F), CP42, CP43, CP44, CP45, CP46, CP47(A,F), or CP48 (e.g. *wzy* polymerase from *S. pneumoniae* CP23B).
22. The host cell according to any one of paragraphs 15 to 21, wherein said host cell comprises nucleic acid that encodes a flippase (*wzx*), optionally wherein said flippase is from *Streptococcus pneumoniae*, optionally from *S. pneumoniae* CP1, CP2, CP4, CP5, CP6(A,B,C,D), CP7(A,B,C), CP8, CP9(A,L,N,V), CP10(A,B,C,F), CP11(A,B,C,D,F), CP12(A,B,F), CP13, CP14 CP15(A,B,C,F), CP16(A,F), CP17(A,F), CP18(A,B,C,F), CP19(A,B,C,F), CP20, CP21, CP22(A,F), CP23(A,B,F), CP24(A,B,F), CP25(A,F), CP26, CP27, CP28(A,F), CP29, CP31, CP32(A,F), CP33(A,B,C,D,F), CP34, CP35(A,B,C,D,F), CP36, CP38, CP39, CP40, CP41(A,F), CP42, CP43, CP44, CP45, CP46, CP47(A,F), or CP48 (e.g. *wzx* flippase from *S. pneumoniae* CP23B).
23. The host cell according to any one of paragraphs 15 to 22, wherein said host cell is engineered so that it produces a repeat unit with a trisaccharide backbone (Glc-Gal-βRha) without a terminal α-Rha.
24. The host cell according to any one of paragraphs 15 to 23, wherein *wchA* attaches a D-glucose-P onto UndP, followed by *WchF* attaching a L-rhamnose onto UndPP-Glucose, followed by *WchV* attaching a D-galactose, followed by *WchX* attaching a glycerol-2-phosphate to the galactose.
25. The host cell according to any one of paragraphs 15 to 24, wherein *Wzx* (e.g. *wzx* flippase from *S. pneumoniae* CP23B) flips a single repeat unit into the periplasm, followed by the *Wzy* polymerase attaching the D-glucose at reducing end of the growing chain to the D-galactose of the single repeating unit, to produce a polysaccharide.
26. The host cell according to any one of paragraphs 15 to 25, wherein said host cell further comprises an enzyme capable of modifying a monosaccharide, optionally an epimerase, optionally wherein said epimerase is from *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species.
27. The host cell according to any one of paragraphs 15 to 26, wherein the host cell is a gram-negative bacterium, optionally selected from the group consisting of *Escherichia* species, *Shigella* species, *Klebsiella* species, *Salmonella* species, *Yersinia* species, *Neisseria* species, *Vibrio* species and *Pseudomonas* species, e.g. *E. coli*.
28. A process of producing a bioconjugate that comprises a recombinant saccharide linked to a carrier protein, said process comprising (i) culturing the host cell according to any one of paragraphs 15 to 27 under conditions suitable for the production of glycoproteins and (ii) isolating the bioconjugate.
29. A bioconjugate produced by the process of paragraph 28.
30. An immunogenic composition comprising a pneumococcal saccharide comprising one or more repeat unit(s) →4)-β-D-Glc*p*-(1→4)-[Gro-(2→P→3)]-β-D-Gal*p*-(1→4)-β-L-Rha*p*-(1→.
31. An immunogenic composition comprising a pneumococcal saccharide according to any one of paragraphs 1 to 13, or a bioconjugate of paragraph 14 or paragraph 29.
32. An immunogenic composition comprising a pneumococcal saccharide comprising one or more repeat unit(s) →4)-β-D-Glc*p*-(1→4)-[Gro-(2→P→3)]-β-D-Gal*p*-(1→4)-β-L-Rha*p*-(1→ conjugated to a carrier protein (e.g. selected from detoxified exotoxin A from *P. aeruginosa* (EPA), TT, DT, CRM197, PhtD, detoxified pneumolysin and protein D, suitably exotoxin A from *P. aeruginosa* (EPA)).
33. The immunogenic composition according to paragraph 30 or paragraph 31 comprising 10 or more, 11 or more, 12 or more, 13 or more, 14 or more, 15 or more, 16 or more, 17 or more, 18 or more, 19 or more, 20 or more capsular saccharide conjugates from different *S. pneumoniae* serotypes.
34. The immunogenic composition according to any of paragraphs 30 to 31 further comprising a pharmaceutically acceptable excipient, carrier or diluent.
35. The immunogenic composition according to any one of paragraphs 30 to 34 further comprising an adjuvant.
36. A method of making the immunogenic composition according to any one of paragraphs 30 to 35, which process comprises the step of mixing the pneumococcal saccharide or the bioconjugate with a pharmaceutically acceptable excipient, carrier, diluent or adjuvant.
37. A vaccine comprising an immunogenic composition any one of paragraphs 30 to 35.
38. A kit comprising a pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37 and instructions for the use thereof.
39. A pneumococcal saccharide according to any one of paragraphs 1 to 13, a bioconjugate according to paragraph 14 or paragraph 29, an immunogenic composition according to any one of paragraphs 30 to 335, or a vaccine according to paragraph 37, for use in the treatment or prevention of a disease caused by *Streptococcus pneumoniae* infection.
40. The pneumococcal saccharide, bioconjugate, immunogenic composition or vaccine for use according to paragraph 39 wherein the disease is pneumonia, invasive pneumococcal disease (IPD), exacerbations of chronic obstructive pulmonary disease (COPD), otitis media, meningitis, bacteraemia, pneumonia and/or conjunctivitis.
41. An pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37, for use in immunizing against infection by *Streptococcus pneumoniae*.
42. A pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37 for inducing an immune response against *Streptococcus pneumoniae*.
43. Use of a pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37, in the manufacture of a medicament for the treatment or prevention of a disease caused by *Streptococcus pneumoniae* infection.
44. The use according to paragraph 42 in the manufacture of a medicament wherein the disease is pneumonia, invasive pneumococcal disease (IPD), exacerbations of chronic obstructive pulmonary disease (COPD), otitis media, meningitis, bacteraemia, pneumonia and/or conjunctivitis.
45. Use of a pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37, in the manufacture of a medicament for immunizing against infection by *Streptococcus pneumoniae*.
46. Use of a pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37, in the manufacture of a medicament for inducing an immune response against *Streptococcus pneumoniae*.
47. A method for the treatment or prevention of a disease caused by *Streptococcus pneumoniae* infection in a subject (e.g. human) in need thereof, comprising administering to said subject a therapeutically effective amount of the pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37.
48. The method of treatment or prevention according to paragraph 46 wherein the disease is pneumonia, invasive pneumococcal disease (IPD), exacerbations of chronic obstructive pulmonary disease (COPD), otitis media, meningitis, bacteraemia, pneumonia and/or conjunctivitis.
49. A method of immunizing a subject (e.g. human) against infection by *Streptococcus pneumoniae* comprising administering to the subject an immunoprotective amount of the pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37.
50. A method of inducing an immune response against *Streptococcus pneumoniae* in a subject (e.g. human), comprising administering to the subject an immunogenic amount of the pneumococcal saccharide according to any one of paragraphs 1 to 13, a bioconjugate according to paragraph 14 or paragraph 29, an immunogenic composition according to any one of paragraphs 30 to 35, or a vaccine according to paragraph 37.

In order that this invention may be better understood, the following examples are set forth. These examples are for purposes of illustration only, and are not to be construed as limiting the scope of the invention in any manner.

### Sequences of proteins and nucleic acids

*SEQ ID NO: 1 - consensus sequence*
   *D*/*E-X-N-Z-S*/*T*
*SEQ ID NO: 2 - consensus sequence*
   *D-Q-N-A-T*
*SEQ ID NO: 3 - consensus sequence*
   *K-D*/*E-X-N-Z-S*/*T-K*
*SEQ ID NO: 4 - consensus sequence*
   *K-D-Q-N-A-T-K*
*SEQ ID NO: 5 - Streptococcus pneumoniae 23A wzg* 
MSRRFKKSGSQKVKRSVNIVLLTIYLLLVCFLLFLIFKYNILAF
RYLNLVVTALVLLVALVGLLLIIYKKAEKFTIFLLVFSILVSSVSLFAVQQFVGLTNR
LNATSNYSEYSISVAVLADSDIENVTQLTSVTAPTGTDNENIQKLLADIKSSQNTDLT
VNQSSSYLAAYKSLIAGETKAIVLNSVFENIIESEYPDYASKIKKIYTKGFTKKVEAP
KTSKNQSFNIYVSGIDTYGPISSVSRSDVNILMTVNRDTKKILLTTTPRDAYVPIADG
GNNQKDKLTHAGIYGVDSSIHTLENLYGVDINYYVRLNFTSFLKLIDLLGGIDVYNDQ
EFTAHTNGKYYPAGNVHLDSEQALGFVRERYSLADGDRDRGRNQQKVIVAILQKLTST
EALKNYSTIINSLQDSIQTNMPLETMINLVNAQLESGGNYKVNSQDLKGTGRTDLPSY
AMPDSNLYVMEIDDSSLAVVKAAIQDVMEGR
*SEQ ID NO: 6 - Streptococcus pneumoniae 23A wzh* MIDIHSHIVFDVDDGPKSREESKALLTESYRQGVRTIVSTSHRR 
KGMFETPEEKIAENFLQVREIAKEVADDLVIAYGAEIYYTLDALEKLEKKEIPTLNDS
RYALIEFSMNTPYRDIHSALSKILMLGITPVIAHIERYDALENNGKRVRELIDMGCYT
QINSYHVSKPKFFGEKYKFMKKRARYFLERDLVHVVASDMHNLDSRPPYMQQAYDIIA
KKYGAKKAKELFVDNPRKIIMDQLI
*SEQ ID NO: 7 - Streptococcus pneumoniae 23A wzd* 
MKEQNTLEIDVLQLFRALWKRKLVILLVAIITSSVAFTYSTFVI
KPEFTSTTRIYVVNRNQGEKSGLTNQDLQAGTYLVKDYREIILSQDVLEEVVSDLKLD
LTPKGLANKIKVTVPVDTRIVSVSVNDRVPEEASRIANSLREVAAQKIISITRVSDVT
TLEEARPAISPSSPNIKRNILIGFLAGVIGTSVIVLLLELLDTRVKRPEDIEDTLQMT
LLGVVPNLNKLK
*SEQ ID NO: 8 - Streptococcus pneumoniae 23A wze* 
MPTLEIAQKKLEFIKKAEEYYNALCTNIQLSGDKLKVISVTSVS
PGEGKTTTSVNIAWSFARAGYKTLLIDGDTRNSVISGFFKSREKITGLTEFLSGTADL
SHGLCDTNIENLFVVQSGSVSPNPTALLQSKNFNDMIETLRKYFDYIIVDTAPIGIVI
DAAIITQKCDASILVTATGEANKRDVQKAKQQLKQTGKLFLGVVLNKLDISVDKYGVY
GFYGNYGKK
*SEQ ID NO: 9 - Streptococcus pneumoniae 23A wchA* 
MDEKGLKIFMAVLQSIIVILLVYFLSFVRETELERSSMVILYLL
HFFVFYVSSYGNNFFKRGYLVEFNSTIRYIFFFAIAISVLNFFIAERFSISRRGMVYF
LTLEGISLYLLNFLVKKYWKHVFFNLKNSKKILLLTVTKNMEKVLDKLLESDELSWKL
VAVSVLNKSDFQHDKIPVIEKEKIIEFATHEVVDEVFVNLPGESYDIGEIISRFETMG
IDVTVNLKAFDKNLGRNKQIHEMVGLNVVTFSTNFYKTSHVISKRILDICGATIGLIL
FAIASLVLVPLIRKDGGPAIFAQTRIGKNGRHFTFYKFRSMRIDAEAIKEQLMDQNTM
QGGMFKIDNDPRVTKIGRFIRKTSLDELPQFWNVFIGDMSLVGTRPPTVDEYDQYTPE
QKRRLSFKPGITGLWQVSGRSKITDFDDVVKLDVSYIDNWTIWKDIEILLKTVKVVFM
RDGAK
*SEQ ID NO: 10 - Streptococcus pneumoniae 23A wchF* 
MKKSVYIIGSKGIPAKYGGFETFVEKLTAFQQDKAIQYYVACMR
ENSAKSGTTEDVFEHNGAICYNVDVPNIGPARAIVYDIAAINRAIEIAKENKDEDPIF
YILACRIGPFIHGIKKKIQAIGGTLLVNPDGHEWLRAKWSTPVRRYWKISEGLMVKHA
DLLVCDSKNIEQYIQEDYKQFQPKTTYIAYGTDTTRSILKSSDEKVRSWFKEKNVSEN
EYYLVVGRFVPENNYESMILGFLASNSKKDFVLITNVEQNKFYNQLLAKTGFDKDPRV
KFVGTVYNQELLKYIRENAFAYFHGHEVGGTNPSLLEALASTKLNLLLDVGFNREVAE
DGADYWEKDNLHKVIEASEQKTQEEINEKNILSTKQVTERFSWDLIVNEYEKLFTRKN
*SEQ ID NO: 11 - Streptococcus pneumoniae 23A wzy* 
MRYGIMRISFSKKTMLCGLLYIGLILSVVTIPTIVTFLYSLLFI
GIVTVLNYNSILASDEDANSFFVALPIILSSFQNVYLGFGADRLNSVTLQVLLSISIA
IITITVFLGIILNRFKSKEFSWLVLSILVIIIQSVILLIFFPTTLPAYLSSMRNILAP
LLIFYFSIYGFKNINLQKFYKYMFIIILVVLIFGFIEYIYGNSLWTRLNIKKLWALKG
LAIENRVVPGNWHSSELIGGKQLRRMVSTFADPVNLGSYLFAAFMLAWYKNKKLLQVL
LLASFVLSVSKAAFLSMLVYIIIYTWVVDKNKILSIFGIIISTVLGLYFYNFSQVSSY
GSINAHIDGFFSALSTPLHYPFGMGVGSVGVLASKLGSQTALSSEVLETGIGMIIAQL
GFVGVIIYLIFFVKLSVIGKNINNKRDKILWFTLIYSFLANAFFNEVALSPNSCTLYF
LILGLLYNKNKIRSTEFS
*SEQ ID NO: 12 - Streptococcus pneumoniae 23A wchV* 
MEKLVSIILPVYNVEQYIKNCLESIQQQTYPNLEVIIVNDGSTD
KSVEYCEQICKIDSRFSITHKENGGLSDARNVGIDKAKGDYLIFVDSDDFVSQDMVSY
LVSCMENNEADIAICDPVHYYSDRQNNDLNIFSPASSVKVYETTEALCEMFYQKSFLV
SAWAKIFKRELFDDIRFPVGKLFEDSAIMYLLLEKCETIAYSDAKLYAYVHRDNSITT
KKFSDRDLDILEITNTIINHYGDNLRVYTAAVSYKVSACFRILLNSPSEEKYKKVQKE
CLSYILQNWRNILFNNNVRLKNKLALISITIFNPFVKLIYSKVNRWE"
*SEQ ID NO: 13 - Streptococcus pneumoniae 23A wchW* 
MNKYEERYQENLSKNDFYKLINKSYLSDKELQVQQVKAGIVLPP
KAFETKLSNKLGLQKSLHGKGGVVDSNGNYIELSAQKAVGMRNRVYGPYKINYDNLPI
RNEKVIYLNYFIKQWGHFLLDVVGRLWYPLLQDNDTKLVYTCYAGTETKIEGNYLEFL
KLLGIDQSRLIMINCPTQFSEVIIPESSILPGGYYTKEYKQLFSSVVENIKLDKYDVN
AKMIYCSRSKLGIAKSKEFGEDGIEGIFKQNGYTSVYMETMSLEEQIKTLLSAKTIVL
TSGSLAHNLLFVNKDIDVFILNKTYRVNLHQFLINEISDATVRFVDIYRSPLPILYGY
GPFLMDLTKPLANFLDDNEFVYEKGTVLSKKDYFKYYLKWLWSYRFFLFRLNGIKEGN
SEFEKSFKIIRRYYKTGR
*SEQ ID NO: 14 - Streptococcus pneumoniae 23A wzx* 
MSKYKELAKNTGIFALANFSSKILIFLLVPIYTRVLTTTEYGFY
DLVYTTIQLFVPILTLNISEAVMRFLMKDGVSKKSVFSIAVLDIFIGSIAFALLLLVN
NLFSLSDLISQYSIYIFVIFVFYTLNNFLIQFSKGIDKIGVTAISGVISTAVMLAMNV
ILLVVFDWGLLGFFIANVCGYVIPCIYIVSRLRLWELFEIKIDKKLQWEMVYYALPLV
LNILSWWVNNTSDRYIVTAIVGIQASAIISVAYKIPQILSTISAIFIQSWQISAIKIQ
EDKSGTTFVSNMLLYYNALLLIIASGIILFVKPISNILFGISFYSAWELVPFLIISSL
FNAISGCIGAIMGAKMDTHNIAKSALVGMIANIILNIVLTFLMGPQGITISTLIASFL
 
IFYMRKDSVKEINSETYRAIYLSWILLVVEACLLIYMDFIIGALIAMVINLFLLKDVI
KPLYLKIFKRN
*SEQ ID NO: 15 - Streptococcus pneumoniae 23A wchX* 
MIVLQYFKILARFVFMFLISAVLLPFKIKPNKIVFINFNGKGYG
DNPKSICEYLRTTYPDLDLVWLARDNEGFPDGVRVVKYGTFQAFYEQASSKVWVYNVR
AFARILKKRGQIYIQTWHGASSFKLIEKQADLPINYVLEAKYDARVTDIMISDSRKQT
EEFQKYFWYSGEIFEVGMPRNDALFHYKEDYDKLNNIRKELSIHSDDYVILYAPTFRD
DGDASYLDINFERLLQCVEHGIKKKCKFLIRLHPNHSHLCNNISFNKNIINATFYSDM
QELTLLADVLVTDYSSSIFDFMLLNKPYVRYVNDLEKYAELRGVSDTYYELPDSIIKT
AEELYDLLPKKIENFDYDSIKKYRNEILCPIFNGTASENVGRRIIQEL
*SEQ ID NO: 16 - Streptococcus pneumoniae 23A gtp1* 
MKNNDLKIGSGAIHQISATLSQNSISGKILYCADPVVDDLYGSI
VRSQIEEIGRVKEESCNYNTIAYAMNIAERAIATDIDCIVGMGGGRVLDVCKYASFIS
KRPYLSIPTTAANDGIASPVAVLKRQDDRPKSLGAAIPSMTLIDIDVIASGPIQNIKA
GIGDTISNYTALKDWELAVERGKDEMHGFAYLMSQNSLDALMKTKYNSITPDFIEVLV
NSLVLSGIAMDFAGSSRPVSGSEHLFSHALDYYGSTRNLHGIQVALGTVAVLKLIENS
VDTVVDYLQRFEVHINPKLLGIDEELFIYCMQHATKMRSNRYTYLHEVDLSTDRLKQI
YKELISEL
*SEQ ID NO: 17 - Streptococcus pneumoniae 23A gtp2* 
MKALILAAGLGTRLAPITNEVPKSLVPVNGKPILMKQIENLYQN
NITDITIIAGYKSSVLTDAVTEKYPEINIIDNVDFKTTNNMYSAYLGKAAMGDSDFLM
MNADVFYDASVIKSLLLHKAPNAIVTDLGIYIEESMKVVEKNGRLVEISKQISPEETL
GASIDVYKFSYEAGARFFEKCKEFIEDKRELQMWSEVALNAILSEVEFVACPLEGRWL
EIDNHEDLVAAEKLFA
*SEQ ID NO: 18 - Streptococcus pneumoniae 23A gtp3* 
MKLTNRVDYFGADISELQNKKLFLFDMDGTIYEEDRLFEGTLEL
LDYIHNIGGEYIFITNNSSKSVVDYVEKVNRLGIKAERDNFFTSAQATIVYIKENYPK
SKVYCQGTKSLIKELSDAGIDVTEQVSADIDVVLVGFDTELTSDKIRNTCEILSTKDV
PFIATNPDIRCPVSFGFIPDCGSICDMISKSVDRKPVYIGKPEPTMVDIVRKKLNYSL
FETVVIGDRLYTDIMTGINAGVTSVCVLTGEATVNDIQQDSIKPTYTFKNVKEMWKGI
V
*SEQ ID NO: 19 - Streptococcus pneumoniae 23A rmlA* 
MKGIILAGGSGTRLYPLTRAASKQLMPVYDKPMIYYPLSTLMLA
GIRDILIISTPQDLPRFKELLQDGSEFGIKLSYAEQPSPDGLAQAFIIGEEFIGDDSV
ALILGDNIYHGPGLSTMLQKAAKKEKGATVFGYHVKDPERFGVVEFDENMNAISIEEK
PEYPRSNYAVTGLYFYDNDVVEIAKSIKPSPRGELEITDVNKAYLDRGDLSVELMGRG
FAWLDTGTHESLLEASQYIETVQRMQNVQVANLEEIAYRRGYISREDVLALAQSLKKN
EYGQYLLRLIGEA
*SEQ ID NO: 20 - Streptococcus pneumoniae 23A rmlC* 
MTDNFFGKTLAARKVEAIPGMLEFDIPVHGDNRGWFKENFQKEK
MLPLGFPESFFAEGKLQNNVSFSRKNVLRGLHAEPWDKYISVADGGKVLGSWVDLREG
ETFGNTYQTVIDASKGIFVPRGVANGFQVLSDTVSYSYLVNDYWALELKPKYAFVNYA
DPSLGIEWENIAEAEVSEADKNHPLLKDVKPLKKEDL
*SEQ ID NO: 21 - Streptococcus pneumoniae 23A rmlB* 
MTEYKNIIVTGGAGFIGSNFVHYVYENFPDVHVTVLDKLTYAGN
RANIEEILGNRVELVVGDIADAELVDKLAAQADAIVHYAAESHNDNSLNDPSPFIHTN
FIGTYTLLEAARKYDIRFHHVSTDEVYGDLPLREDLPGHGEGPGEKFTAETKYNPSSP
YSSTKAASDLIVKAWVRSFGVKATISNCSNNYGPYQHIEKFIPRQITNILSGIKPKLY
GEGKNVRDWIHTNDHSSGVWTILTKGQIGETYLIGADGEKNNKEVLELILKEMGQAVD
AYDHVTDRAGHDLRYAIDASKLRDELGWKPEFTNFEAGLKATIKWYTDNQEWWKAEKE
AVEANYAKTQEIITV
*SEQ ID NO: 22 - Streptococcus pneumoniae 23A rmlD* 
MILITGANGQLGTELRYLLDERNEEYVAVDVAEMDITDAEMVEK
VFEEVKPTLVYHCAAYTAVDAAEDEGRELDFAINVTGTKNVAKASEKHGATLVYISTD
YVFDGKKPVGQEWEVDDRPDPQTEYGRTKRMGEELVEKHVSNFYIIRTAWVFGNYGKN
FVFTMQNLAKTHKTLTVVNDQYGRPTWTRTLAEFMTYLAENRKEFGYYHLSNDATEDT
TWYDFAVEILKGTDVEVKPVDSSQFPAKAKRPLNSTMSLAKAKATGFVIPTWQDALQE
FYKQEVR
*SEQ ID NO: 23 - Streptococcus pneumoniae 23B wzg* 
MLIMSRRFKKSGSQKVKRSVNIVLLTIYLLLVCFLLFLIFKYNI
LAFRYFNLVVTALVLLVALVGLLLIIYKKAEKFTIFLLVFSILVSSVSLFAVQQFVGL
TNRLNATSNYSEYSISVAVLADSDIENVTQLTSVTAPTGTDNENIQKLLADIKSSQNI
DLTVNQSSSYLAAYKSLIAGETKAIVLNSVFENIIESEYPDYASKIKKIYTKGFTKKV
EAPKTSKNQSFNIYVSGIDTYGPISSVSRSDVNILMTVNRDTKKILLTTTPRDAYVPI
ADGGNNQKDKLTHAGIYGVDSSIHTLENLYGVDIHYYVRLNFTSFLKLIDLLGGVDVY
NDQDFTSLHGKFHFPVGNVHLDSEQALGFVRERYSLADGDHDRGRNQQKVIAAILQKL
 
TSSEALKNYSMIIDSLQDSIQTNMPLETMINLVNAQLESGGTYKVNSQDLKGRGRTDL
PSYAMPDSNLYMMEINDSSLASVKTAIQDVLEGR"
*SEQ ID NO: 24 - Streptococcus pneumoniae 23B wzh* 
MHLSKLLFRMCWRADEMIDIHSHIVFDVDDGPKSREESKALLTE
AYRQGVRTIVSTSHRRKGMFETPEEKIAENFLQVREIAKEVASDLVIAYGAEIYYTPD
VLGKLEKNRIPTLNNSRYALIEFSMNTPYRDIHSALIKILMLGITPVIAHIERYDALE
NNEKRVRELINMGCYTQVNSSHVLKSKLFGEPYKFMKKRAQYFLERDLVHVIASDMHN
VDSRPPHMAEAYDLVSQKYGETKAQDLFIDNPRKIVMDQLI"
*SEQ ID NO: 25 - Streptococcus pneumoniae 23B wzd* 
MKEQNTIEIDVFQLLKTLWKHKLIIILLVALVTGAGAFAYSIFIV
KPEYTSTTRIYVVNRNQENKPGLTNQDLQAGTYLVKDYHEIILSQDVLEKVATNLKLD
IPVKTLTSKVQVTVPADTRIVSISVKDKQPEEASRIANSIREVAAEKIIAVTRVSDVT
TLEEARPATTPSSPNVRRNTLVGFLGAAAVTVITVLLIELFDTRVKRPEEVEDVLQMP
LLGVVPDFNKMK
*SEQ ID NO: 26 - Streptococcus pneumoniae 23B wze* 
MPTLEISQAKLELAKKTEEYYNALCTNPQLSGDDLKVFSISSVK
AGEGKTTTSTNIAWAFAHAGYKTLLIDADMRNSVMSGVFKSRERITGLTEFLSGTTDL
SQGLCDTNVENLFVIQAGSVSPNPIALLQSKNFSTMLGTLRKYFDYIVVDTAPIGIVI
DAAIIMQKCDASILVTKAGETKRRELQKAKEQLEQTGKSCLGVVLNKFDTSVDKYGFY
GSYGSYRKQKK
*SEQ ID NO: 27 - Streptococcus pneumoniae 23B wchA* 
MNEKLAKSSVAIVQSFLVILLTYLLSAVRETEIVSTTAIVLYIL
HYFVFYISDYGRNFFKRRYLIELVQTLKYILFFALAISISNFFLEDRFSISRRGMIYF
LLLHVFLVYMLNLFIKWYWKRAYPNFKGSKKVFLLTATSHVEKVLDRLIESDDVVGEL
VAVSVLDKPDFQHDDLKVVAEGEIVNFATREVVDEVFINLPSEKYNIGELVSQFETMG
IDVTVNLNAFDWARNKQICEMAGLNVVTFSTTFYKTSHVIAKRVIDIIGSLVGLILCG
LVSIVLVPLIRKDGGSAIFAQTRIGKNGRHFTFYKFRSMCVDAEDKKRELMEQNTMQG
GMFKVDDDPRITKIGHFIRKTSLDELPQFYNVLKGDMSLVGTRPPTVDEYEHYTPEQK
RRLSFKPGITGLWQVSGRSEIKNFDEVVKLDVVYIDGWTIWKDIEILLKTVKVVLMKD
GAK
*SEQ ID NO: 28 - Streptococcus pneumoniae 23B wchF* 
MERNSLLLFQTIRRKMKKSVYIIGSKGIPAKYGGFETFVEKLTE
YQKDGNIQYYVACMRENSAKSGFTADTFEYNDAICYNIDVPNIGPARAIAYDIAAVNK
AIEIAKKNKDEAPIFYILACRIGPFIARLKKKIQAIGGTLFVNPDGHEWLRAKWSLPV
RKYWKFSEQLMVKYADLLVCDSKNIEKYIQNDYKQYQPKTTYIAYGTDTSPSILKSED
LKIRSWYQEKGLSENGYYLVVGRFVPENNYETMIREFIKSKSKKDFVLITNVEQNKFY
DQLLQETGFDKDPRVKFVGTVYDQELLKYIRENAFAYFHGHEVGGTNPSLLEALASTK
LNLLLDVGFNREVGEDGAIYWRKDNLHKVIEESEQKTIEEIKEIDILSTEQVEKRFTW
DFIVNEYENLFLLGK
*SEQ ID NO: 29 - Streptococcus pneumoniae 23B wzy* 
MTIKINYMFFVCLSFFGIVLSSSQVIVNLGLSSIVQYIAYFLLL
LCIFFTLIKNSPDVIANRIAYFSIISFLFIIGINLQNLPFSTKIYLSFSMLIISSLST
LPIKLINNINDFRRISYFLLNGILLSTFLGWLFNISLVTVAVEGIGFAYGFNGGLTHK
NFYAITILVSYILLFISRKHGTKYQVDSLVLWFDLFLLLVSNTRTIYIILVVFWIVVH
SGFIKYIKKNHRPVIITTWLVISLLSIIFFFKHIINNSESYTHRVLGIVNFFKYYESS
KFHLFFGDAELAFGDMTKGYTHNIRSVLGWDGTVEMPLLSVMIKNGYVGLIGYGVVLF
KFISSVLSMEDRRVKNIGLSILIPLLLSAMVENYIVNISFVFMPVCFCILCSIKNIEF
KNN
*SEQ ID NO: 30 - Streptococcus pneumoniae 23B wchV* 
MKKVSIILPVYNVEQYIKKCLESIQQQTYPNLEVIIVNDGATDK
SVEYCEQICKIDSRFSVTHKENGGLSDARNVGIDKAKGDYLIFVDSDDFVSQDMVSYL
VSSMENNEADIAICDPAHYYSDRQNNDLNIFYPASSVKVYEKTEALCEMFYQKSFLVS
AWAKIYKKELFDDIRFPVGKLFEDSAVMYLLFEKCEKIVYSNAKLYAYVHRDNSITTK
KFSDKDLDILDISNTILDHYSGNFRVYKAAVSYKVSACFRILLNSSSEKKYNQIQKDC
MTYILRNWRNMLFDKNVRLKNKLALISITLFNPFVKFIYSKVNRWE
*SEQ ID NO: 31 - Streptococcus pneumoniae 23B wchW* 
MNKYEERYQEDLSKNDFEKLINRRYLSDKELQVEYVKKGTVLPP
KVFEMKLSNKLGLQKALHGKGGVVDSKGNYVELSEQKAVGMRNRVYGSYKFNHKNLAI
RNEKVIYLNYFINQWGHFLLDVVGRLWYPLLKDTDTKLDYTCYAGTETKLEGNYLEFL
ELLGIDKSRLILINRPTQFSEIIIPESSILPGEYYTKEYKMLFNSLVANVKLDNNLES
KKIYCSRARLDLAKGKEFGENGIEKVFLKNGYTPVYMETMSLKEQIRTLLSATTIVLT
SGSLAHNLLFINNKINVFILNKTYRVNLHQFLINKISEASVSFVDIYRSPLPILYGYG
PFLMDITKPLVNFFEDSGFTYDSGTILDKTDYFKFYLKWLWSYKFFLFRLNHIKEGNS
EFEKSFKIIRRYYKMGRQYE
*SEQ ID NO: 32 - Streptococcus pneumoniae 23B wzx* 
MSKYKELAKNTGTFALANFSSKILIFLLVPIYTKVLTTTEYGFY
DLVYTTIQLLVPILTLNISEAVMRFLMKEDVSKKSVFSIAILDIFLGSIIFCLLLLVN
QIFSLSELISQYSIYIMAIFAFYTLNNFLIQYSKGIDKIGVTAISGVISAAVMLSMNI
LLLVVLNWGLLGFFIANICGYVIPCVYIIVKLKLWDLFELKIDRSLQWEMIYYTLPLI
LNTLSWWVNNTSDRYIITVIIGIQASAIISVAYKIPQIFSTISAIFIQSWQISAIKIQ
EEKEGNTFISKMLLYYNALLLIIASGIILFVKPISNILFGASFYSAWTLVPFLIISSL
FNAISGYIGAIMGAKMDTKNIAKSALVGMIANVFLNIVLTFLMGLQGITISTMIASFL
IFYMRKDSVEEIAPETYRAIYLSWFLLVVEASLLVYIDFIIGATLVTLINLFLLKDTL
KPLCLKLLKGFK
*SEQ ID NO: 33 - Streptococcus pneumoniae 23B wchX* 
MKMNILQYIKILARTIFMLLISTVLLPVRLKNNKILFINFNGKG
YGDNPKSICEYLRTTYPDLDLVWLARDNEGFPDGVRVVKYGTFQAFYEQASSKVWVYN
VRAFARILKKRGQIYIQTWHGASSFKLIEKQADLPINYVLEAKYDARVTDIMISDSRK
QTEEFQKYFWYSGEIFEVGMPRNDALFHYKEDYDKLNNIRKELSIHSDDYVILYAPTF
RDDGDASYLDINFERLLQCVEHGIKKKCKFLIRLHPNHSHLCNNISFNKNIINATFYS
DMQELTLLADVLVTDYSSSIFDFMLLNKPYVRYVNDLEKYAELRGVSDTYYELPDSII
KTAEELYDLLPKKIENFDYDSIKKYRNEILCPIFNGTASENVGRRIIQEL
*SEQ ID NO: 34 - Streptococcus pneumoniae 23B gtp1* 
MKNNDLKIGSGAIHQISATLSQNSISGKILYCADPVVDDLYGSI
VRSQIEEIGRVKEESCNYNTIAYAMNIAERAIATDIDCIVGMGGGRVLDVCKYASFIS
KRPYLSIPTTAANDGIASPVAVLKRQDDRPKSLGAAIPSMTLIDIDVIASGPIQNIKA
GIGDTISNYTALKDWELAVERGKDEMHGFAYLMSQNSLDALMKTKYNSITPDFIEVLV
NSLVLSGIAMDFAGSSRPVSGSEHLFSHALDYYGSTRNLHGIQVALGTVAVLKLIENS
VDTVVDYLQRFEVHINPKLLGIDEELFIYCMQHATKMRSNRYTYLHEVDLSTDRLKQI
YKELISEL
*SEQ ID NO: 35 - Streptococcus pneumoniae 23B gtp2* 
MKALILAAGLGTRLAPITNEVPKSLVPVNGKPILMKQIENLYQN
NITDITIIAGYKSSVLTDAVTEKYPEINIIDNVDFKTTNNMYSAYLGKAAMGDSDFLM
MNADVFYDASVIKSLLLHKAPNAIVTDLGIYIEESMKVVEKNGRLVEISKQISPEETL
GASIDVYKFSYEAGARFFEKCKEFIEDKRELQMWSEVALNAILSEVEFVACPLEGRWL
EIDNHEDLVAAEKLFA
*SEQ ID NO: 36 - Streptococcus pneumoniae 23B gtp3* 
MNRIRRMKLTNRVDYFGADISELQNKKLFLFDMDGTIYEEDRLF
EGTLELLDYIHNIGGEYIFITNNSSKSVVDYVEKVNRLGIKAERDNFFTSAQATIVYI
 
KENYPKSKVYCQGTKSLIKELSDAGIDVTEQVSADIDVVLVGFDTELTSDKIRNTCEI
LSTKDVPFIATNPDIRCPVSFGFIPDCGSICDMISKSVDRKPVYIGKPEPTMVDIVRK
KLNYSLFETVVIGDRLYTDIMTGINAGVTSVCVLTGEATVNDIQQDSIKPTYTFKNVK
EMWKGIV
*SEQ ID NO: 37 - Streptococcus pneumoniae 23B rmlA* 
MKGIILAGGSGTRLYPLTRAASKQLMPVYDKPMIYYPLSTLMLA
GIRDILIISTPQDLPRFKELLQDGSEFGIKLSYAEQPSPDGLAQAFIIGEEFIGDDSV
ALILGDNIYHGPGLSTMLQKAAKKEKGATVFGYHVKDPERFGVVEFDENMNAISIEEK
PEYPRSNYAVTGLYFYDNDVVEIAKSIKPSPRGELEITDVNKAYLDRGDLSVELMGRG
FAWLDTGTHESLLEASQYIETVQRMQNVQVANLEEIAYRMGYISREDVLALAQPLKKN
EYGQYLLRLIGEA
*SEQ ID NO: 38 - Streptococcus pneumoniae 23B rmlC* 
MTDNFFGKTLAARKVEAIPGMLEFDIPVHGDNRGWFKENFQKEK
MLPLGFPESFFAEGKLQNNVSFSRKNVLRGLHAEPWDKYISVADGGKVLGSWVDLREG
ETFGNTYQTVIDASKGIFVPRGVANGFQVLSDTVSYSYLVNDYWALELKPKYAFVNYA
DPSLGIEWENIAEAEVSEADKNHPLLKDVKPLKKEDL
*SEQ ID NO: 39 - Streptococcus pneumoniae 23B rmlB* 
MTEYKNIIVTGGAGFIGSNFVHYVYENFPGVHVTVLDKLTYAGN
RANIEEILGNRVELVVGDIADAELVDKLAAQADAIVHYAAESHNDNSLNDPSPFIHTN
FIGTYTLLEAARKYDIRFHHVSTDEVYGDLPLREDLPGHGEGPGEKFTAETKYNPSSP
YSSTKAASDLIVKAWVRSFGVKATISNCSNNYGPYQHIEKFIPRQITNILSGIKPKLY
GEGKNVRDWIHTNDHSSGVWTILTKGQIGETYLIGADGEKNNKEVLELILKEMGQATD
AYDHVTDRAGHDLRYAIDASKLRDELGWKPEFTNFEAGLKATIKWYTDNQEWWKAEKE
AVEANYAKTQEIITV
*SEQ ID NO: 40 - Streptococcus pneumoniae 23B rmlD* 
MILITGANGQLGTELRYLLDERNEEYVAVDVAEMDITNEEMVEK
VFEEVKPTLVYHCAAYTAVDAAEDEGKELNFAINVTGTKNVAKASEKHGATLVYISTD
YVFDGKKPVGQEWEVDDRPDPQTEYGRTKRMGEELVEKHVSNFYIIRTAWVFGNYGKN
FVFTMQNLAKTHKTLTVVNDQYGRPTWTRTLAEFMTYLAENRKEFGYYHLSNDATEDT
TWYDFAVEILKDTDVEVKPVDSSQFPAKAKRPLNSTMSLAKAKATGFVIPTWQDALQE
FYKQEVR
*SEQ ID NO: 41 - Streptococcus pneumoniae 23F wzg* 
MSRRFKKSRSQKVKRSVNIVLLTIYLLLVCFLLFLIFKYNILAF
RYLNLVVTALVLLVALVGLLLIIYKKAEKFTIFLLVFSILVSSVSLFAVQQFVGLTNR
LNATSNYSEYSISVAVLADSDIENVTQLTSVTAPTGTDNENIQKLLADIKSSQNTDLT
VDQSSSYLAAYKSLIAGETKAIVLNSVFENIIESEYPDYASKIKKIYTKGFTKKVEAP
KTSKNQSFNIYVSGIDTYGPISSVSRSDVNILMTVNRDTKKILLTTTPRDAYVPIADG
GNNQKDKLTHAGIYGVDSSIHTLENLYGVDINYYVRLNFTSFLKMIDLLGGVDVHNDQ
EFSALHGKFHFPVGNVHLDSEQALGFVRERYSLADGDRDRGRNQQKVIVAILQKLTST
EALKNYSTIIDSLQDSIQTNMPLETMINLVNAQLESGGNYKVNSQDLKGTGRTDLPSY
AMPDSNLYVMEIDDSSLAVVKAAIQDVMEGR
*SEQ ID NO: 42 - Streptococcus pneumoniae 23F wzh* 
MIDIHSHIVFDVDDGPKSREESKALLTESYRQGVRTIVSTSHRR
KGMFETPEEKIAENFLQVREIAKEVADDLVIAYGAEIYYTLDALEKLEKKEIPTLNDS
RYALIEFSMNTPYRDIHSALSKILMLGITPVIAHIERYDALENNEKRVRELIDMGCYT
QVNSSHVLKPKLFGERYKFMKKRAQYFLEQDLVHVIASDMHNLDGRPPHMAEAYDLVT
QKYGEAKAQELFIDNPRKIVMDQLI
*SEQ ID NO: 43 - Streptococcus pneumoniae 23F wzd* 
MMKEQNTIEIDVFQLFKTLWKRKLMILIVALVTGTGAFAYSTFI
VKPEYTSTTRIYVVNRNQGDKPGLTNQDLQAGTYLVKDYREIILSQDALEKVATNLKL
DMPAKTLASKVQVAVPADTRIVSISVKDKQPEEASRIANSLREVAAEKIVAVTRVSDV
TTLEEARPATTPSSPNVRRNSLFGFLGGAVVTVIAVLLIELLDTRVKRPEDVEDVLKI
PLLGLVPDFDKIK
*SEQ ID NO: 44 - Streptococcus pneumoniae 23F wze* 
MPTLEISQAKLDSVKKAEEYYNALCTNLQLSGDGLKVFSITSVK
IGEGKSTTSANIAWAFARAGYKTLLIDGDIRNSVMLGVFKARNKITGLTEFLSGTTDL
SQGLCDTNIENLFVIQAGSVSPNPTALLQSKNFTTMLETLRKYFDYIIVDTAPVGVVI
DAAIITRNCDASILVTEAGEINRRDIQKAKEQLEHTGKPFLGIVLNKFDTSVDKYGSY
GNYGNYGKNKK
*SEQ ID NO: 45 - Streptococcus pneumoniae 23F wchA* 
MNEKILRSSLAIIQSFLVILLTYLLSAVRETEIVSTTAIALCIL
HYFVFYISDYGQDFFKRRYLIELVQTLKYILFFALAIGISNFFLEDRFSISRRGMIYF
LTLHALLVYVLNLFIKWYWKRAYPNFKGSKKILLLTATSRVEKVLDRLIESNEVVGKL
VAVSVLDKPDFQHDCLKVVAEGGIVNFATHEVVDEVFINLPSEKYNIGELVSQFETMG
IDVIVNLNAFDRSLARNKQIREMAGLNVVTFSTTFYKTSHVIAKRIIDIVGALVGLIL
CGLVSIVLVPLIRKDGGSAIFAQTRIGKNGRQFTFYKFRSMCVDAEAKKRELMEQNTM
 
QGGMFKVDDDPRITKIGCFIRKTSLDELPQFYNVLKGDMSLVGTRPPTVDEYEHYTPE
QKRRLSFKPGITGLWQVSGRSEIKNFDEVVKLDVAYIDGWTIWKDIEILLKTVKVVFM
RDGAK
*SEQ ID NO: 46 - Streptococcus pneumoniae 23F wchF* 
MKKSVYIIGSKGIPAKYGGFETFVEKLTAFQQDKAIQYYVACMR
ENSAKSGTTEDVFEHNGAICYNVDVPNFGPARAIAYDIAAINRAIEIAKENKDEDPIF
YILACRIGPFIHGIKKKIQEIGGTLLVNPDGHEWLRAKWSAPVRRYWKISEGLMVKHA
DLLVCDSKNIEKYIQEDYKQYQPKTTYIAYGTDTTRSVLKSSDEKVRSWFKEKNVSEN
EYYLVVGRFVPENNYESMIRGFLASNSKKDFVLITNVEQNKFYNQLLAKTGFDKDPRV
KFVGTVYEQELLKYIRENAFAYFHGHEVGGTNPSLLEALASTKLNLLLDVGFNREVAE
DGAIYWKKDNLHEIIETSEQKTQKEIDEKDILSIKQVTERFSWELIVNEYEKLFLCEK
*SEQ ID NO: 47 - Streptococcus pneumoniae 23F wzy* 
MTIKINNLFFVCLSFFGIVLSSSQVIVNLGLSSIIQYISYFMLM
LCVFLTLIKNTLNVFANRIIYFLIISFLFIIGINLQNLPLSRKIYLSFSMLIISSLST
LPIKLINNLSDLRRISYYLLHSIFLSVFLGLVFKISLVTVAVEGIGFSYGFNGGLTHK
NFYAITILVSYILLYVSRKYDAKHQIDSFVLWLDLFLLLISNTRTVYIILVVFWIIIN
RNFINNIKKEHRLVVTATTIVISLLALTFFFKHIINNSESYSHRVLGVVNFFKYYESD
RFHLFFGDAELAFGNTTKGYGHNIRSVLGWDGTVEMPLLSVMIKNGYVGLVGYIIVLF
KFISSIISVKNSTKKNIGLSIFIPLLLSATVENYIVNISFVFMPVCFCILCSIKNIKL
VNNRK
*SEQ ID NO: 48 - Streptococcus pneumoniae 23F wchV* 
MEKLVSIILPVYNVEQYIKNCLESIQQQTYSNLEVIIVNDGSTD
KSVEYCEQICKIDSRFSITHKENGGLSDARNVGIDKSKGDYLIFVDSDDFVSQDMVSY
LVSCMENNEADIAICDPVHYYSDRQNNDLNIFSPASNVKVYETTEALCEMFYQKSFLV
SAWAKIFKRELFDDIRFPVGKLFEDSAIMYLLFEKCETIAYSDAELYAYVHRDNSITT
KKFSDRDLDILEITNTIINHYGDNLRVYTAAVSYKVSACFRILLNSPSGEKYKKVQKE
CLSYILQNWRNILFNNNVRLKNKLALISITIFNPFVKFIYSKVNRWE
*SEQ ID NO: 49 - Streptococcus pneumoniae 23F wchW* 
MNKYEERYQENLSKNDFYKLINKSYLSDKELQVQQVKAGIVLPP
KAFETKLSNKLGLQKSLHGKGGVVDSNGNYIELSAQKAVGMRNRVYGPYKINYDNLPI
RNEKVIYLNYFIKQWGHFLLDVVGRLWYPLLQDNDTKLVYTCYAGTETKIEGNYLEFL
KLLGIDQSRLIMINCPTQFSEVIIPESSILPGGYYTKEYKQLFSSVVENIKLDKYDVN
AKMIYCSRSKLGIAKSKEFGEDGIEGIFKQNGYTSVYMETMSLEEQIKTLLSAKTIVL
TSGSLAHNLLFVNKDIDVFILNKTYRVNLHQFLINEISDATVRFVDIYRSPLPILYGY
 
GPFLMDLTKPLANFLDDNEFVYEKGTVLSKKDYFKYYLKWLWSYRFFLFRLNGIKEGN
SEFEKSFKIIRRYYKTGR
*SEQ ID NO: 50 - Streptococcus pneumoniae 23F wzx* 
MSKYKELAKNTGIFALANFSSKILIFLLVPIYTRVLTTTEYGFY
DLVYTTIQLFVPILTLNISEAVMRFLMKDGVSKKSVFSIAVLDIFIGSIAFALLLLVN
NLFSLSDLISQYSIYIFVIFVFYTLNNFLIQFSKGIDKIGVTAISGVISTAVMLAMNV
ILLVVFDWGLLGFFIANVCGYVIPCIYIVSRLRLWELFEIKIDKKLQWEMVYYALPLV
LNILSWWVNNTSDRYIVTAIVGIQASAIISVAYKIPQILSTISAIFIQSWQISAIKIQ
EDKSDTTFVSNMLLYYNALLLIIASGIILFVKPISNILFGISFYSAWELVPFLIISSL
FNAISGCIGAIMGAKMDTHNIAKSALVGMIANIILNIVLTFLMGPQGITISTLIASFL
IFYMRKDSVKEINSETYRAIYLSWILLVVEACLLIYMDFIIGALIAMVINLFLLKDVI
KPLYLKIFKRN
*SEQ ID NO: 51 - Streptococcus pneumoniae 23F wchX* 
MIVLQYFKILARFVFMFLISAVLLPFKIKPNKIVFINFNGKGYG
DNPKSICEYLRTTYPDLDLVWLARDNEGFPDGVRVVKYGTFQAFYEQASSKVWVYNVR
AFARILKKRGQIYIQTWHGASSFKLIEKQADLPINYVLEAKYDARVTDIMISDSRKQT
EEFQKYFWYSGEIFEVGMPRNDALFHYKEDYDKLNNIRKELSIHSDDYVILYAPTFRD
DGDASYLDINFERLLQCVEHGIKKKCKFLIRLHPNHSHLCNNISFNKNIINATFYSDM
QELTLLADVLVTDYSSSIFDFMLLNKPYVRYVNDLEKYAELRGVSDTYYELPDSIIKT
AEELYDLLPKKIENFDYDSIKKYRNEILCPIFNGTASENVGRRIIQEL
*SEQ ID NO: 52 - Streptococcus pneumoniae 23F gtp1* 
MKNNDLKIGSGAIHQISATLSQNSISGKILYCADPVVDDLYGSI
VRSQIEEIGRVKEESCNYNTIAYAMNIAERAIATDIDCIVGMGGGRVLDVCKYASFIS
KRPYLSIPTTAANDGIASPVAVLKRQDDRPKSLGAAIPSMTLIDIDVIASGPIQNIKA
GIGDTISNYTALKDWELAVERGKDEMHGFAYLMSQNSLDALMKTKYNSITPDFIEVLV
NSLVLSGIAMDFAGSSRPVSGSEHLFSHALDYYGSTRNLHGIQVALGTVAVLKLIENS
VDTVVDYLQRFEVHINPKLLGIDEELFIYCMQHATKMRSNRYTYLHEVDLSTDRLKQI
YKELISEL
*SEQ ID NO: 53 - Streptococcus pneumoniae 23F gtp2* 
MKALILAAGLGTRLAPITNEVPKSLVPVNGKPILMKQIENLYQN
NITDITIIAGYKSSVLTDAVTEKYPEINIIDNVDFKTTNNMYSAYLGKAAMGDSDFLM
MNADVFYDASVIKSLLLHKAPNAIVTDLGIYIEESMKVVEKNGRLVEISKQISPEETL
GASIDVYKFSYEAGARFFEKCKEFIEDKRELQMWSEVALNAILSEVEFVACPLEGRWL
EIDNHEDLVAAEKLFA
*SEQ ID NO: 54 - Streptococcus pneumoniae 23F gtp3* 
MKLTNRVDYFGADISELQNKKLFLFDMDGTIYEEDRLFEGTLEL
LDYIHNIGGEYIFITNNSSKSVVDYVEKVNRLGIKAERDNFFTSAQATIVYIKENYPK
SKVYCQGTKSLIKELSDAGIDVTEQVSADIDVVLVGFDTELTSDKIRNTCEILSTKDV
PFIATNPDIRCPVSFGFIPDCGSICDMISKSVDRKPVYIGKPEPTMVDIVRKKLNYSL
FETVVIGDRLYTDIMTGINAGVTSVCVLTGEATVNDIQQDSIKPTYTFKNVKEMWKGI
V
*SEQ ID NO: 55 - Streptococcus pneumoniae 23F rmlA* 
MKGIILAGGSGTRLYPLTRAASKQLMPVYDKPMIYYPLSTLMLA
GIRDILIISTPQDLPRFKELLQDGSEFGIKLSYAEQPSPDGLAQAFIIGEEFIGDDSV
ALILGDNIYHGPGLSTMLQKAAKKEKGATVFGYHVKDPERFGVVEFDENMNAISIEEK
PEYPRSNYAVTGLYFYDNDVVEIAKSIKPSPRGELEITDVNKAYLDRGDLSVELMGRG
FAWLDTGTHESLLEASQYIETVQRMQNVQVANLEEIAYRRGYISREDVLALAQSLKKN
EYGQYLLRLIGEA
*SEQ ID NO: 56 - Streptococcus pneumoniae 23F rmlC* 
MTDNFFGKTLAARKVEAIPGMLEFDIPVHGDNRGWFKENFQKEK
MLPLGFPESFFAEGKLQNNVSFSRKNVLRGLHAEPWDKYISVADGGKVLGSWVDLREG
ETFGNTYQTVIDASKGIFVPRGVANGFQVLSDTVSYSYLVNDYWALELKPKYAFVNYA
DPSLGIEWENIAEAEVSEADKNHPLLKDVKPLKKEDL
*SEQ ID NO: 57 - Streptococcus pneumoniae 23F rmlB* 
MTEYKNIIVTGGAGFIGSNFVHYVYENFPDVHVTVLDKLTYAGN
RANIEEILGNRVELVVGDIADAELVDKLAAQADAIVHYAAESHNDNSLNDPSPFIHTN
FIGTYTLLEAARKYDIRFHHVSTDEVYGDLPLREDLPGHGEGPGEKFTAETKYNPSSP
YSSTKAASDLIVKAWVRSFGVKATISNCSNNYGPYQHIEKFIPRQITNILSGIKPKLY
GEGKNVRDWIHTNDHSSGVWTILTKGQIGETYLIGADGEKNNKEVLELILKEMGQAVD
AYDHVTDRAGHDLRYAIDASKLRDELGWKPEFTNFEAGLKATIKWYTDNQEWWKAEKE
AVEANYAKTQEIITV
*SEQ ID NO: 58 - Streptococcus pneumoniae 23F rmlD* 
MILITGANGQLGTELRYLLDERNEEYVAVDVAEMDITDAEMVEK
VFEEVKPTLVYHCAAYTAVDAAEDEGRELDFAINVTGTKNVAKASEKHGATLVYISTD
YVFDGKKPVGQEWEVDDRPDPQTEYGRTKRMGEELVEKHVSNFYIIRTAWVFGNYGKN
FVFTMQNLAKTHKTLTVVNDQYGRPTWTRTLAEFMTYLAENRKEFGYYHLSNDATEDT
 
TWYDFAVEILKGTDVEVKPVDSSQFPAKAKRPLNSTMSLAKAKATGFVIPTWQDALQE
FYKQEVR
*SEQ ID NO: 59 E. coli O2 WegR* 
MEENNMKTVAVVGTVGVPACYGGFESLVQNLIDYQSDGIQYQIF
CSSKKYDKKFKNYKNAELIYLPINANGVSSIIYDIMCLIICLFKRPDVVLILGVSGCL
FLPIYKLFSKSKIIVNIDGLEWRRNKWGTFAKKFLKISEAISIRIADIIISDNQAIAD
YVENKYKKKSVVIAYGGDHATNLSTPIDNDQKKEGYYLGLCRIEPENNIEMILNAFIN
TDKKIKFMGNWDNSEYGRQLKKYYSNYPNITLLEPNYNIEELYKLRKNCLAYIHGHSA
GGTNPSLVEAMHFNIPIFAFDCDFNRYTTNNLAHYFNDSEQLSLLAESLSFGNLKCRV
LDLKNYAEDMYNWRHIAAMYESIY
*SEQ ID NO: 60 E. coli 0149 WbuV* 
MTEQFSEKKIDVVGIVGLPACYGGFESLVQNLVDYQSQNIKYNV
YCSRKKYKNTPKKYKRADLKYIPFDANGSSSILYDIYSLFLSLFNKVDVVLILGVSGC
VFLPIYRFFSSSKVIVNIDGLEWKRAKWKGIAKWYLKISEKIAVKYSDVVVADNEAIA
KYVLKKYGLEAKIIAYGGDHSLVKKPISVIKEDYFFTVCRIEPENNIRMILEAFKNTT
HSLKIVGNWDSSLYGRRLKEEFGNYNNIEIIDPIYDSDILFNFRSLCRGYIHGHSAGG
TNPSLVEAMHFQIPIIAFDCDFNRFTTDNYAFYFKNKNELSFIVNDILNGNQNEQAEI
CAKKMKEIATKKYTWDTIAKMYEELY
*SEQ ID NO: 61 Cronobacter sakazakii WepI* 
MKRIAVVGTVGIPACYGGFESLVENLTKYKGAGYQYYIFCSSKN
YPEKSDSHNDAQLIYVPLKANGIQSILYDIVSLWKCLFLKVDTILILGVSGCIFLPVF
RLLSNAKIITNIDGLEWKREKWNYPIKKFLKFSELLAVKYSHAIVTDNRAITDYVKKE
YNVSSFTIAYGGDHAVRPSNNNNNIKSSYALGLCRIEPENNVELILKAFTLSEDKLKF
VGNWNASSYGRMLKKNYSNYSNIELIEPIYDIDKLYILRSGCDKYIHGHSAGGTNPSL
VEMMHFGVPIFAFDCEFNRHSTDNKAFYFKDAQHLADLVKMKDNTELEKNSCNMKVLA
QENYTWQKITASYESLY
*SEQ ID NO: 62 pglB from Campylobacter jejuni* 
IISNDGYAFAEGARDMIAGFHQPNDLSYYGSSLSTLTYWLYKIT
PFSFESIILYMSTFLSSLVVIPIILLANEYKRPLMGFVAALLASIANSYYNRTMSGYY
DTDMLVIVLPMFILFFMVRMILKKDFFSLIALPLFIGIYLWWYPSSYTLNVALIGLFL
IYTLIFHRKEKIFYIAVILSSLTLSNIAWFYQSTIIVILFALFALEQKRLNFVIIGIL
ASVTLIFLILSGGVDPILYQLKFYIFRSDESANLTQGFMYFNVNQTIQEVENVDLSEF
MRRISGSEIVFLFSLFGFVWLLRKHKSMIMALPILVLGFLALKGGLRFTIYSVPVMAL
GFGFLLSEFKAILVKKYSQLTSNVCIVFATILTLAPVFIHIYNYKAPTVFSQNEASLL
NQLKNIANREDYVVTWWDYGYPVRYYSDVKTLVDGGKHLGKDNFFPSFALSKDEQAAA
NMARLSVEYTEKSFYAPQNDILKTDILQAMMKDYNQSNVDLFLASLSKPDFKIDTPKT
 
RDIYLYMPARMSLIFSTVASFSFINLDTGVLDKPFTFSTAYPLDVKNGEIYLSNGVVL
SDDFRSFKIGDNVVSVNSIVEINSIKQGEYKITPIDDKAQFYIFYLKDSAIPYAQFIL
MDKTMFNSAYVQMFFLGNYDKNLFDLVINSRDAKVFKLKI
*SEQ ID NO: 63 E. coli wecA* 
MMVFGKLYLS SLGYIFGSWE MVLGPFGYFL TLFAVWAAIN AFNMVDGIDG LLGGLSCVSF
AAIGMILWFD GQTSLAIWCF AMIAAILPYI MLNLGILGRR YKVFMGDAGS TLIGFTVIWI
LLETTQGKTH PISPVTALWI IAIPLMDMVA IMYRRLRKGM SPFSPDRQHI HHLIMRAGFT
SRQAFVLITL AAALLASIGV LAEYSHFVPE WVMLVLFLLA FFLYGYCIKR AWKVARFIKR
VKRRLRRNRG GSPNLTK
*SEQ ID NO: 64 E. coli RcsA* 
MSTIIMDLCS YTRLGLTGYL LSRGVKKREI NDIETVDDLA IACDSQRPSV VFINEDCFIH
DASNSQRIKL IINQHPNTLF IVFMAIANVH FDEYLLVRKN LLISSKSIKP ESLDDILGDI
LKKETTITSF LNMPTLSLSR TESSMLRMWM AGQGTIQISD QMNIKAKTVS SHKGNIKRKI
KTHNKQVIYH VVRLTDNVTN GIFVNMR
*SEQ ID NO: 65 pglB from Campylobacter jejuni* 
MLKKEYLKNPYLVLFAMIILAYVFSVFCRFYWVWWASEFNEYFFNNQLMIISNDGYAFAE
GARDMIAGFHQPNDLSYYGSSLSALTYWLYKITPFSFESIILYMSTFLSSLVVIPTILLA
NEYKRPLMGFAAALLASIANSYYNRTMSGYYDTDMLVIVLPMFILFFMVRMILKKDFFSL
IALPLFIGIYLWWYPSSYTLNVALIGLFLIYTLIFHRKEKIFYIAVILSSLTLSNIAWFY
QSAIIVILFALFALEQKRLNFMIIGILGSATLIFLILSGGVDPILYQLKFYIFRSDESAN
LTQGFMYFNVNQTIQEVENVDLSEFMRRISGSEIVFLFSLFGFVWLLRKHKSMIMALPIL
VLGFLALKGGLRFTIYSVPVMALGFGFLLSEFKAIMVKKYSQLTSNVCIVFATILTLAPV
FIHIYNYKAPTVFSQNEASLLNQLKNIANREDYVVTWWDYGYPVRYYSDVKTLVDGGKHL
GKDNFFPSFALSKDEQAAANMARLSVEYTEKSFYAPQNDILKTDILQAMMKDYNQSNVDL
FLASLSKPDFKIDTPKTRDIYLYMPARMSLIFSTVASFSFINLDTGVLDKPFTFSTAYPL
DVKNGEIYLSNGVVLSDDFRSFKIGDNVVSVNSIVEINSIKQGEYKITPIDDKAQFYIFY
LKDSAIPYAQFILMDKTMFNSAYVQMFFLGNYDKNLFDLVINSRDAKVFKLKI
*SEQ ID NO: 66 fepE from Salmonella typhimurium*

MPSLNVKQEKNQSFAGYSLPPANSHEIDLFSLIEVLWQAKRRILATVFAFACVGLLLSFLLPQKWTSQAIVTP
AESVQWQGLERTLTALRVLDMEVSVDRGSVFNLFIKKFSSPSLLEEYLRSSPYVMDQLKGAQIDEQDLHRAIV
LLSEKMKAVDSNVGKKNETSLFTSWTLSFTAPTREEAQKVLAGYIQYISDIVVKETLENIRNQLEIKTRYEQE
KLAMDRVRLKNQLDANIQRLHYSLEIANAAGIKRPVYSNGQAVKDDPDFSISLGADGISRKLEIEKGVTDVAE
IDGDLRNRQYHVEQLAAMNVSDVKFTPFKYQLSPSLPVKKDGPGKAIIIILAALIGGMMACGGVLLRHAMVSR
KMENALAIDERLV

### Examples

### Example 1:

### Genetic analysis and predicted structures for serotype 23B capsular polysaccharide repeating units

The presence of the same glycosyltransferase (GT) genes (wchA, wchF, wchV, wchW and wchX) that are present in the *cps* locus of serotype 23F, indicates the possibility that serotype 23A and 23B contain the same monosaccharide composition as serotype 23F (FIG. 1).

In the *cps* cluster of serotype 23B, the highest sequence diversity among GT genes are observed for WchA and WchW. The function of WchA is biochemically demonstrated as undecaprenyl-phosphate glucosyl-1-phosphate transferase [Pelosi, L.; Boumedienne, M.; Saksouk, N.; Geiselmann, J.; Geremia, R.A. Biochem. Biophys. Res. Commun. 2005, 327(3), 857-865] which is a highly conserved function irrelevant to sequence diversity within *S. pneumoniae* [Bentley, S.D.; Aanensen, D.M.; Mavroidi, A.; Saunders, D.; Rabbinowitsch, E.; Collins, M.; Donohoe, K.; Harris, D.; Murphy, L.; Quail, M.A.; Samuel, G. PLoS Genet. 2006, 2(3), e31]. WchW is a putative α-1,2-L-rhamnosyltransferase [Morona, J.K.; Miller, D.C.; Coffey, T.J.; Vindurampulle, C.J.; Spratt, B.G.; Morona, R.; Paton, J.C. Microbiology. 1999, 145(4), 781-789]. A search for WchW homologues revealed no hits outside of serogroup 23 *cps* loci within *S. pneumoniae*; however a homologue to WchW was found in the *cps* locus of *Klebsiella pneumoniae* serotype K56, designated as WcpL (accession CZQ24634 [Follador, R.; Heinz, E.; Wyres, K.L.; Ellington, M.J.; Kowarik, M.; Holt, K.E.; Thomson, N.R. Microb. Genom. 2016, 2(8*)*]). The *K. pneumoniae* K56 CPS repeating unit also contains a rhamnose which is α-1,2-linked to galactose [Choy, Y.M.; Dutton, G.G. Can. J. Biochem. 1973, 51(18), 3021-3026]. The fact that WchW of 23B has the lowest sequence identity to WchW of 23A/F suggests that there may be a different bond between the L-rhamnose and the D-galactose, possibly explaining the serotyping differences. Due to the homology of the 23B and 23F repeat unit polymerase (Wzy), the polymerization is most likely to be similar for both serotypes.

### NMR assignments for serotype 23F capsular polysaccharide repeating unit

The ¹H NMR spectrum (FIG. 2A) shows the expected signals for the 23F tetrasaccharide RU: four H-1, ring signals (including sharp peaks from glycerol) and two methyl signals from α- and β-Rha, together with small signals from residual CWPS (Genetic and structural elucidation of capsular polysaccharides from Streptococcus pneumoniae serotype 23A and 23B, and comparison to serotype 23F.,"/pubmed/28837839","Ravenscroft N, Omar A, Hlozek J, Edmonds-Smith C, Follador R, Serventi F, Lipowsky G, Kuttel MM, Cescutti P, Faridmoayer A.","Carbohydr Res. 2017 Oct 10).

The diagnostic anomeric and methyl proton signals were used as starting points for the ¹H-¹H correlation experiments (COSY and TOCSY) which elucidated H-1 to H-6 for β-Glc, α- and β-Rha and H-1 to H-4 for β-Gal. H-5 of β-Gal was assigned from the H-1/H-5 crosspeak in the NOESY experiment and H-6 from the H-4/C-6 crosspeak in the HSQC-NOESY experiment. All of the HSQC crosspeaks (FIG. 3) could be assigned from the proton assignments already established aided by overlays with 1D TOCSY (200 ms), HSQC-TOCSY, HSQC-NOESY and HMBC experiments. The ¹H and ¹³C NMR data are collected in Table 1. The deshielded carbons and glycosylation shifts compared to the corresponding monosaccharide [Jansson, P.E.; Kenne, L.; Widmalm, G. Carbohydr. Res. 1989, 188, 169-191] confirmed the linkage positions: C-2 (+2.73 ppm), C-3 (+4.45 ppm) and C-4 (+4.65 ppm) of β-Gal, C-4 (+4.96 ppm) of β-Rha and C-4 (+6.90 ppm) of β-Glc. The relatively small glycosylation shift for C-2 of Gal has been observed for other 2,3-β-Gal residues in serotypes 15B and 33F and was attributed to the strong steric hindrance imposed by vicinal 2,3-disubstitution [Jansson, P.E.; Kenne, L.; Wehler, T. Carbohydr. Res. 1988, 179, 359-368]. The sequence of sugar residues indicated by glycosylation shifts followed from the HMBC interresidue correlations (FIG. 9A) and transglycosidic correlations in the NOESY experiment. The ¹H-³¹P HMBC experiment showed major crosspeaks from the phosphodiester signal at -0.09 ppm to H-3 of β-Gal at 4.33 ppm and H-2 (and H-1/H-3) of Gro confirming the presence of the Gro-(2→P→3)-β-D-Gal*p*-linkage. An expansion of the fully assigned ¹³C NMR spectrum is shown in FIG. 4A; the splitting of C-2 of glycerol (6 Hz) is from ³¹P coupling. Lastly the proton-coupled ¹³C spectrum gave J_{H1,C1} for the anomeric carbons confirming the β- configuration of the terminal Rha (174 Hz) and β- for the remaining residues (162-168 Hz). Thus NMR analysis confirmed the structure of the tetrasaccharide repeating unit of serotype 23F polysaccharide as →4)-β-D-Glc*p*-(1→4)-[α-L-Rha*p*-(1→2)]-[Gro-(2→P→3)]-β-D-Gal*p*-(1→4)-β-L-Rhap-(1→.

**Table 1**

| ¹H and ¹³C NMR chemical shifts (δ, ppm) for the serotype 23F polysaccharide repeating unit | | | | | | |
|---|---|---|---|---|---|---|
| **Residue** | **H-1** | **H-2** | **H-3** | **H-4** | **H-5** | **H-6** |
| | **C-1** | **C-2** | **C-3** | **C-4** | **C-5** | **C-6** |
| α-L-Rha*p*-(1→ **α-R** | 5.10 | 4.15 | 3.82 | 3.47 | 4.10 | 1.27 |
| | 101.66 | 70.30 | 70.51 | 72.37 | 69.20 | 16.94 |
| →2,3,4)-β-D-Gal*p*-(1→ **GA** | 4.95 | 3.82 | 4.33 | 4.42 | 3.81 | 3.94 |
| | 101.22 | 75.69 | 78.23 | 74.34 | 74.45 | 61.08 |
| → 4)-β-L-Rhap-(1→ **β**-**R** | 4.86 | 4.04 | 3.80 | 3.70 | 3.44 | 1.36 |
| | 101.05 | 71.55 | 73.91 | 77.79 | 71.41 | 17.59 |
| →4)-β-D-Glc*p*-(1→ **G** | 4.83 | 3.36 | 3.68 | 3.64 | 3.53 | 3.94, 3.83 |
| | 102.76 | 73.81 | 75.88 | 77.61 | 74.78 | 61.42 |

| | | | | | | |
|---|---|---|---|---|---|---|
| Phosphoglycerol at C-3 of Gal: ¹H, ¹³C and ³¹P assignments; δ CH: (4.29, 77.49); δ CH₂: (3.77, 62.03) and ³¹P at -0.09 ppm. | | | | | | |

### Structure of serotype 23B capsular polysaccharide repeating unit

Composition analysis of the 23B polysaccharide gave similar results to those obtained for 23F and 23A. GC-MS analysis of the TMS methyl glycosides (FIG. 10B) showed the presence of Rha, Gal and Glc (relative peak areas of 0.22 : 0.53 : 1.00) together with trace amounts of glycerol. The ¹H NMR spectrum (FIG. 2C) shows signals for the 23B trisaccharide RU: three H-1, ring signals (including sharp signals from glycerol) and one methyl signal from β-Rha, together with signals from residual CWPS. The 1D spectrum indicated that 23B has the same structure as 23F, but without the terminal α-Rha. This was confirmed by the full set of ¹H, ¹³C and ³¹P 1D and 2D NMR experiments performed. All of the HSQC crosspeaks (FIG. 5) could be assigned from the proton assignments aided by appropriate overlays with hybrid and HMBC experiments. The ¹H and ¹³C NMR data are collected in Table 2. The deshielded carbons and glycosylation shifts established the linkage positions: C-3 (+4.15 ppm) and C-4 (+4.38 ppm) of β-Gal, C-4 (+8.96 ppm) of β-Rha and C-4 (+7.10 ppm) of β-Glc. The similarity of the glycosylation shifts to those obtained for 23F provide proof that the bonds have the same configuration factors i.e., that the hexoses have the D absolute configuration and Rha the L absolute configuration. The sequence of sugar residues indicated by glycosylation shifts followed from the HMBC interresidue correlations (FIG. 9C) and transglycosidic correlations in the NOESY experiment. The ¹H-³¹P HMBC experiment showed major crosspeaks from the phosphodiester signal at -0.26 ppm to H-3 of β-Gal at 4.26 ppm and H-2 (and H-1/H-3) of Gro confirming the presence of the Gro-(2→P→3)-β-D-Gal*p*-linkage. An expansion of the fully assigned ¹³C NMR spectrum is shown in FIG. 4C; the splitting of C-2 of glycerol (5.8 Hz) is from ³¹P coupling. Lastly the proton-coupled ¹³C spectrum gave J_{H1,C1} for the anomeric carbons confirming the β-configuration for all the residues (162-165 Hz). Thus NMR analysis established the tetrasaccharide repeating unit of serotype 23B polysaccharide as →4)-β-D-Glc*p*-(1→4)-[Gro-(2→P→3)]-β-D-Gal*p*-(1→4)-β-L-Rhap-(1→.

**Table 2**

| ¹H and ¹³C NMR chemical shifts (δ, ppm) for the serotype 23B polysaccharide repeating unit | | | | | | |
|---|---|---|---|---|---|---|
| **Residue** | **H-1** | **H-2** | **H-3** | **H-4** | **H-5** | **H-6** |
| | **C-1** | **C-2** | **C-3** | **C-4** | **C-5** | **C-6** |
| →3,4)-β-D-Gal*p*-(1→ **GA** | 4.77 | 3.80 | 4.26 | 4.39 | 3.73 | 3.82, 3.76 |
| | 104.10 | 71.34 | 77.93 | 74.07 | 74.77 | 60.92 |
| → 4)-β-L-Rhap-(1→ **β-R** | 4.86 | 4.09 | 3.82 | 3.63 | 3.49 | 1.37 |
| | 101.02 | 71.02 | 73.27 | 81.79 | 71.12 | 17.35 |
| →4)-β-D-Glc*p*-(1→ **G** | 4.81 | 3.34 | 3.68 | 3.64 | 3.53 | 3.94,3.82 |
| | 102.76 | 73.87 | 75.91 | 77.69 | 74.79 | 61.43 |

| | | | | | | |
|---|---|---|---|---|---|---|
| Phosphoglycerol at C-3 of Gal: ¹H, ¹³C and ³¹P assignments; δ CH: (4.29, 77.69); δ CH₂: (3.76, 61.9); ³¹P at -0.26ppm. | | | | | | |

### Molecular models

Molecular models of 10RU of the three polysaccharides were built with CarbBuilder [Kuttel, M.M.; Ståhle, J.; Widmalm, G. J. Comput. Chem. 2016, 37(22), 2098-2105] and subsequently minimized. The models for 23F and 23B show a very similar loose helical conformation (FIG. 6). However, the immunodominant terminal α-Rha in 23F (absent in 23B) is clearly exposed on the edge of the helix (purple residues in FIG. 6), and would present a markedly different surface for antibody binding. In contrast to the conformations of 23F and 23B, the model for 23A is a slightly twisted ribbon, with clear steric crowding at the β-L-Rha branch point: the β-Glc is in close proximity to β-Gal (< 3 Å). This model thus explains the strong deshielding of H-1 of 2,3-β-Gal observed in the NMR spectrum of polysaccharide 23A. Further, the presentation of the terminal α-Rha in 23A is quite different to 23F: the α-Rha forms a long, almost straight line along the chain in 23A, as opposed to its orientation in the 23F helix. These very different conformations depicted in these preliminary models suggest little likelihood of cross-protection between either 23F or 23B with 23A.

### 3. Conclusions

Structural predictions of the 23B polysaccharide based on the genetic analyses is in agreement with the experimentally-obtained structures. The biological repeat units of the polysaccharide can be identified with confidence, and the glycosyltransferases responsible for each elongation step can be assigned by comparison with the 23F *cps* locus (FIG. 7).

WchW activity in 23B is not only different to its 23F counterpart as predicted, but it is even absent. The lack of the α-1-2-linked-rhamnose constitutes the only difference between 23F and 23B capsular polysaccharides. The reasons for WchW's missing activity are not clearly deducible from a comparison of the known homologues; it could be due to either several inactivating mutations in the protein, comparing to 23F, or a halt at the transcriptional level. As Geno at al. [Geno, K.A.; Saad, J.S.; Nahm, M.H. J. Clin. Microbiol. 2017, JCM-00054] have recently shown, a subtle difference of only two amino acid substitutions can be responsible for the inactivation of the acetyltransferase in an isolate of serotype 35B, resulting in a novel, non-acetylated serotype.

Molecular modelling shows similar helical structures for 23F and 23B, but a markedly different sterically-crowded ribbon-like structure for 23A. The repeating unit structures for 23A and 23B may explain why the typing antiserum prepared in rabbits with type 23F bacteria reacts only slightly with serotype 23A and hardly at all with serotype 23B [Robbins, J.B.; Austrian. R.; Lee, C.J.; Rastogi, S.C.; Schiffman, G.; Henrichsen, J.; Mäkelä, P.H.; Broome, C.V.; Facklam, R.R; Tiesjema, R.H.; Parke, J.C. J. Infect. Dis. 1983, 148(6), 1136-1159]. In 23A, the immunodominant terminal α-Rha [Park, S.; Nahm, M .H. PLoS One. 2013, 8(12), e83810] is no longer a pendant group at C-2 of the main backbone 2,3,4-Gal as in 23F, but on C-2 of the sterically constrained 2,3-Gal, now present as a side chain (FIG. 6). This means that the terminal α-Rha of 23A will be less accessible to 23F antibody directed against this dominant epitope. The terminal α-Rha is absent in 23B, which means little or no cross reaction with 23F antisera as reported.

### Experimental

Purified pneumococcal polysaccharide serotypes 23B were purchased from Statens Serum Institut (SSI). The comparator polysaccharide 23F were obtained from GSK.

### Genetic analysis of serogroup 23 cps locus sequence

The published cps locus sequences (serotype 23a: accession CR931683; 23b: CR931684; 23f: CR931685) and Wzy sequences [Bentley, S.D.; Aanensen, D.M.; Mavroidi, A.; Saunders, D.; Rabbinowitsch, E.; Collins, M.; Donohoe, K.; Harris, D.; Murphy, L.; Quail, M.A.; Samuel, G. PLoS Genet. 2006, 2(3), e31.] have been downloaded from GenBank (WorldWideWeb(www).ncbi.nlm.nih.gov/nuccore). Pairwise protein sequence identity has been assessed using BLASTp [Camacho, C.; Coulouris, G.; Avagyan, V.; Ma, N.; Papadopoulos, J.; Bealer, K.; Madden, T.L. BMC Bioinf. 2009, 10(1), 421]. Multiple sequence alignments have been performed using T-Coffee and standard parameters (v11.00) [Notredame, C.; Higgins, D.G.; Heringa, J. J. Mol. Biol. 2000, 302(1), 205-217]. Wzy phylogeny was inferred from multiple sequence alignments by running RAxML using a gamma distribution to model site-specific rate variation and 100 bootstrap replicates [Stamatakis, A. Bioinformatics. 2006, 22(21), 2688-2690].

### Monosaccharide composition analysis by GC-MS

Methanolysis (3 M HCl) of polysaccharide 23F, 23A and 23B samples (0.5 - 1 mg) was performed in a CEM Discover SP-d Microwave reactor at 120 W and 121 °C for 5 minutes and the tri-methyl silyl ethers (TMS) derivatives prepared as described by Kim *et al.* [Kim, J.S.; Laskowich, E.R.; Arumugham, R.G.; Kaiser, R.E.; MacMichael, G.J. Anal. Biochem. 2005, 347(2), 262-274]. GC-MS analysis was performed on an Agilent 8720A Gas Chromatograph equipped with a Agilent 5975 mass spectrometer and a DB-1MS column (30 m); temperature program: 50 °C for 2 min, 50 - 150 °C at 30 °C/min, 150 - 220 °C at 3 °C/min, 220 - 300 °C at 30 °C/min and 300 °C for 10 min. The inlet temperature was set at 250 °C and the MS transfer line at 300 °C. The MS acquisition parameters were set to scan at m/z 50-550 in electron impact (EI) mode. GC-MS data was processed using Agilent Chemstation software. A mixture of standard monosaccharides was used to determine the retention times and corresponding mass spectra for each sugar derivative.

### NMR spectroscopy

Polysaccharide samples (~10 mg) were lyophilized and exchanged twice with 99.9 % deuterium oxide (Sigma Aldrich), then dissolved in 600 µL of D₂O and introduced into a 5 mm NMR tube for data acquisition. Preliminary NMR studies yielded broad lines and poor 2D crosspeaks for polysaccharide 23A and 23F, the spectral resolution was improved by placing the NMR sample in a Branson 1200 Sonicator water bath for 1-2 days. 1D ¹H, ¹³C and ³¹P and 2D, COSY, TOCSY, NOESY, HSQC, HMBC and hybrid H2BC, HSQC-TOCSY and HSQC-NOESY NMR spectra were obtained using a Bruker Advance III 600 MHz NMR spectrometer equipped with a BBO Prodigy cryoprobe and processed using standard Bruker software (Topspin 3.2). The probe temperature was set at 313 or 323 K. 2D TOCSY experiments were performed using mixing times of 120 or 180 ms and the 1D variants using mixing times up to 200 ms. The HSQC experiment was optimized for J = 145 Hz (for directly attached ¹H-¹³C correlations), and the HMBC experiment optimized for a coupling constant of 6 Hz (for long-range ¹H-¹³C correlations). HSQC-TOCSY and HSQC-NOESY NMR spectra were recorded using mixing times of 120 and 250 ms respectively. Polysaccharide spectra were referenced to residual cell wall polysaccharide signals (phosphocholine ¹H signal at 3.23 ppm and ¹³C signal at 54.5 ppm and the shielded ³¹P signal at 1.30 ppm) [Vialle, S.; Sepulcri, P.; Dubayle, J.; Talaga, P. Carbohydr. Res. 2005, 340, 91-96].

### Molecular modeling

Optimal dihedral angle conformations for the glycosidic linkages were taken from the corresponding disaccharide potential of mean force free energy surfaces calculated with the metadynamics routine incorporated into NAMD [Laio, A.; Parrinello, M. Proc. Natl. Acad. Sci. U.S.A. 2002, 99, 12562-12565], with the ϕ,ψ glycosidic linkage torsion angles used as collective variables. The optimal conformations are listed in Table 3.

**Table 3**

| Optimal values for the ϕ,ψ glycosidic linkage torsion angles determined from vacuum metadyamics. | |
|---|---|
| **Disaccharide** | ϕ, ψ |
| α-L-Rha*p*-(1→2)-β-D-Gal*p* | 39, 21 |
| β-D-Glc*p*-(1→3)-β-L-Rha*p* | 46, 11; **59** -**13**^{a} |
| β-D-Gal*p*-(1→4)-β-L-Rha*p* | 26, 26 |
| β-D-Glc*p*-(1→4)-β-D-Gal*p* | 44,16 |
| β-L-Rha*p*-(1→4)-β-D-Glc*p* | -51, -8 |

| | |
|---|---|
| ^{a} Value used for 23A, to avoid atomic collisions. This value is still within the vacuum global minimum energy well. | |

Molecular models of 10 repeat units of 23F, 23A and 23B were built with CarbBuilder version 2.1.17 [Kuttel, M.M.; Ståhle, J.; Widmalm, G. J. Comput. Chem. 2016, 37(22), 2098-2105] using the dihedral angles listed in Table 3. We added bond, angle and dihedral parameters to the CHARMM36 additive force field for carbohydrates [Guvench, O.; Hatcher, E.; Venable, R.M.; Pastor, R.W.; MacKerell, Jr A.D. J Chem Theory Comput, 2009, 5(9), 2353-2370; Mallajosyula, S.S.; Guvench ,O.; Hatcher, E.; MacKerell, Jr AD. J Chem, Theory Comput, 2012, 8, 759-776] to represent the 2-phosphate substitution on glycerol, as well as the glycosidic phosphodiester (2->3) linkage. These parameters were adapted from the ribitol phosphodiester parameters previously added to the force field [Kuttel, M.M.; Jackson, G.E.; Mfata, M.; Ravenscroft, N. Carbohydr. Res, 2015, 406, 27-33]. These initial oligosaccharide structures were optimized through 20000 steps of standard NAMD (version 2.9) minimization in vacuum [Phillips, J.C.; Braun, R; Wang,W; Gumbart, J.; Tajkhorshid, E; Villa, E; et al. J Comput Chem 2005, 26, 1781-1802].

The present disclosure is not to be limited in scope by the specific embodiments described herein. Indeed, various modifications of the subject matter provided herein, in addition to those described, will become apparent to those skilled in the art from the foregoing description and accompanying figures. Such modifications are intended to fall within the scope of the appended claims.

Various publications, patents and patent applications are cited herein, the disclosures of which are incorporated by reference in their entireties.

### Example 2:

### Obtainment of 23B bioconjugate using the glycoengineering strategy.

A protein glycosylation experiment was carried out in order to assess whether the engineered Sp23B polysaccharide could be transferred onto EPA.

At this scope, strain stLMTB13743 (W3110; ΔwaaL; wca::23B cluster) was transformed with different plasmid combinations (see below).

5-mL TB-dev 10 mM MgCl₂ + antibiotics precultures were grown o/n at 30°C. The following day, 50-mL TB-dev 10 mM MgCl₂ + antibiotics + 0.1% arabinose main cultures were inoculated with 0.1 OD₆₀₀ from the precultures. At OD₆₀₀=0.9, main cultures have been induced with 0.1 mM IPTG for the induction and grown o/n at 30°C.

From each culture 2 OD600 were harvested, resuspended in 100 uL Lämmli buffer, incubated 10 min @95°C, 2 uL Proteinase K were added and incubated 1h at 55°C, transferred to 70°C for 10 min, vortexed (LLO sample).

From each culture a periplasmic extraction (PPE) was carried out: 100 ODs were harvested and resuspended completely in 1000ul lysis buffer (30mM Tris-HCl pH 8.5,1mM EDTA,20% Sucrose) at 4°C. 1mg/ml of lysozyme was added to the cells (20mg/ml stock solution in ddH₂O; Sigma-Aldrich ref. # L6876-10G), incubated at 4°C on a rotating wheel during 20 minutes in 2ml tubes. The cells were then centrifuged at 16'000 rcf 30 minutes, and the supernatant was transferred to a new tube.

The PPE were enriched exploiting the poly-His tag of EPA: 500ul of PPE were mixed with 125ul of 4mM MgCl₂, 150mM Tris pH8.0, 50mM Imidazole,2.5M NaCl, 150ul of 50% slurry (=75ul resin) of pre-equilibrated IMAC resin (for equlibration the resin was washed 3x with and resuspended in final equal volume of 1x binding buffer: 30mM Tris pH8.0, 10mM Imidazole,500mM NaCl). The samples were incubated 90 min on a rotating wheel at RT; the resin was centrifuged at 8000 rcf 1 min and supernatant was discarded; the Ni-NTA agarose was resuspended in 500ul 1xbinding buffer supplemented with 0.025%n-Dodecyl-B-maltoside (DDM; Glycon ref.#D97002-C; stock solution: 10%) by pipetting and transferred to Corning Costar Spin-X centrifuge tube filters (Sigma-aldrich CLS8163) and centrifuged 1 min at 4000 rcf. Flowthrough was discarded, the resin was then washed 3 more times with 500ul of 1xBinding buffer. The flowthrough was completely removed using vacuum pump, 80ul of elution buffer (30mM Tris pH8.0 , 500mM imidazole, 200mM NaCl) was added to the resin, mixed by vortexing, incubated at RT 2 h . The eluate was recovered. FIG 11. The LLO samples were analyzed via SDS-PAGE (4-12% NuPAGE Gel, MES Running Buffer, 55min at 200V) followed by transfer onto membrane and anti-23B Western Blot (i). The enriched samples were analyzed via SDS PAGE SDS-PAGE (4-12% NuPAGE Gel, MOPS Running Buffer, 55min at 200V) followed by transfer onto membrane and anti-His (ii) or anti-23A (ii) Western Blot. The volumes corresponding to 0.2 OD600 (i), 0.6 OD600 (ii) and (iii) of culture volume were mixed to loading buffer and loaded. Lane 1: Marker PageRuler from Thermo-Fischer Scientific; lane 2-6 stLMTB13743 transformed with pLMTB3130 (pLAFR^{tet}_Sp15Agtp123) and pLMTB6015 (pEXT22^{Kan}_EPA_rcsA_fepE). Lane 2: additional co-transformed plasmids pGVXN72 (empty pEXT21^{sp}), pLMTB4640 (pDISC^{tmp}_engineering cluster (23AwchX_23AwchV_E. coli O2 wegR_23Awzx)). Lane3: additional plasmids pLMTB4640 and pLMTB4623 (pEXT21^{sp}_pglB_homolog ). Lane 4: additional plasmids pLMTB4623 and pGVXN1387 (empt pDISC). Lane 5: additional plasmids pLMTB4640 and pLMTB4620 (pEXT21^{sp}_pglB homolog). Lane 6 additional plasmids pGVYN1387 and pLMTB4620.

23B LLO's are produced in every combination (FIG. 11 (i)). Conjugate is produced in lanes 3 and 5 only. This indicates that both homologs of *C. jejuni* pglB are able to transfer the engineered polysaccharide but not the not-engineered, as combinations with empty pDISC 's don't result in conjugates.

## Claims

1. An immunogenic composition comprising a pneumococcal saccharide comprising one or more repeat unit(s) →4)-β-D-Glcp-(1→4)-[Gro-(2→P→3)]-β-D-Galp-(1→4)-β-L-Rhap-(1→.

2. An immunogenic composition according to claim 1 wherein the pneumococcal saccharide comprises less than 500 repeat units, for example 1 to 200 repeat units (suitably 2 to 200, 1 to 100, 2 to 100, 2 to 50, 2 to 30, 2 to 20 repeat units).

3. An immunogenic composition according to claim 1 wherein size (Mw) of the pneumococcal saccharide is less than 500kDa, for example in the range 1-500kDa, 10-450-kDa, 30-400kDa.

4. An immunogenic composition according to any of claims 1 to 3 wherein the pneumococcal saccharide is conjugated to a carrier protein (e.g. selected from detoxified exotoxin A from *P. aeruginosa* (EPA), TT, DT, CRM197, PhtD, detoxified pneumolysin and protein D, suitably exotoxin A from *P. aeruginosa* (EPA)).

5. The immunogenic composition according to claim 4 wherein the pneumococcal saccharide is directly coupled to the carrier protein (e.g. via an N-linked glycosidic bond or via an O-linked glycosidic bond).

6. The immunogenic composition according to any of claims 4 to 5 wherein the pneumococcal saccharide is linked to an amino acid on the carrier protein selected from asparagine, aspartic acid, glutamic acid, lysine, cysteine, tyrosine, histidine, arginine or tryptophan (e.g. asparagine).

7. The immunogenic composition according to any of claims 4 to 6 wherein the pneumococcal saccharide is covalently linked to the carrier protein through a chemical linkage obtainable using a chemical conjugation method, optionally selected from the group consisting of carbodiimide chemistry, reductive animation, cyanylation chemistry (for example CDAP chemistry), maleimide chemistry, hydrazide chemistry, ester chemistry, and N-hydroysuccinimide chemistry either directly or via a linker.

8. The immunogenic composition according to any of claims 1 to 7 comprising *S. pneumoniae* capsular saccharides selected from *Streptococcus pneumoniae* serotypes 1, 2, 3, 4, 5, 6A, 6B, 7A, 7B, 7C, 8, 9A, 9L, 9N, 9V, 10A, 10B, 10C, 10F, 11A, 11B, 11C, 11D, 11F, 12A, 12B, 12F, 13, 14, 15A, 15B, 15C, 15F, 16A, 16F, 17A, 17F, 18A, 18B, 18C, 18F, 19A, 19B, 19C, 19F, 20, 21, 22A, 22F, 23A, 23B, 23F, 24A, 24B, 24F, 25A, 25F, 26, 27, 28A, 28F, 29, 31, 32A, 32F, 33A, 33B, 33C, 33D, 33F, 34, 35A, 35B, 35C, 35D, 35F, 36, 37, 38, 39, 40, 41A, 41F, 42, 43, 44, 45, 46, 47A, 47F or 48.

9. The immunogenic composition according to any of claims 1 to 8 comprising 10 or more, 11 or more, 12 or more, 13 or more, 14 or more, 15 or more, 16 or more, 17 or more, 18 or more, 19 or more, 20 or more capsular saccharides from different *S. pneumoniae* serotypes.

10. The immunogenic composition according to any of claims 1 to 9 further comprising *S. pneumoniae* protein antigens.

11. The immunogenic composition according to any of claims 1 to 10 further comprising a pharmaceutically acceptable excipient, carrier or adjuvant, wherein the pharmaceutically acceptable excipient or carrier includes a buffer selected from Tris (trimethamine), phosphate (e.g. sodium phosphate), acetate, borate (e.g. sodium borate), citrate, glycine, histidine and succinate (e.g. sodium succinate).

12. The immunogenic composition according to any of claims 1 to 11 further comprising an adjuvant, optionally wherein the adjuvant is an aluminum salt such as aluminum hydroxide gel (alum) or aluminium phosphate.

13. A vaccine comprising an immunogenic composition any one of claims 1 to 12.

14. An immunogenic composition according to any one of claims 1 to 12, or a vaccine according to claim 13, for use in the treatment or prevention of a disease caused by *Streptococcus pneumoniae* infection.

15. The immunogenic composition or vaccine for use according to claim 14 wherein the disease is pneumonia, invasive pneumococcal disease (IPD), exacerbations of chronic obstructive pulmonary disease (COPD), otitis media, meningitis, bacteraemia, pneumonia and/or conjunctivitis.
